(19) Europäisches Patentamt
European Patent Office
Office européen des brevets

(11) Veröffentlichungsnummer: **0 596 326 B1**

# (12) EUROPÄISCHE PATENTSCHRIFT

(45) Veröffentlichungstag der Patentschrift: **23.08.95**

(21) Anmeldenummer: **93116959.3**

(22) Anmeldetag: **20.10.93**

(51) Int. Cl.6: **C07D 401/10**, C07D 401/14, C07D 413/10, C07D 417/10, A61K 31/415, A61K 31/42, A61K 31/425, A61K 31/445, A61K 31/455

## (54) Aryliden-1-azacycloalkane und Arylalkyl-1-azacycloalkane, deren Salze, diese Verbindungen enthaltende Arzneimittel und deren Verwendung sowie Verfahren zu ihrer Herstellung.

(30) Priorität: **22.10.92 DE 4235590**
**10.02.93 DE 4303840**

(43) Veröffentlichungstag der Anmeldung:
**11.05.94 Patentblatt 94/19**

(45) Bekanntmachung des Hinweises auf die Patenterteilung:
**23.08.95 Patentblatt 95/34**

(84) Benannte Vertragsstaaten:
**AT BE CH DE DK ES FR GB GR IE IT LI LU NL PT SE**

(56) Entgegenhaltungen:
**EP-A- 0 420 116**
**EP-A- 0 468 434**
**EP-A- 0 506 072**
**US-A- 4 863 932**

(73) Patentinhaber: **Dr. Karl Thomae GmbH**

**D-88397 Biberach (DE)**

(72) Erfinder: **Maier, Roland, Dr. Dipl.-Chem.**
**Bodelschwinghstrasse 39**
**W-88400 Biberach (DE)**
Erfinder: **Müller, Peter, Dr. Dipl.-Chem.**
**Buchenweg 6**
**D-88441 Mittelbiberach (DE)**
Erfinder: **Woitun, Eberhard, Dr. Dipl.-Chem.**
**Stecherweg 17**
**D-88400 Biberach (DE)**
Erfinder: **Hurnaus, Rudolf, Dr. Dipl.-Chem.**
**Silcherstrasse 19**
**D-88400 Biberach (DE)**
Erfinder: **Mark, Michael, Dr.**
**Schlehenhang 17**
**D-88400 Biberach (DE)**
Erfinder: **Eisele, Bernhard, Dr. Dipl.-Chem.**
**Beethovenstrasse 12**
**D-88400 Biberach (DE)**
Erfinder: **Budzinski, Ralph-Michael, Dr. Dipl.-Bio.**
**Thüringenstrasse 28**
**D-88400 Biberach (DE)**

EP 0 596 326 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Hallermayer, Gerhard, Dr.**
**Dipl.-Chem.**
**Grüner Weg 8**
**W-88437 Maselheim-Sulmingen (DE)**

## Beschreibung

Die vorliegende Erfindung betrifft Aryliden-1-azacycloalkane und Arylalkyl-1-azacycloalkane, deren Salze mit physiologisch verträglichen organischen und anorganischen Säuren, Verfahren zur Herstellung dieser Verbindungen und diese enthaltende Arzneimittel.

Die erfindungsgemäßen Verbindungen stellen Inhibitoren der Cholesterolbiosynthese dar, insbesondere Inhibitoren des Enzyms 2,3-Epoxisqualen-Lanosterol-Cyclase, eines Schlüsselenzyms der Cholesterolbiosynthese. Die erfindungsgemäßen Verbindungen sind geeignet zur Behandlung und Prophylaxe von Hyperlipidämien, Hypercholesterolämien und der Atherosklerose. Weitere mögliche Anwendungsgebiete ergeben sich für die Behandlung von hyperproliferativen Haut- und Gefäßerkrankungen, Tumoren, Gallensteinleiden sowie von Mykosen.

Verbindungen, die in die Cholesterolbiosynthese eingreifen, sind für die Behandlung einer Reihe von Krankheitsbildern von Bedeutung. Hier sind vor allem Hypercholesterolämien und Hyperlipidämien zu nennen, die Risikofaktoren für das Entstehen atherosklerotischer Gefäßveränderungen und ihrer Folgeerkrankungen wie beispielsweise koronare Herzkrankheit, cerebrale Ischämie, Claudicatio intermittens und Gangrän darstellen.

Die Bedeutung überhöhter Serum-Cholesterol-Spiegel als Hauptrisikofaktor für das Entstehen atherosklerotischer Gefäßveränderungen wird allgemein anerkannt. Umfangreiche klinische Studien haben zu der Erkenntnis geführt, daß durch Erniedrigung des Serumcholesterols das Risiko, an koronaren Herzkrankheiten zu erkranken, verkleinert werden kann (Current Opinion in Lipidology 2(4), 234 [1991]). Da der größte Teil des Cholesterols im Organismus selbst synthetisiert und nur ein geringer Teil mit der Nahrung aufgenommen wird, stellt die Hemmung der Biosynthese einen besonders attraktiven Weg dar, den erhöhten Cholesterolspiegel zu senken.

Daneben werden als weitere mögliche Anwendungsgebiete von Cholesterolbiosynthesehemmern die Behandlung hyperproliferativer Haut- und Gefäßerkrankungen sowie von Tumorerkrankungen, die Behandlung und Prophylaxe von Gallensteinleiden sowie der Einsatz bei Mykosen beschrieben. Hierbei handelt es sich im letzten Fall um einen Eingriff in die Ergosterolbiosynthese in Pilzorganismen, welche weitgehend analog der Cholesterolbiosynthese in Säugerzellen verläuft.

Die Cholesterol- bzw. die Ergosterolbiosynthese verläuft, ausgehend von Essigsäure, über eine größere Zahl von Reaktionsschritten. Dieser Vielstufenprozeß bietet eine Reihe von Eingriffsmöglichkeiten, von denen als Beispiele genannt seien:
Für die Inhibition des Enzyms 3-Hydroxy-3-methylglutaryl-Coenzym A (HMG-CoA)-Synthase werden β-Lactone und β-Lactame mit potentieller antihypercholesterolämischer Wirkung erwähnt (siehe J. Antibiotics <u>40</u>, 1356 [1987], US-A-4,751,237, EP-A-0 462 667, US-A-4,983,597).

Inhibitoren des Enzyms HMG-CoA-Reduktase stellen 3,5-Dihydroxycarbonsäuren vom Mevinolintyp und deren δ-Lactone dar, deren Vertreter Lovastatin, Simvastatin und Pravastatin in der Therapie von Hypercholesterolämien Verwendung finden. Weitere mögliche Anwendungsgebiete dieser Verbindungen sind Pilzinfektionen (US-A-4,375,475, EP-A-0 113 881, US-A-5,106,992), Hauterkrankungen (EP-A-0 369 263) sowie Gallensteinleiden und Tumorerkrankungen (US-A-5,106,992; Lancet <u>339</u>, 1154-1156 [1992]). Die Hemmung der Proliferation glatter Muskelzellen durch Lovastatin ist beschrieben in Cardiovasc. Drugs. Ther. <u>5</u>, Suppl. 3, 354 [1991].

Inhibitoren des Enzyms Squalen-Synthetase sind z.B. Isoprenoid-(phosphinylmethyl)phosphonate, deren Eignung zur Behandlung von Hypercholesterolämien, Gallensteinleiden und Tumorerkrankungen beschrieben ist in EP-A-0 409 181 sowie J. Med. Chemistry <u>34</u>, 1912 [1991], ferner die Squalestatine mit cholesterolsenkender und antimykotischer Wirkung (J. Antibotics <u>45</u>, 639-647 [1992] und J. Biol. Chemistry <u>267</u>, 11705-11708 [1992].

Als Inhibitoren des Enzyms Squalen-Epoxidase sind bekannt Allylamine wie Naftifin und Terbinafin, die als Mittel gegen Pilzerkrankungen Eingang in die Therapie gefunden haben, sowie das Allylamin NB-598 mit antihypercholesterolämischer Wirkung (J. Biol. Chemistry <u>265</u>, 18075-18078, [1990]) und Fluorsqualen-Derivate mit hypocholesterolämischer Wirkung (US-A-5,011,859). Des weiteren sind Piperidine und Azadecaline mit potentieller hypocholesterolämischer und/oder antifungaler Wirkung beschrieben, deren Wirkmechanismus nicht eindeutig geklärt ist und welche Squalenepoxidase- und/oder 2,3-Epoxisqualen-Lanosterol-Cyclase-Inhibitoren darstellen (EP-A-0 420 116, EP-A-0 468 434, US-A-5,084,461 und EP-A-0 468 457).

Beispiele für Inhibitoren des Enzyms 2,3-Epoxisqualen-Lanosterol-Cyclase sind Diphenylderivate (EP-A-0 464 465), Aminoalkoxybenzol-Derivate (EP-A-0 410 359) sowie Piperidin-Derivate (J. Org. Chem. <u>57</u>, 2794-2803, [1992]), die eine antifungale Wirkung besitzen. Des weiteren wird dieses Enzym in Säugetierzellen durch Decaline, Azadecaline und Indanderivate (WO 89/08450, J. Biol. Chemistry <u>254</u>, 11258-11263 [1981], Biochem. Pharmacology <u>37</u>, 1955-1964 [1988] und J 64 003 144), ferner durch 2-Aza-2,3-dihydrosqualen und 2,3-Epiminosqualen (Biochem. Pharmacology 34, 2765-2777 [1985]), durch Squalenoid-Epoxid-Vinylether (J. Chem. Soc. Perkin Trans. I, 1988, 461) und 29-Methyliden-2,3-oxidosqualen (J. Amer. Chem. Soc. 113, 9673-9674 [1991]) inhibiert.

Schließlich sind als Inhibitoren des Enzyms Lanosterol-14α-Demethylase noch Steroidderivate mit potentieller antihyperlipämischer Wirkung zu nennen, die gleichzeitig das Enzym HMG-CoA-Reduktase beeinflussen (US-A-5,041,432, J. Biol. Chemistry 266, 20070-20078 [1991], US-A-5,034,548). Außerdem wird dieses Enzym durch die Antimykotika vom Azol-Typ inhibiert, welche N-substituierte Imidazole und Triazole darstellen. Zu dieser Klasse gehören beispielsweise die auf dem Markt befindlichen Antimykotika Ketoconazol und Fluconazol.

Die Verbindungen der nachfolgenden allgemeinen Formel I sind neu. Es wurde überraschenderweise gefunden, daß sie sehr wirksame Inhibitoren des Enzyms 2,3-Epoxisqualen-Lanosterol-Cyclase (Internationale Klassifizierung: EC5.4.99.7) darstellen.

Das Enzym 2,3-Epoxisqualen-Lanosterol-Cyclase katalysiert einen Schlüsselschritt der Cholesterol- bzw. Ergosterol-Biosynthese, nämlich die Umwandlung des 2,3-Epoxisqualens in das Lanosterol, die erste Verbindung mit Steroidstruktur in der Biosynthesekaskade. Inhibitoren dieses Enzyms lassen gegenüber Inhibitoren früherer Biosyntheseschritte, wie beispielsweise HMG-CoA-Synthase und HMG-CoA-Reduktase, den Vorteil der höheren Selektivität erwarten, da die Inhibierung dieser frühen Biosyntheseschritte zur Abnahme biosynthetisch gebildeter Mevalonsäure führt und dadurch auch die Biosynthese der mevalonsäureabhängigen Substanzen Dolichol, Ubichinon und Isopentenyl-t-RNA negativ beeinflussen kann (vgl. J. Biol. Chemistry 265, 18075-18078 [1990]).

Bei Inhibierung von Biosyntheseschritten nach der Umwandlung von 2,3-Epoxisqualen in Lanosterol besteht die Gefahr der Anhäufung von Intermediärprodukten mit Steroidstruktur im Organismus und der Auslösung dadurch bedingter toxischer Effekte. Dies ist beispielsweise für Triparanol, einem Desmosterol-Reduktase-Inhibitor, beschrieben. Diese Substanz mußte wegen Bildung von Katarakten, Ichthyosis und Alopecie vom Markt genommen werden (zitiert in J. Biol. Chemistry 265, 18075-18078 [1990]).

Wie bereits eingangs dargelegt sind Inhibitoren der 2,3-Epoxisqualen-Lanosterol-Cyclase vereinzelt in der Literatur beschrieben. Die Strukturen dieser Verbindungen sind jedoch völlig verschieden von der Struktur der erfindungsgemäßen Verbindungen der nachstehend genannten allgemeinen Formel I.

Die Erfindung betrifft die Bereitstellung von antihypercholesterolämischen Substanzen, die zur Behandlung und Prophylaxe der Atherosklerose geeignet sind und, im Vergleich zu bekannten Wirkstoffen, durch eine bessere antihypercholesterolämische Wirkung bei erhöhter Selektivität und damit erhöhter Sicherheit ausgezeichnet sind. Da die erfindungsgemäßen Verbindungen auf Grund ihrer hohen Wirksamkeit als Inhibitoren des Enzyms 2,3-Epoxisqualen-Lanosterol-Cyclase auch die Ergosterol-Biosynthese im Pilzorganismus inhibieren können, sind sie auch zur Behandlung von Mykosen geeignet.

Die Aryliden-1-azacycloalkane und Arylalkyl-1-azacycloalkane der vorliegenden Erfindung und ihre Salze besitzen die allgemeine Formel I. Die Verbindungen können gegebenenfalls auch in Form von Enantiomeren, Diastereomeren oder deren Gemischen vorliegen.

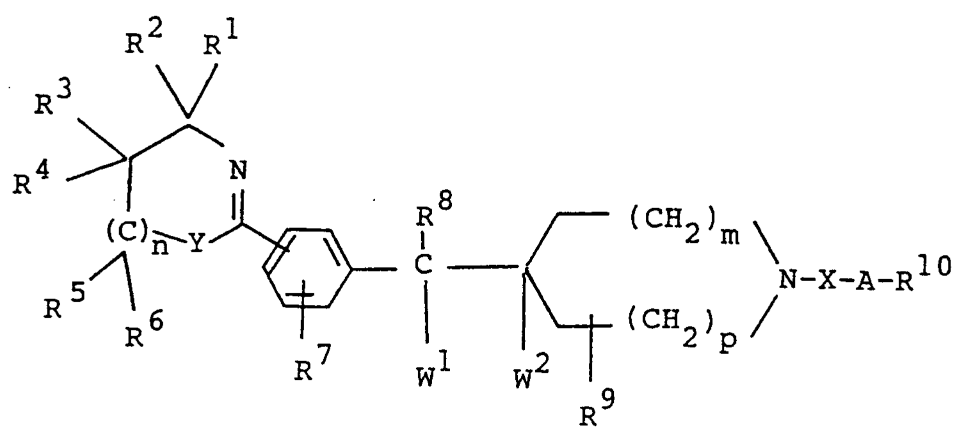

(I)

In der allgemeinen Formel I bedeuten
n die Zahlen 0 oder 1,
m die Zahlen 1 oder 2,
p die Zahlen 0 oder 1,
A eine Einfachbindung, eine geradkettige oder verzweigte Alkylengruppe mit 1 bis 17 Kohlenstoffatomen,

eine Alkenylengruppe mit 2 bis 17 Kohlenstoffatomen oder eine Alkinylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,
Y ein Sauerstoff- oder Schwefelatom oder eine $>NR^{11}$-Gruppe,
$R^1$ bis $R^6$ jeweils ein Wasserstoffatom oder
einer, zwei oder drei der Reste $R^1$ bis $R^6$, wobei die Reste gleich oder verschieden sein können, eine geradkettige oder verzweigte Alkylgruppe mit 1 bis 4 Kohlenstoffatomen, die durch eine Hydroxy-, Alkoxy-, Alkylthio- oder Dialkylaminogruppe oder durch eine gegebenenfalls durch ein Halogenatom oder eine Alkylgruppe substituierte Phenylgruppe substituiert sein kann, oder eine Alkoxycarbonylgruppe und die übrigen der Reste $R^1$ bis $R^6$ jeweils ein Wasserstoffatom,
wobei einer, zwei oder alle drei der Reste $R^1$, $R^3$ und $R^5$ auch eine gegebenenfalls durch eine Alkylgruppe oder ein Halogenatom substituierte Phenylgruppe bedeuten können,
$R^7$ ein Wasserstoff- oder Halogenatom, eine Alkyl- oder Alkoxygruppe,
$R^8$ und $R^9$ unabhängig voneinander jeweils ein Wasserstoffatom oder eine Alkylgruppe,
$R^{10}$ ein Wasserstoffatom, eine Cycloalkylgruppe mit 3 bis 6 Kohlenstoffatomen, eine gegebenenfalls durch ein Halogenatom, eine geradkettige oder verzweigte Alkylgruppe mit 1 bis 4 Kohlenstoffatomen, eine Trifluormethyl-, Alkoxy-, Cyano-, Nitro-, Alkylsulfonyl- oder Phenylgruppe substituierte Phenylgruppe, eine durch zwei Trifluormethylgruppen, zwei bis fünf Halogenatome oder durch ein Halogenatom und eine Alkylgruppe substituierte Phenylgruppe, eine gegebenenfalls durch ein Fluoratom substituierte Naphthyl- oder Tetrahydronaphthylgruppe, eine Pyridylgruppe oder eine gegebenenfalls durch ein Halogenatom oder eine Alkylgruppe substituierte Thienylgruppe,
$R^{11}$ ein Wasserstoffatom oder eine Alkylgruppe,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkyl-, Alkoxy-, Alkylthio- und Alkylsulfonylreste jeweils 1 bis 3 Kohlenstoffatome enthalten können und die vorstehend erwähnten Halogenatome jeweils ein Fluor-, Chlor- oder Bromatom bedeuten können.

Bevorzugt sind die Verbindungen der allgemeinen Formel Ia,

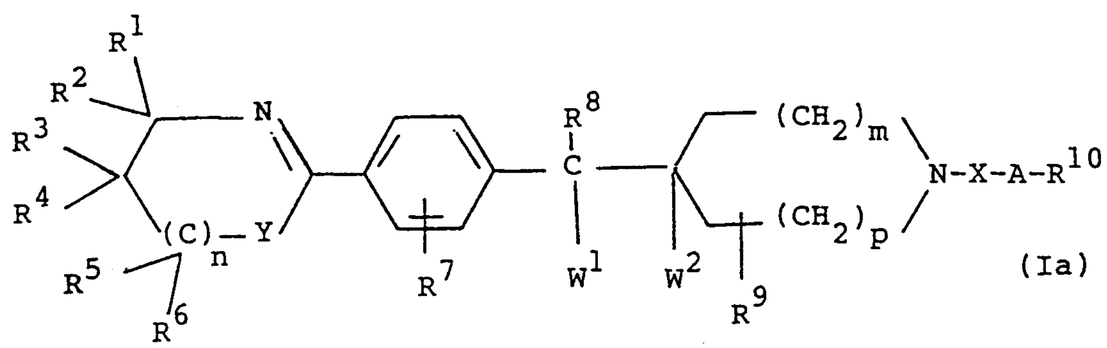

(Ia)

in der
n die Zahlen 0 oder 1,
m die Zahlen 1 oder 2,
p die Zahl 0 oder 1,
A eine Einfachbindung, eine geradkettige oder verzweigte Alkylengruppe mit 1 bis 17 Kohlenstoffatomen, eine Alkenylengruppe mit 2 bis 4 Kohlenstoffatomen oder eine Alkinylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,
Y ein Sauerstoff- oder Schwefelatom oder eine $>NR^{11}$-Gruppe,
$R^1$ bis $R^4$ jeweils ein Wasserstoffatom oder
einer oder zwei der Reste $R^1$ bis $R^4$ unabhängig voneinander jeweils eine geradkettige oder verzweigte Alkylgruppe mit 1 bis 4 Kohlenstoffatomen, die durch eine Hydroxy-, Alkylthio- oder Dialkylaminogruppe oder durch eine gegebenenfalls durch ein Halogenatom substituierte Phenylgruppe substituiert sein kann, oder eine Phenylgruppe und die übrigen der Reste $R^1$ bis $R^4$ jeweils ein Wasserstoffatom,

$R^5$ und $R^6$, die gleich oder verschieden sein können, ein Wasserstoffatom oder eine Methylgruppe,
$R^7$ ein Wasserstoff- oder Halogenatom, eine Alkyl- oder Alkoxygruppe,
$R^8$ ein Wasserstoffatom oder eine Alkylgruppe,
$R^9$ ein Wasserstoffatom,
$R^{10}$ ein Wasserstoffatom, eine Cycloalkylgruppe mit 3 bis 6 Kohlenstoffatomen, eine gegebenenfalls durch ein oder zwei Halogenatome, eine geradkettige oder verzweigte Alkylgruppe mit 1 bis 4 Kohlenstoffatomen, eine Trifluormethyl-, Methoxy-, Cyano-, Nitro-, Methylsulfonyl- oder Phenylgruppe substituierte Phenylgruppe, eine durch zwei Trifluormethylgruppen oder durch ein Halogenatom und eine Methylgruppe substituierte Phenylgruppe, eine durch drei bis fünf Fluoratome substituierte Phenylgruppe, eine gegebenenfalls durch ein Fluoratom substituierte Naphthylgruppe, eine Tetrahydronaphthyl- oder Pyridylgruppe oder eine gegebenenfalls durch ein Halogenatom substituierte Thienylgruppe und
$R^{11}$ ein Wasserstoffatom oder eine Alkylgruppe bedeuten,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkylreste jeweils 1 bis 3 Kohlenstoffatome enthalten können und die vorstehend erwähnten Halogenatome jeweils ein Fluor-, Chlor- oder Bromatom bedeuten können, deren Enantiomere, Diastereomere und deren Salze.

Besonders bevorzugt sind die Verbindungen der allgemeinen Formel Ia, in der
n die Zahlen 0 oder 1,
m die Zahlen 1 oder 2,
p die Zahlen O oder 1,
A eine Einfachbindung, eine geradkettige oder verzweigte Alkylengruppe mit 1 bis 17 Kohlenstoffatomen oder eine Alkenylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,
Y ein Sauerstoffatom oder eine $>NR^{11}$-Gruppe,
$R^1$ bis $R^4$ jeweils ein Wasserstoffatom oder
einer oder zwei der Reste $R^1$ bis $R^4$ unabhängig voneinander jeweils eine geradkettige oder verzweigte Alkylgruppe mit 1 bis 4 Kohlenstoffatomen, und die übrigen der Reste $R^1$ bis $R^4$ jeweils ein Wasserstoffatom,
$R^5$ und $R^6$, die gleich oder verschieden sein können, ein Wasserstoffatom oder eine Methylgruppe,
$R^7$ ein Wasserstoff- oder Halogenatom, eine Methyl- oder Methoxygruppe,
$R^8$ ein Wasserstoffatom oder eine Methylgruppe,
$R^9$ ein Wasserstoffatom,
$R^{10}$ ein Wasserstoffatom, eine Cycloalkylgruppe mit 3 bis 6 Kohlenstoffatomen, eine gegebenenfalls durch ein oder zwei Halogenatome, fünf Fluoratome, eine Alkylgruppe, eine oder zwei Trifluormethylgruppen oder durch ein Halogenatom und eine Alkylgruppe substituierte Phenylgruppe, eine in 4-Position gegebenenfalls durch ein Fluoratom substituierte 1-Naphthylgruppe, eine 2-Naphthylgruppe, eine 1,2,3,4-Tetrahydro-2-naphthylgruppe, eine Pyridyl- oder 4-Biphenylgruppe oder eine gegebenenfalls durch ein Halogenatom substituierte Thienylgruppe und
$R^{11}$ ein Wasserstoffatom oder eine Methylgruppe bedeuten,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkylteile jeweils 1 bis 3 Kohlenstoffatome enthalten können und die vorstehend erwähnten Halogenatome jeweils ein Fluor- oder Chloratom bedeuten können, deren Enantiomere, Diastereomere und deren Salze.

Ganz besonders bevorzugt sind die Verbindungen der allgemeinen Formel Ia, in der
n die Zahlen 0 oder 1,
m die Zahl 1,
p die Zahlen O oder 1,
A eine Einfachbindung,
$W^1$ und $W^2$ jeweils ein Wasserstoffatom oder zusammen eine Kohlenstoff-Kohlenstoffbindung,
X eine Carbonylgruppe,
Y ein Sauerstoffatom,
$R^1$ bis $R^6$ jeweils ein Wasserstoffatom,
$R^7$ ein Wasserstoff- oder Halogenatom oder eine Methylgruppe,
$R^8$ und $R^9$ jeweils ein Wasserstoffatom,
$R^{10}$ eine in Position 4 durch ein Fluor-, Chlor- oder Bromatom oder eine Trifluormethylgruppe substituierte

Phenylgruppe, eine 4-Chlor-3-methylphenylgruppe, eine 5-Chlor-2-thienylgruppe oder eine Cyclohexylgruppe bedeuten,
und deren Salze,
insbesondere jedoch die Verbindungen
(1) 1-(4-Chlorbenzoyl)-4-[4-(2-oxazolin-2-yl)benzyliden]piperidin
(2) 1-(4-Chlorbenzoyl)-4-[4-(4,5-dihydro-6H-oxazin-2-yl)benzyliden]piperidin
(3) 4-[4-(2-Oxazolin-2-yl)benzyliden]-1-(4-trifluormethylbenzoyl)piperidin
(4) 1-(4-Chlor-3-methylbenzoyl)-4-[4-(2-oxazolin-2-yl)benzyliden]piperidin
(5) 1-(4-Fluorbenzoyl)-4-[4-(2-oxazolin-2-yl)benzyliden]piperidin
(6) 1-(5-Chlor-2-thienoyl)-4-[4-(2-oxazolin-2-yl)benzyliden]piperidin
(7) 1-Cyclohexancarbonyl-4-[4-(2-oxazolin-2-yl)benzyliden]piperidin,
(8) 4-[4-(2-Oxazolin-2-yl)benzyl]-1-(4-trifluormethylbenzoyl)piperidin
(9) 1-(4-Chlorbenzoyl)-4-[4-(2-oxazolin-2-yl)benzyl]piperidin
(10) 1-(4-Chlorbenzoyl)-3-[4-(2-oxazolin-2-yl)benzyliden]pyrrolidin
(11) 1-(4-Chlorbenzoyl)-4-[2-fluor-4-(2-oxazolin-2-yl)benzyliden]piperidin
(12) 1-(4-Chlorbenzoyl)-4-[3-methyl-4-(2-oxazolin-2-yl)benzyliden]piperidin
und deren Salze.

Herstellungsmethoden:

Die Verbindungen der allgemeinen Formel I lassen sich nach folgenden Methoden darstellen:
a) Durch Umsetzung von Verbindungen der allgemeinen Formel II,

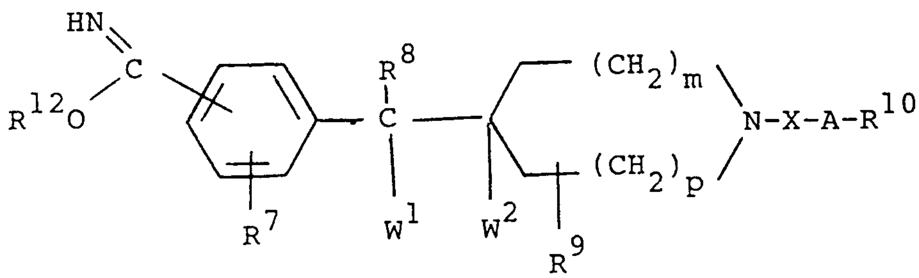

(II)

in der
m, p, A, $W^1$, $W^2$, X und $R^7$ bis $R^{10}$ die eingangs erwähnten Bedeutungen besitzen und $R^{12}$ eine Alkylgruppe mit 1 bis 10 Kohlenstoffatomen bedeutet, mit Verbindungen der allgemeinen Formel III,

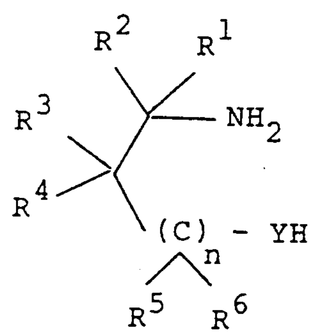

(III)

in der
n, Y und $R^1$ bis $R^6$ die eingangs erwähnten Bedeutungen besitzen.

Die Umsetzungen werden zweckmäßigerweise in einem geeigneten Lösungsmittel, z. B. in einem Alkohol wie Methanol, Ethanol oder Propanol, in einem Ether wie beispielsweise Diethylether, Di-n-propylether, Tetrahydrofuran oder Dioxan, in Dimethylformamid, Dimethylsulfoxid oder in einem Gemisch der vorstehend erwähnten Lösungsmittel, gegebenenfalls in Anwesenheit eines halogenwasserstoffbindenden Mittels wie tertiäre Amine, Natriumcarbonat oder Calciumcarbonat bei einer Temperatur zwischen 0 und 100 °C durchgeführt. Vorzugsweise wird die Reaktion jedoch in einem Alkohol wie Methanol oder Ethanol bei einer Temperatur zwischen 20 und 80 °C durchgeführt. Vorteilhafterweise werden die Verbindungen der allgemeinen Formel II als Salze, vorzugsweise als Hydrochloride, eingesetzt und die Reaktion in Gegenwart von anorganischen oder organischen Basen, vorzugsweise tertiären organischen Aminen wie Triethylamin oder Ethyldiisopropylamin oder einem Überschuß der Verbindungen der allgemeinen Formel III durchgeführt.

b) Zur Herstellung von Verbindungen der allgemeinen Formel I, in der n, m, p, A, X, Y und $R^1$ bis $R^{10}$ die eingangs erwähnten Bedeutungen besitzen und $W^1$ und $W^2$ zusammen eine Kohlenstoff-Kohlenstoffbindung bedeuten:

Umsetzung von Phosphonestern der allgemeinen Formel IV,

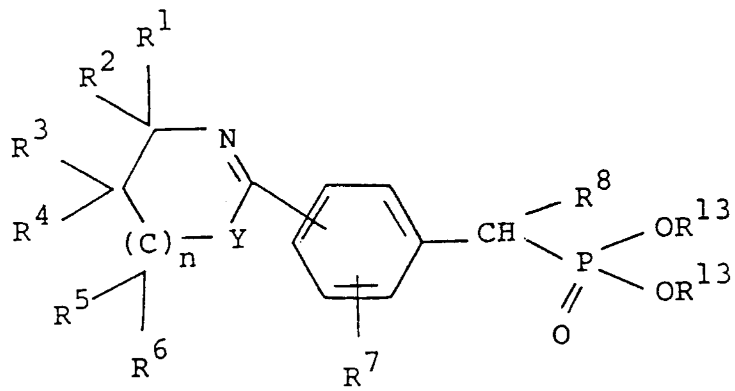

(IV)

in der

n, Y und $R^1$ bis $R^8$ die eingangs erwähnten Bedeutungen besitzen und $R^{13}$ eine Alkylgruppe mit 1 bis 10 Kohlenstoffatomen bedeutet, mit Verbindungen der allgemeinen Formel V,

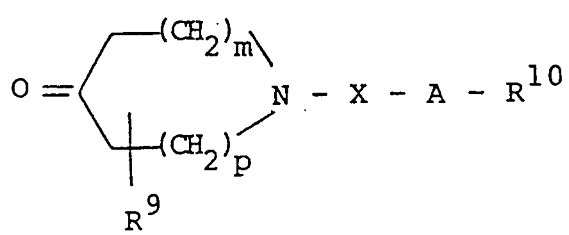

(V)

in der

m, p, A, X, $R^9$ und $R^{10}$ die eingangs erwähnten Bedeutungen besitzen.

Die Umsetzungen werden zweckmäßigerweise in einem geeigneten Lösungsmittel, z. B. in einem Ether wie Diethylether, Di-n-propylether, Tetrahydrofuran oder Dioxan, oder in einem Kohlenwasserstoff wie beispielsweise Benzol, Toluol, n-Hexan oder Cyclohexan, oder in einem Gemisch der vorstehend erwähnten Lösungsmittel, vorzugsweise jedoch in einem Gemisch aus Tetrahydrofuran und n-Hexan oder einem Gemisch aus Tetrahydrofuran und Cyclohexan, durchgeführt. Dabei werden zunächst die Verbindungen der allgemeinen Formel IV bei einer Temperatur zwischen -78 und 20 °C mit einer geeigneten Base wie beispielsweise n-Butyllithium, Phenyllithium, Natriumamid, Natriumhydrid oder Lithiumdiisoproylamid in die entsprechenden Phosphonatanionen übergeführt und diese anschließend bei einer Temperatur zwischen -78 und 100 °C, vorzugsweise jedoch bei einer Temperatur zwischen -15 und 50 °C mit den Verbindungen der allgemeinen Formel V umgesetzt.

Besitzt eine Verbindung der allgemeinen Formel IV eine Hydroxygruppe, empfiehlt es sich, diese vor Durchführung der Reaktion durch eine geeignete Schutzgruppe wie beispielsweise die Silylgruppe zu schützen und die Schutzgruppe nach beendeter Reaktion wieder abzuspalten. Als Silylierungsreagens eignet sich z. B. Trimethylchlorsilan. Die Abspaltung erfolgt beispielsweise mittels saurer Hydrolyse oder durch Behandeln mit Fluoridionen, z. B. mit Cäsiumfluorid oder Tetrabutylammoniumfluorid.

c) Durch Umsetzung von Verbindungen der allgemeinen Formel VI,

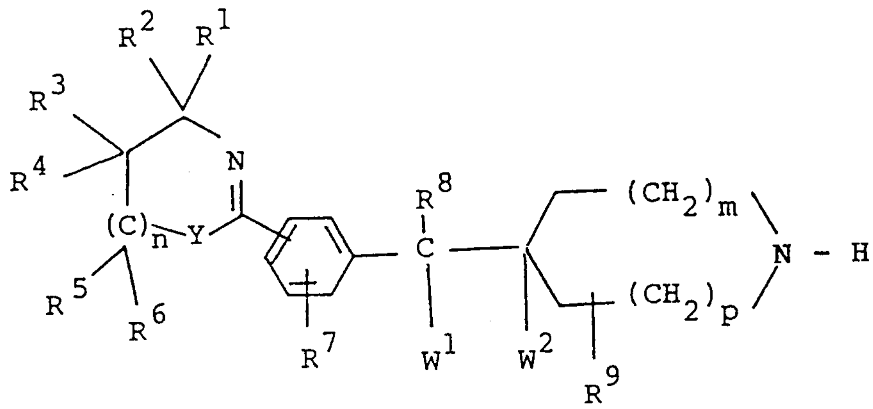

(VI)

in der
n, m, p, $W^1$, $W^2$, Y und $R^1$ bis $R^9$ die eingangs erwähnten Bedeutungen besitzen, mit Verbindungen der allgemeinen Formel VII,

$Z^1$ - X - A - $R^{10}$ (VII)

in der
A, X und $R^{10}$ die eingangs erwähnten Bedeutungen besitzen und $Z^1$ eine reaktive Austrittsgruppe wie z. B. ein Halogenatom, vorzugsweise ein Chloratom, oder die Imidazolidgruppe bedeutet.

Bedeutet $Z^1$ ein Halogenatom, werden die Umsetzungen in einem geeigneten inerten Lösungsmittel wie Diethylether, Toluol, Methylenchlorid und dergleichen, vorzugsweise bei Temperaturen zwischen -50 °C und 50 °C und in Gegenwart eines halogenwasserstoffbindenden Mittels, wie tertiäre Amine, Natriumcarbonat oder Calciumcarbonat, durchgeführt. Dabei können nicht nur die freien Amine der allgemeinen Formel VI eingesetzt werden, sondern auch deren Salze, aus denen in situ die Amine durch geeignete Basen, z. B. tertiäre organische Amine, freigesetzt werden können.

Bedeutet $Z^1$ den Imidazolidrest, werden die Umsetzungen vorzugsweise in einem inerten Lösungsmittel, wie Xylol oder Tetrahydrofuran, bei Temperaturen zwischen Raumtemperatur und der Siedetemperatur der Reaktionsmischung durchgeführt.

Besitzt eine Verbindung der allgemeinen Formel VI eine Hydroxygruppe, kann die Umsetzung so abgewandelt werden, daß zwei Äquivalente der Verbindung der allgemeinen Formel VII verwendet werden und nach beendeter Umsetzung die aus der Hydroxygruppe gebildete Estergruppe wieder verseift wird.

Die gegebenenfalls anschließende Verseifung einer so gebildeten Estergruppe erfolgt vorzugsweise durch alkalische Hydrolyse in einem wässrigen Lösungsmittel, z.B. in Wasser, Isopropanol/Wasser, Tetrahydrofuran/Wasser oder Dioxan/Wasser, beispielsweise in Gegenwart einer Alkalibase wie Natriumhydroxid oder Kaliumhydroxid bei Temperaturen zwischen 0 und 100 °C.

d) Zur Herstellung von Verbindungen der allgemeinen Formel I, in der n, m, p, X und $R^7$ bis $R^9$ wie eingangs erwähnt definiert sind, A eine Einfachbindung oder eine geradkettige oder verzweigte Alkylengruppe mit 1 bis 17 Kohlenstoffatomen, $W^1$ und $W^2$ jeweils ein Wasserstoffatom und Y ein Sauerstoffatom oder die $>NR^{11}$-Gruppe bedeuten, wobei $R^{11}$ wie eingangs erwähnt definiert ist, $R^1$ bis $R^6$ mit Ausnahme der durch eine Alkylthiogruppe substituierten geradkettigen oder verzweigten Alkylgruppe mit 1 bis 4 Kohlenstoffatomen und $R^{10}$ mit Ausnahme der gegebenenfalls durch ein Halogenatom substituierten Thienylgruppe wie eingangs erwähnt definiert sind, wobei A keine Einfachbindung sein kann, wenn X die Sulfonylgruppe und $R^{10}$ ein Wasserstoffatom bedeuten:
Hydrierung einer Verbindung der allgemeinen Formel I',

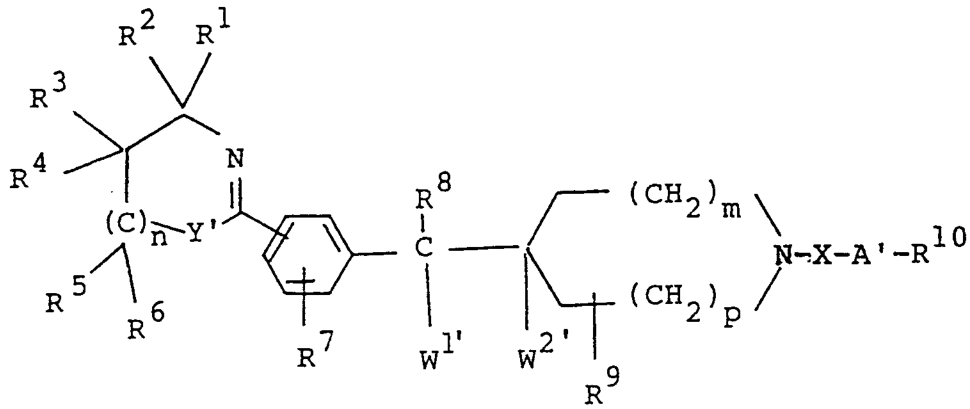

(I')

in der

n, m, p, X und $R^7$ bis $R^9$ wie eingangs erwähnt definiert sind, A' eine Einfachbindung oder eine geradkettige oder verzweigte Alkylengruppe mit 1 bis 17 Kohlenstoffatomen, $W^{1'}$ und $W^{2'}$ zusammen eine Kohlenstoff-Kohlenstoffbindung und Y' ein Sauerstoffatom oder die $>NR^{11}$-Gruppe bedeuten, wobei $R^{11}$ wie eingangs erwähnt definiert ist, $R^1$ bis $R^6$ mit Ausnahme der durch eine Alkylthiogruppe substituierten geradkettigen oder verzweigten Alkylgruppe mit 1 bis 4 Kohlenstoffatomen und $R^{10}$ mit Ausnahme der gegebenenfalls durch ein Halogenatom substituierten Thienylgruppe wie eingangs erwähnt definiert sind, wobei A keine Einfachbindung sein kann, wenn X die Sulfonylgruppe und $R^{10}$ ein Wasserstoffatom bedeuten.

Die Hydrierung wird in einem geeigneten Lösungsmittel wie in einem Alkohol, beispielsweise in Methanol, Ethanol oder Propanol, in einem Ester, beispielsweise in Ethylacetat, in einem Ether, beispielsweise in Diethylether, Tetrahydrofuran oder Dioxan, oder deren Gemischen mit katalytisch angeregtem Wasserstoff, z.B. mit Wasserstoff in Gegenwart eines Hydrierungskatalysators wie Raney-Nickel, Rhodium, Palladium, Palladium/Kohle, Platin oder Platin/Kohle und einem Wasserstoffdruck von 10 bis 500 psi, vorzugsweise jedoch 50 bis 100 psi, bei Temperaturen zwischen 0 und 100°C, vorzugsweise jedoch bei Raumtemperatur, durchgeführt.

Bei der vorstehenden Hydrierung kann ein in den Resten $R^1$ bis $R^6$ und $R^{10}$ vorhandenes Halogenatom gegebenenfalls durch ein Wasserstoffatom ausgetauscht werden.

e) Zur Herstellung von Verbindungen der allgemeinen Formel I, in der n, m, p, A, X, $W^1$, $W^2$ und $R^1$ bis $R^{10}$ die eingangs erwähnten Bedeutungen besitzen und Y ein Sauerstoffatom bedeutet:

Cyclisierung von Verbindungen der allgemeinen Formel VIII,

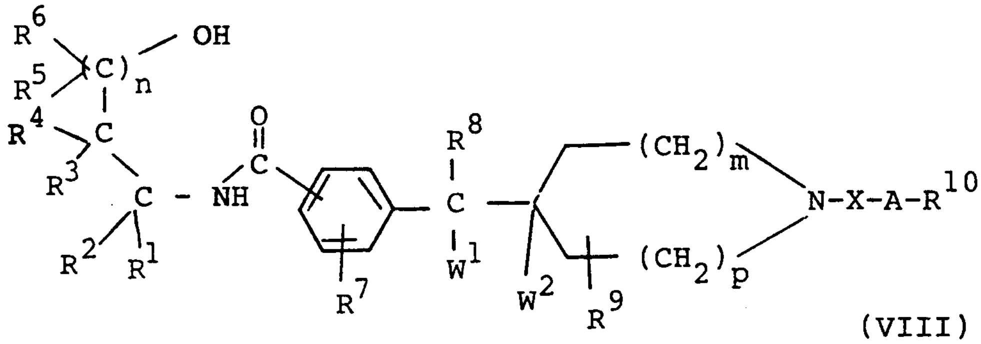

(VIII)

in der

n, m, p, A, X, $W^1$, $W^2$ und $R^1$ bis $R^{10}$ wie eingangs erwähnt definiert sind.

Die Cyclisierung wird zweckmäßigerweise in einem geeigneten Lösungsmittel, z. B. in Diethylether, Tetrahydrofuran, Dioxan, Diethylenglykoldimethylether, Methylenchlorid, Chloroform, Benzol, Toluol oder n-Hexan oder in einem Gemisch der vorstehend erwähnten Lösungsmittel in Gegenwart eines wasserentziehenden Mittels wie der Kombination Azodicarbonsäuredialkylester und Triphenylphosphin oder von Methyl-N-(triethylamoniosulfonyl)carbamat (Burgess-Reagenz) bei einer Temperatur zwischen 0 und 150°C, vorzugsweise bei einer Temperatur zwischen 0 und 100°C, durchgeführt. Die Verwendung des Burgess-Reagenz zur Herstellung von Oxazolinen ist beschrieben in Tetrahedron Letters 33, 907-910 [1992]. Eine

weitere Cyclisierungsmethode ist beschrieben in Tetrahedron Letters 33, 2807-2810 [1992].

Die nach den vorstehenden Verfahren hergestellten Verbindungen der allgemeinen Formel I lassen sich nach bekannten Methoden, z. B. Kristallisation, Destillation oder Chromatographie reinigen und isolieren. Sie können nach an sich bekannten Methoden in ihre Säureadditionssalze mit anorganischen oder organischen Säuren übergeführt werden.

In den erfindungsgemäßen Verbindungen der Formel I können je nach Natur der Reste $R^1$ bis $R^6$ die mit diesen Resten verbundenen Kohlenstoffatome in optisch aktiver Form vorliegen. Die Erfindung umfaßt sowohl die reinen Isomeren als auch die Gemische der verschiedenen Isomeren.

Ausgangsmaterialien:

Die Ausgangsverbindungen der allgemeinen Formel II lassen sich aus Verbindungen der allgemeinen Formel IX,

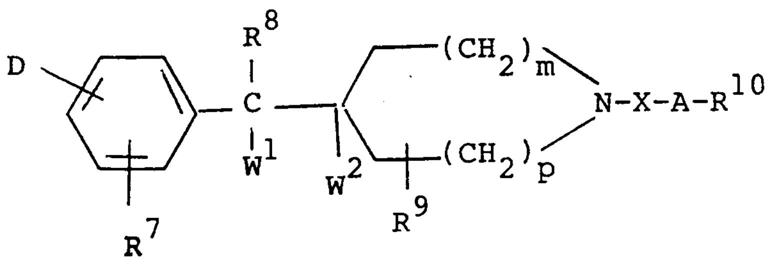

(IX)

in der
m, p, A, $W^1$, $W^2$, X und $R^7$ bis $R^{10}$ wie eingangs erwähnt definiert sind und D eine Cyanogruppe darstellt, durch Umsetzung mit Alkoholen der Formel $R^{12}OH$, wobei $R^{12}$ eine Alkylgruppe mit 1 bis 10 Kohlenstoffatomen bedeutet, in Gegenwart von Chlorwasserstoff herstellen.

Verbindungen der Formel IX, in der D eine Cyano- oder Alkoxycarbonylgruppe darstellt, sind ihrerseits zugänglich durch Umsetzung von Phosphonestern der Formel X,

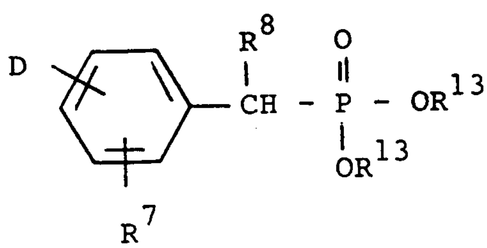

(X)

in der
$R^7$ und $R^8$ wie eingangs erwähnt definiert sind, $R^{13}$ eine Alkylgruppe mit 1 bis 10 Kohlenstoffatomen und D eine Cyano- oder Alkoxycarbonylgruppe darstellt, mit Ketonen der Formel V,

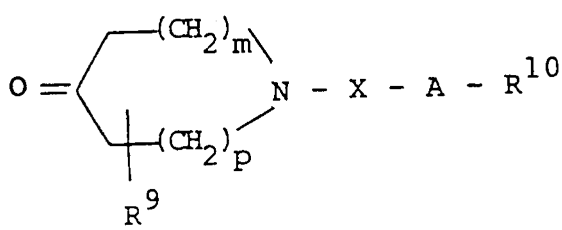

(V)

in der
m, p, A, X, $R^9$ und $R^{10}$ wie eingangs erwähnt definiert sind, in Gegenwart von Basen wie beispielsweise n-Butyllithium, Phenyllithium, Natriumamid, Natriumhydrid oder Lithiumdiisopropylamid und gegebenenfalls anschließender Hydrierung der so erhaltenen olefinischen Doppelbindung.

Eine alternative Methode zur Herstellung von Verbindungen der Formel IX, in der m, p, A, $W^1$, $W^2$, X und $R^7$ bis $R^{10}$ wie eingangs erwähnt definiert sind und D eine Alkoxycarbonyl- oder Cyanogruppe darstellt, besteht darin, daß Phosphonester der Formel X, in der $R^7$, $R^8$ und $R^{13}$ wie vorstehend erwähnt definiert sind und D eine Alkoxycarbonyl- oder Cyanogruppe bedeutet, mit Ketonen der Formel XI,

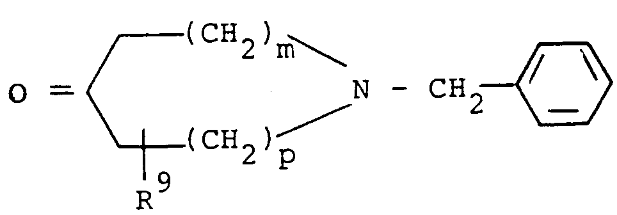

(XI)

in der
m, p und $R^9$ wie eingangs erwähnt definiert sind, umgesetzt werden.

Anschließend wird der Benzylrest entfernt, beispielsweise durch Umsetzung mit Chlorameisensäure-1-chlorethylester, das so gebildete Urethan mit Methanol gespalten und abschließend mit Verbindungen der allgemeinen Formel VII,

$Z^1$ - X - A - $R^{10}$ (VII)

in der
A, X und $R^{10}$ die eingangs erwähnten Bedeutungen besitzen und $Z^1$ eine reaktive Austrittsgruppe wie z. B. ein Halogenatom, vorzugsweise ein Chloratom, oder die Imidazolidgruppe bedeutet, umgesetzt. Die olefinische Doppelbindung kann anschließend, falls gewünscht, hydriert werden.

Die Phosphonester der Formel X lassen sich durch Arbusow-Reaktion aus Halogeniden der Formel XII,

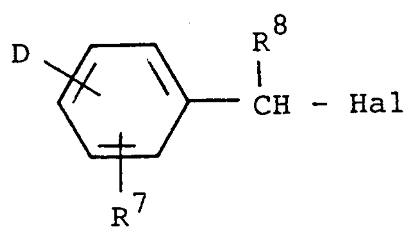

(XII)

in der
$R^7$ und $R^8$ wie eingangs erwähnt definiert sind, D eine Cyano- oder Alkoxycarbonylgruppe darstellt und Hal ein Halogenatom wie beispielsweise ein Chlor- oder Bromatom bedeutet, und Trialkylphosphiten der Formel $(R_{13}O)_3P$, in der $R^{13}$ wie vorstehend erwähnt definiert ist, darstellen. Eine bevorzugte Variante der Reaktion zur Herstellung von Verbindungen der Formel X, in der $R^8$ eine Alkylgruppe bedeutet, besteht darin, zunächst Verbindungen der Formel X herzustellen, in der $R^8$ ein Wasserstoffatom bedeutet, und diese anschließend mit einem Alkylierungsmittel der Formel $R^8$-$Z^2$, in der $R^8$ einen wie eingangs definierten Alkylrest und $Z^2$ ein Halogenatom wie ein Chlor-, Brom- oder Jodatom oder eine Sulfonyloxygruppe bedeutet, umzusetzen.

Die Ausgangsverbindungen der allgemeinen Formel III und XII sind literaturbekannt bzw. lassen sich nach bekannten Methoden herstellen.

Die Phosphonester der allgemeinen Formel IV lassen sich aus Phosphonestern der allgemeinen Formel XIII,

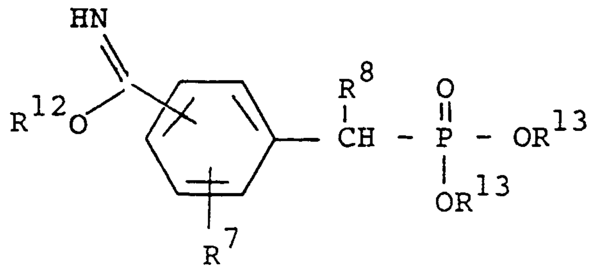

(XIII)

in der
$R^7$, $R^8$ und $R^{12}$ wie eingangs erwähnt definiert sind und $R^{13}$ eine Alkylgruppe mit 1 bis 10 Kohlenstoffatomen bedeutet, durch Umsetzung mit Verbindungen der allgemeinen Formel III erhalten.

Die Phosphonester der Formel XIII sind aus den Phosphonestern der Formel X, in der D eine Cyanogruppe bedeutet, durch Umsetzung mit Alkoholen der Formel $R^{12}OH$, in der $R^{12}$ wie vorstehend erwähnt definiert ist, in Gegenwart von Chlorwasserstoff zugänglich.

Die Verbindungen der allgemeinen Formel V werden durch Umsetzung von Verbindungen der Formel XIV,

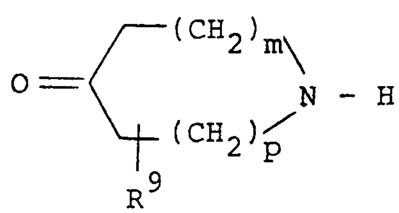

(XIV)

in der
m, p und $R^9$ wie eingangs erwähnt definiert sind, mit Verbindungen der allgemeinen Formel VII erhalten.

Die Ausgangsverbindungen der allgemeinen Formel VI lassen sich aus den Phosphonestern der allgemeinen Formel IV durch Umsetzung mit Verbindungen der allgemeinen Formel XV,

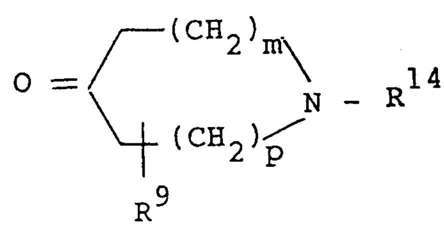

(XV)

in der
m, p und $R^9$ wie eingangs erwähnt definiert sind und $R^{14}$ die Triphenylmethyl- oder tert.Butyloxycarbonylgruppe bedeutet, in Gegenwart starker Basen wie n-Butyllithium, Phenyllithium, Natriumamid, Natriumhydrid oder Lithiumdiisopropylamid und gegebenenfalls anschließender katalytischer Hydrierung der gebildeten olefinischen Doppelbindung sowie Abspaltung des wie vorstehend definierten Restes $R^{14}$ mittels Trifluoressigsäure, wobei im Falle der Triphenylmethylverbindung diese Schutzgruppe gleichzeitig hydrogenolytisch abgespalten werden kann, darstellen.

Eine alternative Methode zur Herstellung von Verbindungen der allgemeinen Formel VI, in der p die Zahl O, m die Zahl 1 und $R^8$ ein Wasserstoffatom bedeutet und n, $W^1$, $W^2$, Y, $R^1$ bis $R^7$ sowie $R^9$ wie eingangs erwähnt definiert sind, besteht darin, daß zunächst eine Verbindung der Formel XVI,

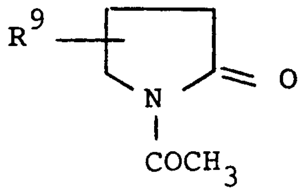

(XVI)

in der
$R^9$ wie eingangs definiert ist, mit einem Aldehyd der Formel XVII,

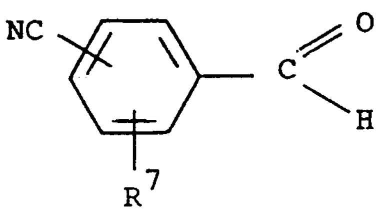

(XVII)

in der
$R^7$ wie eingangs definiert ist, unter gleichzeitiger Abspaltung des N-Acetylrestes kondensiert wird, anschließend unter Einwirkung von Chlorwasserstoff mit einem Alkohol der Formel $R^{12}OH$, in der $R^{12}$ eine Alkylgruppe mit 1 bis 10 Kohlenstoffatomen darstellt, umgesetzt wird und das so erhaltene Zwischenprodukt mit einer Verbindung der Formel III,

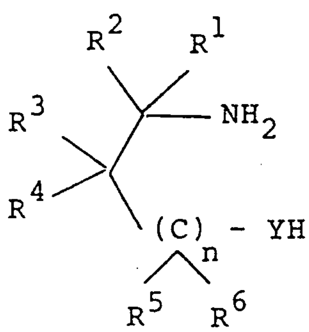

(III)

in der
n, Y und $R^1$ bis $R^6$ die eingangs erwähnten Bedeutungen besitzen, cyclisiert wird. Danach wird die Amidgruppe beispielsweise mit Lithiumaluminiumhydrid reduziert und, falls gewünscht, die olefinische Doppelbindung hydriert.

Ausgangsverbindungen der allgemeinen Formel VIII lassen sich aus Verbindungen der allgemeinen Formel IX, in der m, p, $W^1$, $W^2$, X, A sowie $R^7$ bis $R^{10}$ wie eingangs erwähnt definiert sind und D eine Alkoxycarbonylgruppe bedeutet, durch Verseifung der Alkoxycarbonylgruppe und Umsetzung der entstandenen Carbonsäure zunächst mit N,N'-Carbonyldiimidazol und anschließend mit Verbindungen der Formel III, in der n und $R^1$ bis $R^6$ wie eingangs definiert sind und Y ein Sauerstoffatom darstellt, herstellen.

Die Verbindungen der allgemeinen Formel I besitzen interessante biologische Eigenschaften. Sie stellen Inhibitoren der Cholesterolbiosynthese, insbesondere Inhibitoren des Enzyms 2,3-Epoxisqualen-Lanosterol-Cyclase dar. Aufgrund ihrer biologischen Eigenschaften sind sie besonders geeignet zur Behandlung und Prophylaxe von Hyperlipidämien, insbesondere der Hypercholesterolämie, der Hyperlipoproteinämie und der Hypertriglyceridämie und den daraus resultierenden atherosklerotischen Gefäßveränderungen mit ihren Folgeerkrankungen wie koronare Herzkrankheit, cerebrale Ischämie, Claudicatio intermittens, Gangrän und andere.

Zur Behandlung dieser Erkrankungen können die Verbindungen der allgemeinen Formel I dabei entweder alleine zur Monotherapie eingesetzt werden oder in Kombination mit anderen cholesterol- oder lipidsenkenden Substanzen zur Anwendung gelangen, wobei die Verbindungen vorzugsweise als orale

Formulierung, gegebenenfalls auch in Form von Suppositorien als rektale Formulierung verabreicht werden können. Als Kombinationspartner kommen dabei beispielsweise in Frage:

- gallensäurebindende Harze wie z. B. Cholestyramin, Cholestipol und andere,
- Verbindungen, die die Cholesterolresorption hemmen, wie z. B. Sitosterol und Neomycin,
- Verbindungen, die in die Cholesterolbiosynthese eingreifen, wie z. B. HMG-CoA-Reduktaseinhibitoren wie Lovastatin, Simvastatin, Pravastatin und andere,
- Squalen-Epoxidaseinhibitoren wie beispielsweise NB 598 und analoge Verbindungen sowie
- Squalen-Synthetaseinhibitoren wie beispielsweise Vertreter der Klasse der Isoprenoid-(phosphinylmethyl)phosphonate und Squalestatin.

Als weitere mögliche Kombinationspartner sind noch zu erwähnen die Klasse der Fibrate, wie Clofibrat, Bezafibrat, Gemfibrozil und andere, Nikotinsäure, ihre Derivate und Analoge wie beispielsweise Acipimox sowie Probucol.

Desweiteren sind die Verbindungen der allgemeinen Formel I geeignet zur Behandlung von Erkrankungen, die mit überhöhter Zellproliferation im Zusammenhang stehen. Cholesterol ist ein essentieller Zellbestandteil und muß für die Zellproliferation, d. h. Zellteilung, in ausreichender Menge vorhanden sein. Die Inhibierung der Zellproliferation durch Inhibierung der Cholesterolbiosynthese ist am Beispiel der glatten Muskelzellen mit dem HMG-CoA-Reduktaseinhibitor des Mevinolintyps Lovastatin, wie eingangs erwähnt, beschrieben.

Als Beispiele für Erkrankungen, die mit überhöhter Zellproliferation zusammenhängen sind zunächst Tumorerkrankungen zu nennen. In Zellkultur- und in-vivo-Experimenten wurde gezeigt, daß die Senkung des Serumcholesterols oder der Eingriff in die Cholesterolbiosynthese durch HMG-CoA-Reduktaseinhibitoren das Tumorwachstum vermindert (Lancet 339, 1154-1156 [1992]). Die erfindungsgemäßen Verbindungen der Formel I sind deshalb aufgrund ihrer cholesterolbiosynthese-inhibitorischen Wirkung potentiell für die Behandlung von Tumorerkrankungen geeignet. Sie können dabei alleine oder zur Unterstützung bekannter Therapieprinzipien Verwendung finden.

Als weitere Beispiele sind hyperproliferative Hauterkrankungen wie beispielsweise Psoriasis, Basalzellkarzinome, Plattenepithelkarzinome, Keratosis und Keratinisierungsstörungen zu nennen. Der hier verwendete Ausdruck "Psoriasis" bezeichnet eine hyperproliferativ-entzündliche Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und polymorphonucleäre Leukozyteninfiltration in die Epidermis, die eine Zunahme des Basalzellzyklus bedingt, gekennzeichnet. Zusätzlich sind hyperkeratotische und parakeratotische Zellen anwesend. Der Ausdruck "Keratosis", "Basalzellkarzinome", "Plattenepithelkarzinome" und "Keratinisierungsstörungen" bezieht sich auf hyperproliferative Hauterkrankungen, bei denen der Regulierungsmechanismus für die Proliferation und Differenzierung der Hautzellen unterbrochen ist.

Die Verbindungen der Formel I sind wirksam als Antagonisten der Hauthyperproliferation, d. h. als Mittel, die die Hyperproliferation menschlicher Keratinozyten hemmen. Die Verbindungen sind infolgedessen als Mittel zur Behandlung hyperproliferativer Hauterkrankungen wie Psoriasis, Basalzellkarzinomen, Keratinisierungsstörungen und Keratosis geeignet. Zur Behandlung dieser Krankheiten können die Verbindungen der Formel I entweder oral oder topisch appliziert werden, wobei sie entweder alleine in Form der Monotherapie oder in Kombination mit bekannten Wirkstoffen eingesetzt werden können.

Des weiteren zu nennen sind durch chirurgische Maßnahmen wie PTCA (perkutane transluminale coronare Angioplastie) oder Bypass-Operationen ausgelöste hyperproliferative Gefäßerkrankungen wie Stenosen und Gefäßverschlüsse, die auf der Proliferation glatter Muskelzellen beruhen. Wie eingangs erwähnt läßt sich diese Zellproliferation bekanntlich durch HMG-CoA-Reduktaseinhibitoren vom Mevinolintyp, wie Lovastatin, unterdrücken. Aufgrund ihrer inhibitorischen Wirkung auf die Cholesterolbiosynthese sind auch die Verbindungen der allgemeinen Formel I geeignet zur Behandlung und Prophylaxe dieser Erkrankungen, wobei sie entweder alleine oder in Kombination mit bekannten Wirkstoffen, wie z. B. intravenös appliziertes Heparin, vorzugsweise in oraler Applikation Verwendung finden können.

Eine weitere Einsatzmöglichkeit der erfindungsgemäßen Verbindungen der allgemeinen Formel I ist die Prophylaxe und Behandlung von Gallensteinleiden. Die Gallensteinbildung wird dadurch ausgelöst, daß die Cholesterolkonzentration in der Galle die maximale Löslichkeit des Cholesterols in der Gallenflüssigkeit überschreitet, wodurch es zur Ausfällung des Cholesterols in Form von Gallensteinen kommt. Lipidsenker aus der Klasse der Fibrate führen zu einer erhöhten Ausscheidung von Neutralsteroiden über die Galle und erhöhen die Neigung zur Gallensteinbildung.

Im Gegensatz dazu führen Cholesterolbiosynthesehemmer wie Lovastatin oder Pravastatin zu keiner erhöhten Gallensteinbildung, sondern können im Gegenteil eine Reduktion der Cholesterolkonzentration in der Galle bewirken und damit den sogenannten lithogenen Index, ein Maß für die Wahrscheinlichkeit der Gallensteinbildung, vermindern. Dies ist beschrieben in Gut 31, 348-350 [1990] sowie in Z. Gastroenterol. 29, 242-245 [1991].

Darüber hinaus ist in Gastroenterology 102, No. 4, Pt. 2, A 319 [1992] die Wirksamkeit von Lovastatin bei der Auflösung von Gallensteinen, insbesondere in Kombination mit Ursodeoxycholsäure beschrieben. Aufgrund ihrer Wirkungsweise sind die Verbindungen der allgemeinen Formel I deshalb auch für die Prophylaxe und Behandlung von Gallensteinleiden von Bedeutung. Sie können dabei entweder allein oder in Kombination mit bekannten Therapien wie beispielsweise der Behandlung mit Ursodeoxycholsäure oder der Schockwellenlithotripsie vorzugsweise in oraler Applikation Verwendung finden.

Schließlich sind die Verbindungen der allgemeinen Formel I geeignet zur Therapie von Infektionen durch pathogene Pilze wie z. B. Candida albicans, Aspergillus niger, Trichophyton mentagrophytes, Penicillium sp., Cladosporium sp. und andere. Wie bereits eingangs erwähnt ist das Endprodukt der Sterolbiosynthese im Pilzorganismus nicht Cholesterol, sondern das für die Integrität und Funktion der Pilzzellmembranen essentielle Ergosterol. Die Inhibierung der Ergosterolbiosynthese führt deshalb zu Wachstumsstörungen und gegebenenfalls zur Abtötung der Pilzorganismen.

Zur Behandlung von Mykosen können die Verbindungen der allgemeinen Formel I entweder oral oder topisch appliziert werden. Dabei können sie entweder alleine oder in Kombination mit bekannten antimykotischen Wirkstoffen eingesetzt werden, insbesondere mit solchen, die in andere Stufen der Sterolbiosynthese eingreifen, wie beispielsweise den Squalen-Epoxidasehemmern Terbinafin und Naftifin oder den Lanosterol-14$\alpha$-Demethylaseinhibitoren vom Azol-Typ wie beispielsweise Ketoconazol und Fluconazol.

Eine weitere Verwendungsmöglichkeit der Verbindungen der allgemeinen Formel I betrifft die Anwendung in der Geflügelhaltung. Die Senkung des Cholesterolgehaltes von Eiern durch Verabreichung des HMG-CoA-Reduktaseinhibitors Lovastatin an Legehennen ist beschrieben (FASEB Journal 4, A 533, Abstracts 1543 [1990]). Die Erzeugung cholesterolarmer Eier ist von Interesse, da die Cholesterolbelastung des Körpers durch Eier mit reduziertem Cholesterolgehalt ohne eine Änderung der Ernährungsgewohnheiten vermindert werden kann. Aufgrund ihrer inhibitorischen Wirkung auf die Cholesterolbiosynthese können die Verbindungen der allgemeinen Formel I auch in der Geflügelzucht zur Erzeugung cholesterolarmer Eier Verwendung finden, wobei die Substanzen vorzugsweise als Zusatz zum Futter verabreicht werden.

Die biologische Wirkung von Verbindungen der allgemeinen Formel I wurde nach folgenden Methoden bestimmt:

I. Messung der Hemmung des $^{14}C$-Acetat-Einbaus in die mit Digitonin fällbaren Steroide:

Methode:

Humane Hepatoma-Zellen (HEP-G2) werden nach 3-tägiger Anzucht für 16 Stunden in cholesterolfreiem Medium stimuliert. Die zu testenden Substanzen (gelöst in Dimethylsulfoxid, Endkonzentration 0,1%) werden während dieser Stimulationsphase zugesetzt. Anschließend wird nach Zugabe von 200 $\mu$Mol/l 2-$^{14}C$-Acetat für weitere zwei Stunden bei 37°C im Brutschrank weiterinkubiert.

Nach Ablösung der Zellen und Verseifen der Sterolester werden nach Extraktion Sterole mit Digitonin zur Fällung gebracht. Das in digitoninfällbare Sterole eingebaute $^{14}C$-Acetat wird durch Szintillationsmessung bestimmt.

Die Untersuchung der Hemmwirkung wurde bei Testkonzentrationen von $10^{-7}$ Mol/l und $10^{-8}$ Mol/l durchgeführt. Beispielhaft werden die Testergebnisse der folgenden Verbindungen A bis AG der allgemeinen Formel I bei diesen Testkonzentrationen angegeben:

A = 1-(4-Chlorbenzoyl)-4-[4-(2-oxazolin-2-yl)-benzyliden]piperidin
B = 1-(4-Chlorbenzoyl)-4-[4-(4,5-dihydro-6H-oxazin-2-yl)benzyliden]piperidin
C = 4-[4-(2-Oxazolin-2-yl)benzyliden]-1-(4-trifluormethylbenzoyl)piperidin
D = 1-(4-Chlor-3-methylbenzoyl)-4-[4-(2-oxazolin-2-yl)benzyliden]piperidin
E = 1-(4-Fluorbenzoyl)-4-[4-(2-oxazolin-2-yl)benzyliden]piperidin
F = 1-(5-Chlor-2-thienoyl)-4-[4-(2-oxazolin-2-yl)benzyliden]piperidin
G = 1-Cyclohexancarbonyl-4-[4-(2-oxazolin-2-yl)benzyliden]piperidin.
H = 4-[4-(2-Oxazolin-2-yl)benzyl]-1-(4-trifluormethylbenzoyl)piperidin
I = 1-(4-Chlorbenzoyl)-4-[4-(2-oxazolin-2-yl)-benzyl]piperidin
K = 1-(4-Chlorbenzoyl)-3-[4-(2-oxazolin-2-yl)-benzyliden]pyrrolidin
L = 1-(4-Chlorbenzoyl)-4-[2-fluor-4-(2-oxazolin-2-yl)benzyliden]piperidin

M = 1-(4-Chlorbenzoyl)-4-[3-methyl-4-(2-oxazolin-2-yl)benzyliden]piperidin
N = 1-(4-Chlorbenzolsulfonyl)-4-[4-(2-oxazolin-2-yl)benzyliden]piperidin
O = 1-(4-Chlorbenzolsulfonyl)-4-[4-(2-imidazolin-2-yl)benzyliden]piperidin
P = 1-(4-Chlorbenzoyl)-4-[4-(2-thiazolin-2-yl)benzyl]piperidin
Q = 1-(4-Chlorbenzoyl)-4-[4-(S-5-methyl-2-oxazolin-2-yl)benzyliden]piperidin
R = 1-(4-Chlorbenzoyl)-4-[4-(R-4-methyl-2-oxazolin-2-yl)benzyliden]piperidin
S = 1-(4-Chlorbenzoyl)-4-[4-(5-phenyl-2-oxazolin-2-yl)benzyliden]piperidin
T = 1-(4-Chlorbenzoyl)-4-[4-(5-diethylaminomethyl-2-oxazolin-2-yl)benzyliden]piperidin
U = 1-(4-Chlorbenzoyl)-4-[4-(4-hydroxmethyl-2-oxazolin-2-yl)benzyliden]piperidin
V = 4-[4-(S-4-Benzyl-2-oxazolin-2-yl)benzyliden]-1-(4-chlorbenzoyl)piperidin
W = 1-(4-Chlorbenzoyl)-4-[4-(2-oxazolin-2-yl)-α-methylbenzyliden]piperidin
X = 1-(5-Chlor-2-thienoyl)-4-[4-(2-oxazolin-2-yl)benzyliden]piperidin
Y = 1-(4-Cyanobenzoyl)-4-[4-(2-oxazolin-2-yl)benzyliden]piperidin
Z = 4-[4-(2-Oxazolin-2-yl)benzyliden]-1-(pentafluorbenzoyl)piperidin
AA = 1-Benzoyl-4-[4-(2-oxazolin-2-yl)benzyliden]piperidin
AB = 1-(4-tert.-Butylbenzoyl)-4-[4-(2-oxazolin-2-yl)benzyliden]piperidin
AC = 1-(4-Methoxybenzoyl)-4-[4-(2-oxazolin-2-yl)benzyliden]piperidin
AD = 1-(4-Brombenzoyl)-4-[4-(2-oxazolin-2-yl)benzyliden]piperidin
AE = 1-(4-Nitrobenzoyl)-4-[4-(2-oxazolin-2-yl)benzyliden]piperidin
AF = 1-(4-Chlorphenylacetyl)-4-[4-(2-oxazolin-2-yl)benzyliden]piperidin
AG = 1-(1-Naphthylcarbonyl)-4-[4-(2-oxazolin-2-yl)benzyliden]piperidin

Die Prozentwerte, um die die obigen Verbindungen den $^{14}C$-Acetat-Einbau hemmen, sind in der folgenden Tabelle angegeben:

| Mol/l | $10^{-7}$ | $10^{-8}$ |
|---|---|---|
| A | -81 | -58 |
| B | -77 | -51 |
| C | -90 | -74 |
| D | -88 | -55 |
| E | -81 | -36 |
| F | -84 | -55 |
| G | -80 | -45 |
| H | -86 | -62 |
| I | -89 | -75 |
| K | -90 | -56 |
| L | -83 | -52 |
| M | -76 | -32 |
| N | -86 | -63 |
| O | -45 | - 1 |
| P | -14 | - 5 |
| Q | -89 | -44 |
| R | -88 | -57 |
| S | -24 | 0 |
| T | -57 | -29 |

| | | |
|---|---|---|
| U | -60 | -21 |
| V | -29 | -15 |
| W | -77 | -35 |
| X | -84 | -35 |
| Y | -74 | -51 |
| Z | -45 | - 8 |
| AA | -70 | -25 |
| AB | -18 | - 6 |
| AC | -25 | -16 |
| AD | -85 | -44 |
| AE | -71 | -22 |
| AF | -63 | -22 |
| AG | -67 | -30 |

Wie eingangs erwähnt, sind in der Literatur vereinzelt Inhibitoren des Enzyms 2,3-Epoxisqualen-Lanosterol-Cyclase beschrieben, die sich jedoch strukturell sehr stark von den erfindungsgemäßen Verbindungen der Formel I unterscheiden. Die zu den Verbindungen der allgemeinen Formel I strukturell nächstverwandten Verbindungen sind in der EP 0 468 457 A1 beschrieben. Zum Vergleich wurde deshalb das Beispiel 1 dieser Publikation nach der oben beschriebenen Bestimmungsmethode in Testkonzentrationen von $10^{-5}$ Mol/l und $10^{-6}$ Mol/l geprüft. Die dabei gefundenen Hemmwerte von 41% bzw. 13% zeigen, daß diese Verbindungen den erfindungsgemäßen Verbindungen der allgemeinen Formel I deutlich unterlegen sind.

II. Messung der in-vivo-Wirkung an der Ratte nach oraler Gabe

Die Inhibierung des Enzyms 2,3-Epoxisqualen-Lanosterol-Cyclase bewirkt eine Erhöhung der 2,3-Epoxisqualenspiegel in Leber und Plasma. Die Menge an gebildetem 2,3-Epoxisqualen dient daher als direktes Maß für die Wirkstärke am Ganztier. Die Bestimmung wird nach folgender Methode durchgeführt: Männlichen Wistar-Ratten (160-190 g Körpergewicht) wird die in 1,5%iger wässriger Methylcellulose suspendierte Prüfsubstanz via Schlundsonde appliziert. 5 Stunden nach Applikation wird Blut retroorbital aus dem Venenplexus gewonnen. Plasma wird nach der Methode von Bligh und Dyer (Canad. J. Biochem. Physiol. 37, 912, [1959]) aufgearbeitet, über eine Vorsäule gereinigt und danach mittels HPLC analysiert. Die erhaltenen Peaks werden über Eichsubstanzen identifiziert und quantifiziert. Ein interner Standard dient der Überprüfung der Reproduzierbarkeit der Ergebnisse.

Die Untersuchungen wurden mit Konzentrationen von 0,1 bzw. 1,0 mg/kg durchgeführt. In der folgenden Tabelle sind beispielhaft die Ergebnisse für die vorstehend erwähnten Substanzen A bis M zusammengefaßt:

2,3-Epoxisqualen-Konzentration (µg/ml) im Plasma (Ratte)

| mg/kg | 0,1 | 1,0 |
|---|---|---|
| A | 0,71 | 3,75 |
| B | 0,46 | 3,14 |
| C | 0,91 | 5,90 |
| D | 0,78 | 3,35 |
| E | 0,67 | 6,09 |
| F | 0,00 | 2,00 |
| G | 0,36 | 0,31 |
| H | 1,70 | 9,15 |
| I | 3,41 | 8,41 |
| K | 0,0 | 4,14 |
| L | 0,44 | 6,46 |
| M | 0,78 | 6,53 |

Bei den Kontrolltieren treten unter den Versuchsbedingungen keine meßbaren 2,3-Epoxisqualenspiegel auf.

Von keinem der in der Literatur beschriebenen Inhibitoren des Enzyms 2,3-Epoxisqualen-Lanosterol-Cyclase ist bisher eine Inhibierung der Cholesterolbiosynthese am Ganztier beschrieben.

III. Lipidsenkung am normolipämischen Goldhamster

Männliche Goldhamster werden für 12 Tage ad lib. mit cholesterolfreier Hamsterdiät gefüttert. Die zu testende Substanz wird dem Futter in Konzentrationen von 0,01 bis 0,10% zugemischt. Am Ende der Versuchsperiode werden das Gesamtcholesterol, die HDL-Fraktion sowie die VLDL + LDL-Fraktion nach Standardmethoden bestimmt, wobei zum Vergleich eine ohne Testsubstanz gefütterte Kontrollgruppe herangezogen wird.

Geprüft wurde die lipidsenkende Wirkung der vorstehend erwähnten Verbindung A. Das Ergebnis ist in der folgenden Tabelle zusammengefaßt:

| Konzentration | Gesamt-Cholesterol | VLDL + LDL | HDL |
|---|---|---|---|
| 0,01% | -19,8% | -25,0% | -12,4% |
| 0,03% | -26,3% | -31,2% | -17,5% |
| 0,10% | -25,8% | -36,6% | -13,9% |

IV. Bestimmung des lithogenen Index am normolipämischen Goldhamster

Der lithogene Index ist ein Maß für die Neigung zur Gallensteinbildung und ist definiert als der Quotient aus der maximalen Gleichgewichts-Löslichkeit des Cholesterols bei der vorliegenden Gallensäure- und Phospholipidkonzentration und des aktuellen Cholesterolgehalts in Mol-%. Er wurde bestimmt nach der Methode von Carey und Small, beschrieben in J. Clin. Investig. 61, 998-1026 [1978]. Je höher der lithogene Index ist, desto höher ist die Wahrscheinlichkeit der Gallensteinbildung. Die Bestimmung des lithogenen Index wurde wie folgt durchgeführt:
Männliche Goldhamster werden für 20 Tage ad lib. mit cholesterolfreier Hamsterdiät gefüttert. Die zu testende Substanz wird dem Futter in Konzentrationen von 0,01 bis 0,1% zugemischt. Am Ende der Versuchsperiode werden im Serum Gesamtcholesterol, die HDL-Fraktion sowie die VLDL + LDL-Fraktion und in der Galle die Gallensäuren, das Cholesterol sowie die Phospholipide nach Standardmethoden bestimmt.

Getestet wurde die vorstehend beschriebene Verbindung A. Bestimmt wurde dabei die prozentuale Abnahme des lithogenen Index, verglichen mit der Kontrolle. Das Ergebnis ist in der folgenden Tabelle zusammengefaßt.

| Konzentration | lithogener Index |
|---|---|
| 0,01 % | -24 % |
| 0,03 % | -10 % (n.s.) |
| 0,1 % | -28 % |
| n.s. = nicht signifikant | |

V. Hemmung der Zellproliferation

Normale humane epidermale Keratinozyten (NHEK) werden in Keratinozyten-Wachstumsmedium (Gibco) in einer befeuchteten, 5% $CO_2$ enthaltenden Atmosphäre unter sterilen Bedingungen gezüchtet. Zellen der dritten Passage werden mit einer Dichte von 12000 Zellen/ml ausgesät.

Nach 24 Stunden wird die Testsubstanz zum Medium gegeben und die Zahl der Zellen nach weiteren 48 Stunden bestimmt. Die Ergebnisse wurden im Vergleich zu einer Kontrolle berechnet und sind als prozentuale Abnahme der Zellzahlen angegeben.

Geprüft wurde die proliferationshemmende Wirkung der vorstehend erwähnten Verbindung A im Vergleich zu Simvastatin in Konzentrationen von $10^{-6}$ bis $10^{-10}$ Mol/l.

Das Ergebnis ist in der folgenden Tabelle zusammengefaßt:

| | $10^{-6}$ | $10^{-7}$ | $10^{-8}$ | $10^{-9}$ | $10^{-10}$ |
|---|---|---|---|---|---|
| A | -72% | -73% | -70% | -48% | -16 |
| Simvastatin | -68% | -51% | -20% | -21% | - 7 |

VI. Bestimmung der fungistatischen Wirkung

Die fungistatische Wirkung wurde über den Reihenverdünnungstest (Mikrotitersystem) bestimmt. Als Nährmedium diente dabei Sabouraud-Bouillon. Das Inoculum betrug ca. $10^4$ bis $10^5$ KBE/ml (KBE = koloniebildende Einheiten); die Bebrütungszeit betrug 2 bis 4 Tage bei 26 °C.

Bestimmt wurde die minimale Hemmkonzentration (MHK), d. h. die niedrigste Konzentration, die sichtbares Wachstum nicht mehr zuließ, sowie die niedrigste Konzentration, bei der es im Vergleich mit einer Kontrolle zu einer Wachstumsminderung des Testkeims kam.

Geprüft wurde die vorstehend erwähnte Verbindung I. Die Ergebnisse sind in der folgenden Tabelle dargestellt.

| | MHK µg/ml | Wachstumsminderung µg/ml |
|---|---|---|
| Candida albicans ATCC 10231 | 128 | 64 |
| Rhodotorula rubra 49 | 128 | 32 |
| Sacc. carlsbergensis ATCC 9080 | 128 | 64 |
| Aspergillus niger ATCC 16404 | >1024 | 128 |
| Trichophyton mentagrophytes ATCC 9129 | 512 | |
| Penicillium notatum CBS 19746 | 128 | 64 |

Die Verbindungen A bis I zeigten sich in der kurativen Dosierung als untoxisch. Beispielsweise zeigten die Verbindungen A, H und I nach oraler Applikation von 100 mg/kg, einmal täglich über 4 Tage, an der Maus keine toxischen Wirkungen.

Zur pharmazeutischen Anwendung lassen sich die Verbindungen der allgemeinen Formel I in an sich bekannter Weise in die üblichen pharmazeutischen Zubereitungsformen für die orale, rektale und topische Verabreichung einarbeiten.

Formulierungen für die orale Verabreichung umfassen beispielsweise Tabletten, Dragées und Kapseln, für die rektale Verabreichung kommen vorzugsweise Suppositorien in Betracht. Die Tagesdosis beträgt zwischen 1 und 1200 mg für einen Menschen mit 60 kg Körpergewicht, bevorzugt ist jedoch eine Tagesdosis von 5 bis 100 mg für einen Menschen mit 60 kg Körpergewicht. Die Tagesdosis wird

vorzugsweise in 1 bis 3 Einzelgaben aufgeteilt.

Bei topischer Anwendung können die Verbindungen in Zubereitungen, die etwa 1 bis 1000 mg, insbesondere 10 bis 300 mg Wirkstoff pro Tag enthalten, verabreicht werden. Die Tagesdosis wird vorzugsweise in 1 bis 3 Einzelgaben aufgeteilt.

Topische Formulierungen umfassen Gele, Cremes, Lotionen, Salben, Puder, Aerosole und andere herkömmliche Formulierungen zur Anwendung von Heilmitteln auf der Haut. Die Wirkstoffmenge für die topische Anwendung beträgt 1 bis 50 mg pro Gramm Formulierung, vorzugsweise jedoch 5 bis 20 mg pro Gramm Formulierung. Neben der Anwendung auf der Haut können die topischen Formulierungen der vorliegenden Erfindung auch angewandt werden bei der Behandlung von Schleimhäuten, die der topischen Behandlung zugänglich sind. Beispielsweise können die topischen Formulierungen auf die Schleimhäute des Mundes, des unteren Colons und andere aufgebracht werden.

Zur Anwendung in der Geflügelzucht zur Erzeugung cholesterolarmer Eier werden die Wirkstoffe der allgemeinen Formel I den Tieren nach den üblichen Methoden als Zusatz zu geeigneten Futtermitteln verabreicht. Die Konzentration der Wirkstoffe im Fertigfutter beträgt normalerweise 0,01 bis 1%, vorzugsweise jedoch 0,05 bis 0,5%.

Die Wirkstoffe können als solche dem Futter zugesetzt werden. So enthalten die erfindungsgemäßen Futtermittel für Legehennen neben dem Wirkstoff und gegebenenfalls neben einer üblichen Vitamin-Mineral-Mischung beispielsweise Mais, Sojabohnenmehl, Fleischmehl, Futterfett und Sojaöl. Zu diesem Futter wird eine der eingangs erwähnten Verbindungen der Formel I als Wirkstoff in einer Konzentration von 0,01 bis 1%, vorzugsweise jedoch 0,05 bis 0,5% zugemischt.

Die nachfolgenden Beispiele dienen der näheren Erläuterung der Erfindung. Die angegebenen $R_f$-Werte wurden an Fertigplatten der Firma E. Merck, Darmstadt bestimmt und zwar an:

a) Aluminiumoxid F-254 (Typ E)

b) Kieselgel 60 F-254.

Beispiele zur Herstellung der Ausgangsmaterialien:

Beispiel A

4-Cyano-benzylphosphonsäurediethylester

98 g 4-(Brommethyl)benzonitril und 300 ml Triethylphosphit werden auf 140°C erwärmt bis zum Einsetzen der Reaktion. Anschließend wird noch zwei Stunden bei einer Badtemperatur von 150-160°C zum Rückfluß erhitzt, das gebildete Ethylbromid abdestilliert, noch eine weitere Stunde auf 150°C erwärmt und anschließend Triethylphosphit im Vakuum abdestilliert. Der Rückstand wird im Eisbad mit 250 ml Cyclohexan versetzt, die gebildeten Kristalle abgesaugt und mit 150 ml Cyclohexan gewaschen. Man erhält 125,6 g (99,2 % der Theorie) der Titelverbindung vom Schmelzpunkt 41,5-43°C.

Auf diesselbe Weise wurde erhalten:

a) 3-Cyano-benzylphosphonsäurediethylester
aus 3-(Brommethyl)benzonitril und Triethylphosphit.
Farbloses Öl.

b) 4-Methoxycarbonyl-3-methyl-benzylphosphonsäurediethylester
aus 4-Methoxycarbonyl-3-methyl-benzylbromid (J.Med. Chem. 33, 2437-2451 [1990]) und Triethylphosphit.
Farbloses Öl.

c) 2-Fluor-4-methoxycarbonyl-benzylphosphonsäurediethylester
aus 2-Fluor-4-methoxycarbonyl-benzylbromid (hergestellt aus 3-Fluor-4-methyl-benzoesäuremethylester und Brom in Gegenwart von Benzoylperoxid in Tetrachlorkohlenstoff unter Bestrahlung mit einer 1000 Watt-Wolframlampe) und Triethylphosphit.
Farbloses Öl.

d) 2-Brom-4-methoxycarbonyl-benzylphosphonsäurediethylester
aus 2-Brom-4-methoxycarbonyl-benzylbromid (hergestellt aus 3-Brom-4-methyl-benzoesäuremethylester und Brom in Gegenwart von Benzoylperoxid in Tetrachlorkohlenstoff unter Bestrahlung mit einer 1000 Watt-Wolframlampe) und Triethylphosphit.
Farbloses Öl.

e) 2-Methoxy-4-methoxycarbonyl-benzylphosphonsäurediethylester
aus 2-Methoxy-4-methyl-benzylbromid (hergestellt aus 3-Methoxy-4-methyl-benzoesäuremethylester und Brom in Gegenwart von Benzoylperoxid in Tetrachlorkohlenstoff unter Bestrahlung mit einer 1000 Watt-Wolframlampe) und Triethylphosphit.

Farbloses Öl.

Beispiel B

1-(4-Cyanophenyl)ethylphosphonsäurediethylester

Zu einer Lösung von 7,5 g 4-Cyano-benzylphosphonsäurediethylester in 60 ml Tetrahydrofuran werden bei -50 °C unter Rühren 18,7 ml einer 1,6 M Lösung von n-Butyllithium in n-Hexan zugetropft, anschließend wird 25 Minuten bei -40 °C gerührt und danach bei -40 °C 4,7 g Methyljodid in 20 ml Tetrahydrofuran zugetropft. Nach Rühren über Nacht bei Raumtemperatur wird eingedampft, in Essigsäureethylester aufgenommen, mit Wasser gewaschen, die organische Phase getrocknet und eingedampft. Nach Reinigung durch Säulenchromatographie an Kieselgel (Petrolether/2-Propanol = 6:1 bis 2:1, v:v) erhält man 5,7 g der Titelverbindung als farbloses Öl.
$R_f$-Wert: 0,52 (Kieselgel, Petrolether/2-Propanol = 3:1, v:v).

Beispiel C

N-(4-Chlorbenzoyl)-4-piperidon

Zu einer Suspension von 80,6 g pulverförmigen 4-Piperidon-hydrochloridhydrat in 1 l Tetrahydrofuran werden unter Rühren nacheinander 87,5 g 4-Chlorbenzoylchlorid und eine auf 5 °C abgekühlte Lösung von 276 g Kaliumcarbonat in 552 ml Wasser zugegeben. Es wird 45 Minuten bei Raumtemperatur weiter gerührt. Danach wird die organische Phase abgetrennt, die wässrige Phase noch zweimal mit Essigsäureethylester extrahiert, die organischen Phasen vereinigt, getrocknet und eingedampft. Der Rückstand wird in Essigsäureethylester gelöst und mit Petrolether versetzt. Man erhält 88,6 g der Titelverbindung vom Schmelzpunkt 61-63 °C.

Auf dieselbe Weise wurden erhalten:

a) N-(4-Chlorbenzolsulfonyl)-4-piperidon
aus 4-Piperidon-hydrochloridhydrat und 4-Chlorbenzolsulfochlorid.
Schmelzpunkt: 158-160 °C.

b) N-(4-Methylbenzoyl)-4-piperidon
aus 4-Methylbenzoylchlorid und 4-Piperidon-hydrochloridhydrat.
Farbloses Harz.

c) N-(4-Dihydrocinnamoyl)-4-piperidon
aus 4-Dihydrozimtsäurechlorid und 4-Piperidon-hydrochloridhydrat.
Farblose Kristalle.

d) N-(4-Chlorcinnamoyl)-4-piperidon
aus 4-Chlorzimtsäurechlorid und 4-Piperidon-hydrochloridhydrat.
Farbloses Harz.

e) N-Hexanoyl-4-piperidon
aus Hexansäurechlorid und 4-Piperidon-hydrochloridhydrat.
Farbloses Öl.

f) N-Pivaloyl-4-piperidon.
aus Pivaloylchlorid und 4-Piperidon-hydrochloridhydrat. Farblose Kristalle.

g) N-Cyclohexancarbonyl-4-piperidon
aus Cyclohexancarbonsäurechlorid und 4-Piperidon-hydrochloridhydrat.
Farblose Kristalle.

Beispiel D

1-(4-Chlorbenzoyl)-4-(4-cyano-benzyliden)piperidin

Zu einer aus 3,34 g Diisopropylamin in 20 ml Tetrahydrofuran und 19 ml einer 1,6 M Lösung von n-Butyllithium in n-Hexan hergestellten Lösung von Lithiumdiisopropylamid werden bei -50 °C 7,6 g 4-Cyano-benzylphosphonsäurediethylester in 50 ml Tetrahydrofuran zugetropft. Nach 20 minütigem Rühren bei dieser Temperatur wird eine Lösung von 7,13 g N-(4-Chlorbenzoyl)-4-piperidonzugetropft. Man läßt auf Raumtemperatur erwärmen, gießt nach weiteren zwei Stunden auf Eis und extrahiert mit Essigsäureethylester. Die organische Phase wird getrocknet und eingedampft. Man erhält nach Umkristallisieren aus

Essigsäureethylester 7,6 g der Titelverbindung vom Schmelzpunkt 134-135,5 ° C.

Auf dieselbe Weise wurden erhalten:

a) 1-(4-Chlorbenzoyl)-4-(3-cyano-benzyliden)piperidin
aus 3-Cyano-benzylphosphonsäurediethylester und N-(4-Chlorbenzoyl)-4-piperidon.
Farbloser Schaum.

b) 1-(4-Chlorbenzolsulfonyl)-4-(4-cyano-benzyliden)piperidin
aus 4-Cyano-benzylphosphonsäurediethylester und N-(4-Chlorbenzolsulfonyl)-4-piperidon.
Schmelzpunkt: 129-130 ° C.

c) 1-(4-Chlorbenzoyl)-4-(4-cyano-$\alpha$-methyl-benzyliden)piperidin
aus 1-(4-Cyanophenyl)ethylphosphonsäurediethylester und N-(4-Chlorbenzoyl)-4-piperidon.
Schmelzpunkt: 134 ° C.

d) 1-Benzyl-3-(4-cyano-benzyliden)piperidin
aus 4-Cyano-benzylphosphonsäurediethylester und N-Benzyl-3-piperidon.
Schmelzpunkt: 113 ° C.

e) 1-(4-Chlorbenzoyl)-4-(4-methoxycarbonyl-3-methyl-benzyliden)piperidin
aus 4-Methoxycarbonyl-3-methyl-benzylphoshonsäurediethylester und N-(4-Chlorbenzoyl)-4-piperidon.
Schmelzpunkt: 142-144 ° C.

f) 1-(4-Chlorbenzoyl)-4-(2-fluor-4-methoxycarbonyl-benzyliden)piperidin
aus 2-Fluor-4-methoxycarbonyl-benzylphosphonsäurediethylester und N-(4-Chlorbenzoyl)-4-piperidon.
Schmelzpunkt: 110-112 ° C.

g) 4-(2-Brom-4-methoxycarbonyl-benzyliden)-1-(4-chlorbenzoyl)piperidin
aus 2-Brom-4-methoxycarbonyl-benzylphosphonsäurediethylester und N-(4-Chlorbenzoyl)-4-piperidon.
Schmelzpunkt: 140-142 ° C.

h) 1-(4-Chlorbenzoyl)-4-(2-methoxy-4-methoxycarbonyl-benzyliden)piperidin
aus 2-Methoxy-4-methoxycarbonyl-benzylphosphonsäurediethylester und N-(4-Chlorbenzoyl)-4-piperidon.
Farbloses Öl.

## Beispiel E

### 1-(4-Chlorbenzoyl)-3-(4-cyano-benzyliden)piperidin

Zu 2,9 g 1-Benzyl-3-(4-cyano-benzyliden)piperidin in 20 ml Methylenchlorid werden 1,8 g Chlorameisensäure-1-chlorethylester in 5 ml Methylenchlorid bei 0 ° C zugetropft und 30 Minuten bei 0 ° C gerührt. Nach 20 Minuten bei Raumtemperatur wird eingedampft, der Rückstand in 20 ml Methanol aufgenommen und 30 Minuten zum Rückfluß erhitzt. Die Reaktionslösung wird eingedampft, der Rückstand mit 100 ml Essigsäureethylester verrieben und abgesaugt. Man erhält 1,9 g 3-(4-Cyanobenzyliden)piperidin-hydrochlorid als farbloses Pulver.

Dieses Produkt und 2,1 g Triethylamin werden in 15 ml Methylenchlorid gelöst und 1,75 g 4-Chlorbenzoylchlorid in 5 ml Methylenchlorid bei Raumtemperatur zugetropft. Nach 30 Minuten bei Raumtemperatur werden 100 ml Methylenchlorid zugegeben, zweimal mit Wasser ausgeschüttelt, mit wasserfreiem Magnesiumsulfat getrocknet, eingedampft und durch Säulenchromatographie gereinigt (Kieselgel, Essigsäureethylester). Man erhält 1,8 g der Titelverbindung als farblose Kristalle vom Schmelzpunkt > 210 ° C.

## Beispiel F

### 1-(4-Chlorbenzoyl)-4-(4-cyano-benzyl)piperidin

8,4 g 1-(4-Chlorbenzoyl)-4-(4-cyano-benzyliden)piperidin werden in 400 ml Toluol unter Zusatz von 2 g Palladium-Mohr 15 Minuten bei Raumtemperatur hydriert (5 bar). Nach Zugabe von 1 g Palladium-Mohr wird 2,5 Stunden weiter hydriert, nochmals 1 g Katalysator zugegeben und nochmals eine Stunde weiter hydriert. Man erhält 7,9 g (93,3 % der Theorie) der Titelverbindung als farbloses Pulver vom Schmelzpunkt 137-139 ° C.

Beispiel G

1-(4-Chlorbenzoyl)-4-(4-ethoxyimidoyl-benzyliden)piperidin-hydrochlorid

In eine Suspension von 10,8 g 1-(4-Chlorbenzoyl)-4-(4-cyano-benzyliden)piperidin in 70 ml wasserfreiem Ethanol werden unter Eiskühlung 40,7 g Chlorwasserstoff eingeleitet. Nach 16 Stunden Rühren bei Raumtemperatur wird das Lösungsmittel bei einer Badtemperatur von 30°C verdampft, der Rückstand erneut in 35 ml Ethanol gelöst und anschließend wieder eingedampft. Der Rückstand wird mit Essigsäureethylester verrieben. Man erhält 13,2 g der Titelverbindung als farbloses Pulver.

Auf dieselbe Weise wurden erhalten:

a) 1-(4-Chlorbenzoyl)-4-(3-methoxyimidoyl-benzyliden)piperidin-hydrochlorid
aus 1-(4-Chlorbenzoyl)-4-(3-cyano-benzyliden)piperidin und Methanol.
Farbloses Harz.

b) 1-(4-Chlorbenzolsulfonyl)-4-(4-methoxyimidoyl-benzyliden)piperidin-hydrochlorid
aus 1-(4-Chlorbenzolsulfonyl)-4-(4-cyano-benzyliden)piperidin und Methanol.
Farbloses Harz.

c) 1-(4-Chlorbenzoyl)-4-(4-ethoxyimidoyl-α-methyl-benzyliden)piperidin-hydrochlorid
aus 1-(4-Chlorbenzoyl)-4-(4-cyano-α-methyl-benzyliden)piperidin und Ethanol.
Farbloser Schaum.

d) 1-(4-Chlorbenzoyl)-3-(4-ethoxyimidoyl-benzyliden)piperidin-hydrochlorid
aus 1-(4-Chlorbenzoyl)-3-(4-cyano-benzyliden)piperidin.
Farblose Kristalle.

e) 1-(4-Chlorbenzoyl)-4-(4-ethoxyimidoyl-benzyl)piperidin-hydrochlorid
aus 1-(4-Chlorbenzoyl)-4-(4-cyano-benzyl)piperidin.
Schmelzpunkt: 236°C.

Beispiel H

4-Ethoxyimidoyl-benzylphosphonsäurediethylester-hydrochlorid

5,6 g 4-Cyano-benzylphosphonsäurediethylester und 24 g Chlorwasserstoff in 35 ml wasserfreiem Ethanol werden 17 Stunden bei Raumtemperatur gerührt. Anschließend wird die Reaktionslösung eingedampft und der Rückstand jeweils nach Zugabe von Ethanol noch zweimal zur Trockne eingedampft. Die erhaltenen farblosen Kristalle werden ohne Reinigung weiter umgesetzt.

Beispiel I

4-(2-Oxazolin-2-yl)benzylphosphonsäurediethylester

Das unter Beispiel H erhaltene Produkt wird in 50 ml Ethanol gelöst. Nach Zugabe von 1,8 g Ethanolamin und 4,1 g Triethylamin wird die Reaktionslösung zwei Stunden zum Rückfluß erhitzt, anschließend bei 50°C eingedampft und der Rückstand in 100 ml Essigsäureethylester gelöst. Die Lösung wird dreimal mit Wasser gewaschen, getrocknet und eingedampft. Man erhält 4,2 g der Titelverbindung vom Schmelzpunkt 65°C.

Beispiel K

4-[4-(2-Oxazolin-2-yl)benzyliden]-1-tritylpiperidin

Zu 2,97 g 4-(2-Oxazolin-2-yl)benzylphosphonsäurediethylester werden bei -60°C zunächst 6,25 ml einer 1,6 M Lösung von n-Butyllithium in Hexan und nach 20 Minuten Rühren 3,4 g N-Trityl-4-piperidon (hergestellt aus 4-Piperidon-hydrochloridhydrat und Tritylchlorid) in 20 ml Tetrahydrofuran zugetropft. Es wird 25 Minuten bei -60°C gerührt. Anschließend läßt man die Reaktionslösung auf Raumtemperatur erwärmen und rührt weitere 1,5 Stunden bei dieser Temperatur. Danach wird die Reaktionslösung in 150 ml Eiswasser eingerührt, dreimal mit 150 ml Essigsäureethylester extrahiert, die organische Phase getrocknet und eingedampft. Der Rückstand wird durch Säulenchromatographie an Kieselgel (Petrolether/Essigsäureethylester = 1:1, v:v) gereinigt. Man erhält 1,7 g der Titelverbindung vom Schmelzpunkt 110-115°C.

Analog wurde dargestellt:

a) 4-[4-(2-Oxazolin-2-yl)benzyliden]-1-trityl-hexahydroazepin
aus 4-(2-Oxazolin-2-yl)benzylphosphonsäurediethylester und 1-Trityl-hexahydroazepin-4-on (hergestellt aus Hexahydroazepin-4-on und Tritylchlorid).
Farbloser Schaum.
$R_f$-Wert: 0,40 (Kieselgel; Petrolether/Essigsäureethylester = 1:1, v:v).

b) 1-tert.Butyloxycarbonyl-4-[4-(2-oxazolin-2-yl)benzyliden]piperidin
aus 4-(2-Oxazolin-2-yl)benzylphosphonsäurediethylester und N-tert.Butyloxycarbonyl-4-piperidon (hergestellt aus 4-Piperidon-hydrochloridhydrat und Pyrokohlensäure-di-tert.-butylester.
Schmelzpunkt: 74°C.

Beispiel L

1-tert.Butyloxycarbonyl-4-[4-(2-oxazolin-2-yl)benzyl)piperidin

2,4 g (7 mMol) 1-tert.Butyloxycarbonyl-4-[4-(2-oxazolin-2-yl)benzyliden]piperidin, gelöst in 70 ml wasserfreiem Ethanol und 20 ml Essigsäureethylester, werden in Gegenwart von 2,5 g Palladium-Kohle (10%-ig) katalytisch hydriert (Raumtemperatur, 20 Minuten, Wasserstoffdruck 50 psi). Der Katalysator wird abgesaugt, das Filtrat eingedampft und der Rückstand ohne Reinigung weiter umgesetzt.

Beispiel M

4-[4-(2-Oxazolin-2-yl)benzyliden]piperidin

1,0 g 4-[4-(2-Oxazolin-2-yl)benzyliden]-1-tritylpiperidin in 20 ml Methylenchlorid werden bei -10°C langsam mit 2 ml Trifluoressigsäure versetzt. Nach einer Stunde Rühren bei -10°C wird die Reaktionsmischung in 150 ml Eiswasser gegossen und sofort mit 6N-Natronlauge auf pH 11,5 gestellt. Nach 10 Minuten Rühren wird zweimal mit je 150 ml Ether extrahiert, die vereinigten organischen Phasen gewaschen, getrocknet und eingedampft. Der Rückstand wird durch Säulenchromatographie an Aluminiumoxid (Methylenchlorid/Methanol = 40:1, v:v) gereinigt. Man erhält 340 mg der Titelverbindung vom Schmelzpunkt 126°C.

Auf dieselbe Weise wurde erhalten:

a) 4-[4-(2-Oxazolin-2-yl)benzyliden]hexahydroazepin
aus 4-[4-(2-Oxazolin-2-yl)benzyliden]-1-trityl-hexahydroazepin und Trifluoressigsäure.
Schmelzpunkt: sintert ab 46°C.

b) 4-[4-(2-Oxazolin-2-yl)benzyl]piperidin
aus 1-tert.Butyloxycarbonyl-4-[4-(2-oxazolin-2-yl)benzyl]piperidin und Trifluoressigsäure.
Schmelzpunkt: 110°C.

Beispiel N

3-[4-(2-Oxazolin-2-yl)benzyliden]pyrrolidin

Zu einer Suspension von 5,36 g Natriumhydrid (55%-ig in Öl) in 50 ml Tetrahydrofuran wird eine Lösung von 5,0 g N-Acetyl-2-pyrrolidon und 5,17 g 4-Cyan-benzaldehyd in 50 ml Tetrahydrofuran bei 5-10°C zugetropft und dann eine Stunde bei 0°C nachgerührt. Überschüssiges Natriumhydrid wird durch Zugabe von etwas Methanol zerstört, das Reaktionsgemisch auf Eis gegossen und mit Eisessig neutralisiert. Der Niederschlag wird abgesaugt, in einem Methylenchlorid-Methanol-Gemisch gelöst, getrocknet und eingeengt. Der Rückstand wird mit Ether verrieben und abgesaugt. Man erhält 1,4 g 3-(4-Cyano-benzyliden)-2-pyrrolidon als farbloses Pulver vom Schmelzpunkt 260°C.

1,36 g dieses Produkts werden in 30 ml Methanol suspendiert. Unter Eiskühlung werden etwa 10 g Chlorwasserstoff eingeleitet, anschließend wird 20 Stunden bei Raumtemperatur gerührt und zwei Tage bei Raumtemperatur stehen gelassen. Nach dem Einengen wird der Rückstand in 40 ml Ethanol aufgenommen, 0,68 g Ethanolamin und 1,52 g Triethylamin zugegeben, das Gemisch zwei Stunden zum Rückfluß erhitzt und anschließend eingeengt. Der Rückstand wird mit einem Wasser/Essigsäureethylestergemisch verrieben und der Niederschlag anschließend abgesaugt und getrocknet. Man erhält 1,5 g (3-[4-(2-Oxazolin-2-yl)-benzyliden]-2-pyrrolidon vom Schmelzpunkt 258-262°C.

Zu 0,49 g dieses Produkts in 10 ml Tetrahydrofuran werden 152 mg Lithiumaluminiumhydrid gegeben und es wird zwei Stunden bei Raumtemperatur gerührt. Nach Zugabe von 0,3 ml Wasser wird eine halbe Stunde bei Raumtemperatur gerührt und der Niederschlag anschließend abgesaugt. Die erhaltene Titelverbindung wird als Lösung weiter umgesetzt.

Beispiel O

1-(4-Chlorbenzoyl)-4-[2-fluor-4-(2-hydroxyethylaminocarbonyl)benzyliden]piperidin

9,0 g 1-(4-Chlorbenzoyl)-4-[2-fluor-4-methoxycarbonyl-benzyliden)piperidin in 150 ml Methanol werden mit einer Lösung von 4,0 g 84%-igem Ätzkali in 100 ml Wasser vesetzt und über Nacht bei Raumtemperatur gerührt. Das Methanol wird verdampft, der Rückstand unter Kühlung mit Wasser versetzt und mit verdünnter Salzsäure angesäuert. Der Niederschlag wird abgesaugt, mit Wasser gewaschen und im Vakuum getrocknet. Man erhält 8,6 g (95% der Theorie) 4-(4-Carboxy-2-fluor-benzyliden)-1-(4-chlorbenzoyl)piperidin vom Schmelzpunkt 170-173 °C.

2,0 g dieses Produkts und 0,88 g N.N'-Carbonyldiimidazol in 50 ml Xylol werden eine Stunde auf 60 °C erwärmt, anschließend werden 0,32 g Ethanolamin zugegeben und es wird drei Stunden bei 160 °C Badtemperatur gerührt. Nach Verdampfen des Lösungsmittels wird der Rückstand mit Wasser versetzt, dreimal mit Methylenchlorid extrahiert, der organische Extrakt getrocknet und verdampft. Der Rückstand wird durch Säulenchromatographie gereinigt (Kieselgel; Essigsäureethylester/Methanol = 10:0,5, v:v). Man erhält 0,4 g der Titelverbindung als farbloses Öl.

$R_f$-Wert: 0,25 (Kieselgel; Essigsäureethylester/Methanol = 10:0,5, v:v).

Auf dieselbe Weise wurden dargestellt:

a) 1-(4-Chlorbenzoyl)-4-[4-(2-hydroxyethylaminocarbonyl)-3-methyl-benzyliden]piperidin
aus 1-(4-Chlorbenzoyl)-4-(4-methoxycarbonyl-3-methyl-benzyliden)piperidin und Ethanolamin.
Farbloses Öl.
$R_f$-Wert: 0,2 (Kieselgel; Essigsäureethylester/Petrolether/Methanol = 10:5:1, v:v:v).
Die Verseifung des Ausgangsesters wurde in der Siedehitze durchgeführt.

b) 4-[2-Brom-4-(2-hydroxyethylaminocarbonyl)benzyliden]-1-(4-chlorbenzoyl)piperidin
aus 4-(2-Brom-4-methoxycarbonyl-benzyliden)-1-(4-chlorbenzoyl)piperidin und Ethanolamin.
Schmelzpunkt: 90-92 °C.

c) 1-(4-Chlorbenzoyl)-4-[4-(2-hydroxyethylaminocarbonyl)-2-methoxy-benzyliden]piperidin
aus 4-(2-Methoxy-4-methoxycarbonyl-benzyliden)-1-(4-chlorbenzoyl)piperidin und Ethanolamin.
Farbloses Öl.

Beispiele zur Herstellung der Endprodukte:

Beispiel 1

1-(4-Chlorbenzoyl)-4-[4-(2-oxazolin-2-yl)benzyliden]piperidin

13,2 g (0,0315 Mol) 1-(4-Chlorbenzoyl)-4-(4-ethoxyimidoyl-benzyliden)piperidin-hydrochlorid werden in 59 ml wasserfreiem Ethanol aufgeschlämmt. Nach Zugabe von 2,64 g (0,042 Mol) Ethanolamin und 6,5 g (0,063 Mol) Triethylamin wird 1,5 Stunden zum Rückfluß erhitzt. Nach Kühlen im Eisbad wird abgesaugt und mit Ethanol gewaschen. Man erhält 10,4 g (88,6% der Theorie) der Titelverbindung vom Schmelzpunkt 181-183 °C.

$^1$H-NMR-Spektrum (200 MHz, $CDCl_3$); Signale bei ppm:
2.3-2.6 (m,4H), 3.3-3.9 (m,4H), 4.05 (t,2H), 4.4 (t,2H), 6.4 (s,1H), 7.2 (d,2H), 7.4 (s,4H), 7.9 (d,2H).

Das Hydrochlorid der Titelverbindung wurde in Ethanol mit ätherischer Salzsäure in der Kälte dargestellt. Es sintert bei 69-71 °C und wird bei 80-81 °C unter Gasentwicklung wieder fest.

Auf dieselbe Weise wurden erhalten:

a) 1-(4-Chlorbenzolsulfonyl)-4-[4-(2-oxazolin-2-yl)benzyliden]piperidin
aus 1-(4-Chlorbenzolsulfonyl)-4-(4-methoxyimidoyl-benzyliden)piperidin-hydrochlorid und Ethanolamin.
Schmelzpunkt: 191,5-192,5 °C.

b) 1-(4-Chlorbenzoyl)-4-[4-(4,5-dihydro-6H-oxazin-2-yl)benzyliden]piperidin
aus 1-(4-Chlorbenzoyl)-4-(4-ethoxyimidoyl-benzyliden)piperidin-hydrochlorid und 3-Aminopropanol.
Schmelzpunkt: 159-160 °C.

c) 1-(4-Chlorbenzoyl)-4-[4-(4,4-dimethyl-2-oxazolin-2-yl)benzyliden]piperidin
aus 1-(4-Chlorbenzoyl)-4-(4-ethoxyimidoyl-benzyliden)piperidin-hydrochlorid und 2-Amino-2-methylpropanol.
Schmelzpunkt: 145-146 °C.

d) 1-(4-Chlorbenzoyl)-4-[4-(2-thiazolin-2-yl)benzyliden]piperidin
aus 1-(4-Chlorbenzoyl)-4-(4-ethoxyimidoyl-benzyliden)piperidin-hydrochlorid und Cysteamin.
Schmelzpunkt: 162-163 °C.

e) 1-(4-Chlorbenzoyl)-4-[4-(R-5-methyl-2-oxazolin-2-yl)benzyliden]piperidin
aus 1-(4-Chlorbenzoyl)-4-(4-ethoxyimidoyl-benzyliden)piperidin-hydrochlorid und R-1-Amino-2-propanol.
Schmelzpunkt: 104-106 °C.

f) 1-(4-Chlorbenzoyl)-4-[4-(S-5-methyl-2-oxazolin-2-yl)benzyliden]piperidin
aus 1-(4-Chlorbenzoyl)-4-(4-ethoxyimidoyl-benzyliden)piperidin-hydrochlorid und S-1-Amino-2-propanol.
Schmelzpunkt: 107-108 °C.

g) 1-(4-Chlorbenzoyl)-4-[3-(2-oxazolin-2-yl)benzyliden]piperidin
aus 1-(4-Chlorbenzoyl)-4-(3-methoxyimidoyl-benzyliden)piperidin-hydrochlorid und Ethanolamin.
Schmelzpunkt: 90-92 °C.

h) 1-(4-Chlorbenzoyl)-4-[4-(5,5-dimethyl-2-oxazolin-2-yl)benzyliden]piperidin
aus 1-(4-Chlorbenzoyl)-4-(4-ethoxyimidoyl-benzyliden)piperidin-hydrochlorid und 2-Hydroxy-2-methylpropylamin.
Schmelzpunkt: 145-146 °C.

i) 1-(4-Chlorbenzolsulfonyl)-4-[4-(2-imidazolin-2-yl)benzyliden]piperidin
aus 1-(4-Chlorbenzolsulfonyl)-4-(4-methoxyimidoyl-benzyliden)piperidin-hydrochlorid und Ethylendiamin.
Schmelzpunkt: 222-224 °C.

j) 1-(4-Chlorbenzolsulfonyl)-4-[4-(N-methyl-2-imidazolin-2-yl)benzyliden]piperidin
aus 1-(4-Chlorbenzolsulfonyl)-4-(4-methoxyimidoyl-benzyliden)piperidin-hydrochlorid und N-Methylethylendiamin. Farbloser Schaum.
$R_f$-Wert: 0,24 (Aluminiumoxid; Essigsäureethylester).

k) 1-(4-Chlorbenzoyl)-4-[4-(S-4-methyl-2-oxazolin-2-yl)benzyliden]piperidin
aus 1-(4-Chlorbenzoyl)-4-(4-ethoxyimidoyl-benzyliden)piperidin-hydrochlorid und L-Alaninol.
Schmelzpunkt: 143 °C.

l) 1-(4-Chlorbenzoyl)-4-[4-(R-4-methyl-2-oxazolin-2-yl)benzyliden]piperidin
aus 1-(4-Chlorbenzoyl)-4-(4-ethoxyimidoyl-benzyliden)piperidin-hydrochlorid und D-Alaninol.
Schmelzpunkt: 143 °C.

m) 1-(4-Chlorbenzoyl)-4-[4-(5-phenyl-2-oxazolin-2-yl)benzyliden]piperidin
aus 1-(4-Chlorbenzoyl)-4-(4-ethoxyimidoyl-benzyliden)piperidin-hydrochlorid und 2-Amino-1-phenylethanol.
Schmelzpunkt: 147 °C.

n) 1-(4-Chlorbenzoyl)-4-[4-(5-diethylaminomethyl-2-oxazolin-2-yl)benzyliden]piperidin
aus 1-(4-Chlorbenzoyl)-4-(4-ethoxyimidoyl-benzyliden)piperidin-hydrochlorid und 1-Amino-3-diethylamino-2-propanol.
Schmelzpunkt: 91 °C.

o) 1-(4-Chlorbenzoyl)-4-[4-(4-hydroxmethyl-2-oxazolin-2-yl)benzyliden]piperidin
aus 1-(4-Chlorbenzoyl)-4-(4-ethoxyimidoyl-benzyliden)piperidin-hydrochlorid und DL-Serinol.
Schmelzpunkt: 137 °C.

p) 1-(4-Chlorbenzoyl)-4-[4-(2-oxazolin-2-yl)-$\alpha$-methyl-benzyliden]piperidin
aus 1-(4-Chlorbenzoyl)-4-(4-ethoxyimidoyl-$\alpha$-methyl-benzyliden)piperidin-hydrochlorid und Ethanolamin.
Schmelzpunkt: 192 °C.

q) 1-(4-Chlorbenzoyl)-4-[4-(R-4-phenyl-2-oxazolin-2-yl)benzyliden]piperidin
aus 1-(4-Chlorbenzoyl)-4-(4-ethoxyimidoyl-benzyliden)piperidin und R-2-Amino-2-phenylethanol.
Schmelzpunkt: 130 °C.

r) 4-[4-(R-4-Benzyl-2-oxazolin-2-yl)benzyliden]-1-(4-chlorbenzoyl)piperidin
aus 1-(4-Chlorbenzoyl)-4-(4-ethoxyimidoyl-benzyliden)piperidin und R-2-Amino-3-phenyl-1-propanol.
Schmelzpunkt: 142 °C.

s) 4-[4-(S-4-Benzyl-2-oxazolin-2-yl)benzyliden]-1-(4-chlorbenzoyl)piperidin
aus 1-(4-Chlorbenzoyl)-4-(4-ethoxyimidoyl-benzyliden)piperidin und S-2-Amino-3-phenyl-1-propanol.
Schmelzpunkt: 142-143 °C.

t) 1-(4-Chlorbenzoyl)-4-[4-(4-((4-chlorbenzyl))-2-oxazolin-2-yl)benzyliden]piperidin
aus 1-(4-Chlorbenzoyl)-4-(4-ethoxyimidoyl-benzyliden)piperidin und 4-Chlorphenylalaninol.
Schmelzpunkt: 127 °C.

u) 1-(4-Chlorbenzoyl)-4-[4-(4-((2-methylthioethyl))-2-oxazolin-2-yl)benzyliden]piperidin
aus 1-(4-Chlorbenzoyl)-4-(4-ethoxyimidoyl-benzyliden)piperidin und L-Methioninol.
Schmelzpunkt: 95-96 °C.
v) 1-(4-Chlorbenzoyl)-4-[4-(4-((1-methyl-S-propyl))-2-oxazolin-2-yl)benzyliden]piperidin
aus 1-(4-Chlorbenzoyl)-4-(4-ethoxyimidoyl-benzyliden)piperidin und S-Isoleucinol.
Schmelzpunkt: 101 °C.
w) 1-(4-Chlorbenzoyl)-4-[4-(2-imidazolin-2-yl)benzyl]piperidin
aus 1-(4-Chlorbenzoyl)-4-(4-ethoxyimidoyl-benzyl)piperidin-hydrochlorid und Ethylendiamin.
Schmelzpunkt: 155-157 °C.
x) 1-(4-Chlorbenzoyl)-4-[4-(S-4-methyl-2-oxazolin-2-yl)benzyl]piperidin
aus 1-(4-Chlorbenzoyl)-4-(4-ethoxyimidoyl-benzyl)piperidin-hydrochlorid und L-Alaninol.
Schmelzpunkt: 92-95 °C.
y) 1-(4-Chlorbenzoyl)-4-[4-(N-methyl-2-imidazolin-2-yl)benzyl]piperidin
aus 1-(4-Chlorbenzoyl)-4-(4-ethoxyimidoyl-benzyl)piperidin-hydrochlorid und N-Methylethylendiamin.
Schmelzpunkt: 163-167 °C.
z) 1-(4-Chlorbenzoyl)-4-[4-(S-5-methyl-2-oxazolin-2-yl)benzyl]piperidin
aus 1-(4-Chlorbenzoyl)-4-(4-ethoxyimidoyl-benzyl)piperidin-hydrochlorid und S-1-Amino-2-propanol.
Schmelzpunkt: 98-100 °C.
aa) 1-(4-Chlorbenzoyl)-4-[4-(R-5-methyl-2-oxazolin-2-yl)benzyl]piperidin
aus 1-(4-Chlorbenzoyl)-4-(4-ethoxyimidoyl-benzyl)piperidin-hydrochlorid und R-1-Amino-2-propanol.
Schmelzpunkt: 99-101 °C.
ab) 1-(4-Chlorbenzoyl)-4-[4-(2-thiazolin-2-yl)benzyl]piperidin
aus 1-(4-Chlorbenzoyl)-4-(4-ethoxyimidoyl-benzyl)piperidin-hydrochlorid und Cysteamin.
Schmelzpunkt: 106-108 °C.
ac) 1-(4-Chlorbenzoyl)-4-[4-(4,5-dihydro-6H-oxazin-2-yl)benzyl]piperidin
aus 1-(4-Chlorbenzoyl)-4-(4-ethoxyimidoyl-benzyl)piperidin-hydrochlorid und 3-Aminopropanol.
Schmelzpunkt: 106-108 °C.
ad) 1-(4-Chlorbenzoyl)-3-[4-(2-oxazolin-2-yl)benzyliden]piperidin
aus 1-(4-Chlorbenzoyl)-3-(4-ethoxyimidoyl-benzyliden)piperidin-hydrochlorid und Ethanolamin.
Schmelzpunkt: 151 °C.

Beispiel 2

1-(4-Chlorbenzoyl)-4-[4-(2-oxazolin-2-yl)benzyliden]piperidin

Zu 6 g (0,02 Mol) 4-(2-Oxazolin-2-yl)benzylphosphonsäurediethylester in 80 ml Tetrahydrofuran werden bei -50 °C 12,5 ml (0,021 Mol) einer 1,6 M Lösung von n-Butyllithium in n-Hexan zugetropft. Die tiefrote Lösung wird 25 Minuten bei -55 °C gerührt und anschließend werden 4,7 g (0,02 Mol) N-(4-Chlorbenzoyl)-4-piperidon in 20 ml Tetrahydrofuran zugetropft. Nach 5 Stunden Rühren bei Raumtemperatur wird die Reaktionsmischung in 300 ml Wasser eingerührt, der Niederschlag nach weiteren 20 Minuten abgesaugt und aus Ethanol umkristallisiert. Man erhält 3,2 g (42% der Theorie) der mit dem in Beispiel 1 genannten Produkt identischen Titelverbindung.

Auf dieselbe Weise wurden erhalten:
a) 1-(4-Methylbenzoyl)-4-[4-(2-oxazolin-2-yl)benzyliden]piperidin
aus 4-(2-Oxazolin-2-yl)benzylphosphonsäurediethylester und N-(4-Methylbenzoyl)-4-piperidon.
Schmelzpunkt: 169-171 °C.
b) 1-(Dihydrocinnamoyl)-4-[4-(2-oxazolin-2-yl)benzyliden]piperidin
aus 4-(2-Oxazolin-2-yl)benzylphosphonsäurediethylester und N-Dihydrocinnamoyl-4-piperidon.
Schmelzpunkt: 98-100 °C.
c) 1-(4-Chlorcinnamoyl)-4-[4-(2-oxazolin-2-yl)benzyliden]piperidin
aus 4-(2-Oxazolin-2-yl)benzylphosphonsäurediethylester und N-(4-Chlorcinnamoyl)-4-piperidon.
$R_f$-Wert: 0,86 (Aluminiumoxid; Essigsäureethylester)
d) 1-Hexanoyl-4-[4-(2-oxazolin-2-yl)benzyliden]piperidin
aus 4-(2-Oxazolin-2-yl)benzylphosphonsäurediethylester und N-Hexanoyl-4-piperidon.
Schmelzpunkt: 98-100 °C.
e) 4-[4-(2-Oxazolin-2-yl)benzyliden-1-pivaloylpiperidin
aus 4-(2-Oxazolin-2-yl)benzylphosphonsäurediethylester und N-Pivaloyl-4-piperidon.
Schmelzpunkt: 163-165 °C.

f) 1-Cyclohexancarbonyl-4-[4-(2-oxazolin-2-yl)benzyliden]piperidin
aus 4-(2-Oxazolin-2-yl)benzylphosphonsäurediethylester und N-Cyclohexancarbonyl-4-piperidon.
Schmelzpunkt: 163-164 °C.

Beispiel 3

4-[4-(2-Oxazolin-2-yl)benzyliden]-1-(4-trifluormethylbenzoyl)piperidin

120 mg (0,5 mMol) 4-[4-(2-Oxazolin-2-yl)benzyliden]piperidin und 75 mg (0,75 mMol) Triethylamin in 3 ml Essigsäureethylester und 2 ml Methylenchlorid werden auf -5 bis -10 °C gekühlt und 104,3 mg (0,5 mMol) 4-Trifluormethylbenzoylchlorid zugegeben. Es wird 1,5 Stunden bei Raumtemperatur gerührt, mit Essigsäureethylester verdünnt, nacheinander mit Wasser, 2N-Natronlauge und gesättigter Kochsalzlösung gewaschen, getrocknet und eingedampft. Man erhält 115 mg (56% der Theorie) der Titelverbindung vom Schmelzpunkt 142 °C.
$^{1}H$-NMR-Spektrum (200 MHz, $CDCl_3$); Signale bei ppm:
2.3-2.7 (m,4H), 3.3-3.5 (m,2H), 3.7-3.9 (m,2H), 4.05 (t,3H), 4.4 (t,3H), 6.4 (s,1H), 7.2 (d,2H), 7.55 (d,2H), 7.7 (d,2H), 7.9 (d,2H).

Auf dieselbe Weise wurden erhalten:

a) 4-[4-(2-Oxazolin-2-yl)benzyliden]-1-stearoylpiperidin
aus 4-[4-(2-Oxazolin-2-yl)benzyliden]piperidin und Stearinsäurechlorid.
Schmelzpunkt: 72 °C.

b) 1-Cyclopropylcarbonyl-4-[4-(2-oxazolin-2-yl)benzyliden]piperidin
aus 4-[4-(2-Oxazolin-2-yl)benzyliden]piperidin und Cyclopropancarbonsäurechlorid.
Schmelzpunkt: 117 °C.

c) 1-(4-Chlor-3-methylbenzoyl)-4-[4-(2-oxazolin-2-yl)benzyliden]piperidin
aus 4-[4-(2-Oxazolin-2-yl)benzyliden]piperidin und 4-Chlor-3-methylbenzoylchlorid.
Schmelzpunkt: 145 °C.

d) 1-(3,4-Dichlorphenylacetyl)-4-[4-(2-oxazolin-2-yl)benzyliden]piperidin
aus 4-[4-(2-Oxazolin-2-yl)benzyliden]piperidin und 3,4-Dichlorphenylessigsäurechlorid.
Schmelzpunkt: 132 °C.

e) 1-(4-Fluorbenzoyl)-4-[4-(2-oxazolin-2-yl)benzyliden]piperidin
aus 4-[4-(2-Oxazolin-2-yl)benzyliden]piperidin und 4-Fluorbenzoesäurechlorid.
Schmelzpunkt: 146 °C.

f) 1-(5-Chlor-2-thienoyl)-4-[4-(2-oxazolin-2-yl)benzyliden]piperidin
aus 4-[4-(2-Oxazolin-2-yl)benzyliden]piperidin und 5-Chlorthiophencarbonsäurechlorid.
Schmelzpunkt: 145 °C.

g) 1-(2-Naphthylacetyl)-4-[4-(2-oxazolin-2-yl)benzyliden]piperidin
aus 4-[4-(2-Oxazolin-2-yl)benzyliden]piperidin und 2-Naphthalinessigsäurechlorid.
Schmelzpunkt: 133 °C.

h) 1-(4-Chlorbenzoyl)-4-[4-(2-oxazolin-2-yl)benzyliden]hexahydroazepin
aus 4-[4-(2-Oxazolin-2-yl)benzyliden]hexahydroazepin und 4-Chlorbenzoylchlorid.
Zähes Öl.
$R_f$-Wert: 0,42 (Aluminiumoxid; Petrolether/Essigsäureethylester = 1:1, v:v).

i) 1-(4-Chlorbenzoyl)-4-[4-(2-oxazolin-2-yl)benzyl]piperidin
aus 4-[4-(2-Oxazolin-2-yl)benzyl]piperidin und 4-Chlorbenzoylchlorid.
Schmelzpunkt: 140 °C.

j) 1-(1-Naphthylcarbonyl)-4-[4-(2-oxazolin-2-yl)benzyliden]piperidin
aus 4-[4-(2-Oxazolin-2-yl)benzyliden]piperidin und 1-Naphthalincarbonsäurechlorid.
Schmelzpunkt: 146-149 °C.

k) 1-(3,4-Difluorbenzoyl)-4-[4-(2-oxazolin-2-yl)benzyliden]piperidin
aus 4-[4-(2-Oxazolin-2-yl)benzyliden]piperidin und 3,4-Difluorbenzoylchlorid.
Schmelzpunkt: 133-135 °C.

l) 1-(3,5-Bis-trifluormethylbenzoyl)-4-[4-(2-oxazolin-2-yl)benzyliden]piperidin
aus 4-[4-(2-Oxazolin-2-yl)benzyliden]piperidin und 3,5-Bis-trifluormethylbenzoylchlorid.
Schmelzpunkt: 137 °C.

m) 1-(4-Cyanobenzoyl)-4-[4-(2-oxazolin-2-yl)benzyliden]piperidin
aus 4-[4-(2-Oxazolin-2-yl)benzyliden]piperidin und 4-Cyanobenzoylchlorid.
Schmelzpunkt: 166 °C.

n) 1-(2-Naphthylcarbonyl)-4-[4-(2-oxazolin-2-yl)benzyliden]piperidin
aus 4-[4-(2-Oxazolin-2-yl)benzyliden]piperidin und 2-Naphthalincarbonsäurechlorid.
Schmelzpunkt: 167 ° C.
o) 4-[4-(2-Oxazolin-2-yl)benzyliden]-1-(2-trifluormethylbenzoyl)piperidin
aus 4-[4-(2-Oxazolin-2-yl)benzyliden]piperidin und 2-Trifluormethylbenzoylchlorid.
Schmelzpunkt: 128 ° C.
p) 1-(3,4-Dichlorbenzoyl)-4-[4-(2-oxazolin-2-yl)benzyliden]piperidin
aus 4-[4-(2-Oxazolin-2-yl)benzyliden]piperidin und 3,4-Dichlorbenzoylchlorid.
Schmelzpunkt: 159 ° C.
q) 1-(4-Fluor-1-naphthylcarbonyl)-4-[4-(2-oxazolin-2-yl)benzyliden]piperidin
aus 4-[4-(2-Oxazolin-2-yl)benzyliden]piperidin und 4-Fluornapthalincarbonsäurechlorid.
Schmelzpunkt: zuerst bei 132 ° C, dann nach zwischenzeitlichen Erstarren bei 157 ° C.
r) 4-[4-(2-Oxazolin-2-yl)benzyliden]-1-(pentafluorbenzoyl)piperidin
aus 4-[4-(2-Oxazolin-2-yl)benzyliden]piperidin und Pentafluorbenzoylchlorid.
Schmelzpunkt: 212 ° C.
s) 1-Benzoyl-4-[4-(2-oxazolin-2-yl)benzyliden]piperidin
aus 4-[4-(2-Oxazolin-2-yl)benzyliden]piperidin und Benzoylchlorid.
Schmelzpunkt: 136 ° C.
t) 1-(4-Methylsulfonylbenzoyl)-4-[4-(2-oxazolin-2-yl)benzyliden]piperidin
aus 4-[4-(2-Oxazolin-2-yl)benzyliden]piperidin und 4-Methylsulfonylbenzoylchlorid.
Schmelzpunkt: 191 ° C.
u) 1-(4-tert.-Butylbenzoyl)-4-[4-(2-oxazolin-2-yl)benzyliden]piperidin
aus 4-[4-(2-Oxazolin-2-yl)benzyliden]piperidin und 4-tert.-Butylbenzoylchlorid.
Schmelzpunkt: 193 ° C.
v) 4-[4-(2-Oxazolin-2-yl)benzyliden]-1-(3-trifluormethylbenzoyl)piperidin
aus 4-[4-(2-Oxazolin-2-yl)benzyliden]piperidin und 3-Trifluormethylbenzoylchlorid.
Schmelzpunkt: 99 ° C.
w) 1-(3-Chlorbenzoyl)-4-[4-(2-oxazolin-2-yl)benzyliden]piperidin
aus 4-[4-(2-Oxazolin-2-yl)benzyliden]piperidin und 3-Chlorbenzoylchlorid.
Schmelzpunkt: 116 ° C.
x) 1-(4-Methoxybenzoyl)-4-[4-(2-oxazolin-2-yl)benzyliden]piperidin
aus 4-[4-(2-Oxazolin-2-yl)benzyliden]piperidin und 4-Methoxybenzoylchlorid.
Schmelzpunkt: 153 ° C.
y) 1-(2,5-Difluorbenzoyl)-4-[4-(2-oxazolin-2-yl)benzyliden]piperidin
aus 4-[4-(2-Oxazolin-2-yl)benzyliden]piperidin und 2,5-Difluorbenzoylchlorid.
Schmelzpunkt: 137 ° C.
z) 1-(4-Brombenzoyl)-4-[4-(2-oxazolin-2-yl)benzyliden]piperidin
aus 4-[4-(2-Oxazolin-2-yl)benzyliden]piperidin und 4-Brombenzoylchlorid.
Schmelzpunkt: 199 ° C.
aa) 4-[4-(2-Oxazolin-2-yl)benzyliden]-1-(1,2,3,4-Tetrahydro-2-naphthylcarbonyl)piperidin
aus 4-[4-(2-Oxazolin-2-yl)benzyliden]piperidin und 1,2,3,4-Tetrahydro-2-naphthalincarbonsäurechlorid.
Schmelzpunkt: 151 ° C.
ab) 1-(4-Nitrobenzoyl)-4-[4-(2-oxazolin-2-yl)benzyliden]piperidin
aus 4-[4-(2-Oxazolin-2-yl)benzyliden]piperidin und 4-Nitrobenzoylchlorid.
Schmelzpunkt: 192 ° C.
ac) 1-(4-Chlorphenylacetyl)-4-[4-(2-oxazolin-2-yl)benzyliden]piperidin
aus 4-[4-(2-Oxazolin-2-yl)benzyliden]piperidin und 4-Chlorphenylacetylchlorid.
Schmelzpunkt: 107 ° C.
ad) 1-Benzoyl-4-[4-(2-oxazolin-2-yl)benzyl]piperidin
aus 4-[4-(2-Oxazolin-2-yl)benzyl]piperidin und Benzoylchlorid.
Schmelzpunkt: 78-80 ° C.
ae) 1-(2-Chlorbenzoyl)-4-[4-(2-oxazolin-2-yl)benzyl]piperidin
aus 4-[4-(2-Oxazolin-2-yl)benzyl]piperidin und 2-Chlorbenzoylchlorid.
Schmelzpunkt: 105-108 ° C.
af) 1-(3-Chlorbenzoyl)-4-[4-(2-oxazolin-2-yl)benzyl]piperidin
aus 4-[4-(2-Oxazolin-2-yl)benzyl]piperidin und 3-Chlorbenzoylchlorid.
Harz.
$R_f$-Wert: 0,6 (Kieselgel; Essigsäureethylester/Petrolether = 10:2, v:v).

ag) 1-(4-Fluorbenzoyl)-4-[4-(2-oxazolin-2-yl)benzyl]piperidin
aus 4-[4-(2-Oxazolin-2-yl)benzyl]piperidin und 4-Fluorbenzoylchlorid.
Schmelzpunkt: 111-113 °C.
ah) 1-(4-tert.-Butylbenzoyl)-4-[4-(2-oxazolin-2-yl)benzyl]piperidin
aus 4-[4-(2-Oxazolin-2-yl)benzyl]piperidin und 4-tert.-Butylbenzoylchlorid.
Harz.
$R_f$-Wert: 0,3 (Kieselgel; Essigsäureethylester).
ai) 1-(4-Biphenylcarbonyl)-4-[4-(2-oxazolin-2-yl)benzyl]piperidin
aus 4-[4-(2-Oxazolin-2-yl)benzyl]piperidin und 4-Biphenylcarbonylchlorid.
Schmelzpunkt: 158-160 °C.
aj) 1-(2-Naphthylcarbonyl)-4-[4-(2-oxazolin-2-yl)benzyl]piperidin
aus 4-[4-(2-Oxazolin-2-yl)benzyl]piperidin und 2-Naphthalincarbonsäurechlorid.
Schmelzpunkt: 100-105 °C.
ak) 1-(1-Naphthylcarbonyl)-4-[4-(2-oxazolin-2-yl)benzyl]piperidin
aus 4-[4-(2-Oxazolin-2-yl)benzyl]piperidin und 1-Naphthalincarbonsäurechlorid.
Schmelzpunkt: 70-73 °C.
al) 1-(4-Chlorphenylsulfonyl)-4-[4-(2-oxazolin-2-yl)benzyl]piperidin
aus 4-[4-(2-Oxazolin-2-yl)benzyl]piperidin und 4-Chlorphenylsulfonylchlorid.
Schmelzpunkt: 155-157 °C.
am) 1-(4-Methylbenzoyl)-4-[4-(2-oxazolin-2-yl)benzyl]piperidin
aus 4-[4-(2-Oxazolin-2-yl)benzyl]piperidin und 4-Methylbenzoylchlorid.
Schmelzpunkt: 143-145 °C.
an) 4-(4-Cyanobenzoyl)-4-[4-(2-oxazolin-2-yl)benzyl]piperidin
aus 4-[4-(2-Oxazolin-2-yl)benzyl]piperidin und 4-Cyanobenzoylchlorid.
Schmelzpunkt: 148-150 °C.
ao) 4-[4-(2-Oxazolin-2-yl)benzyl]-1-(4-pyridoyl)piperidin
aus 4-[4-(2-Oxazolin-2-yl)benzyl]piperidin und Isonicotinsäurechlorid.
Schmelzpunkt: 148-150 °C.
ap) 1-(4-Chlorbenzoyl)-3-[4-(2-oxazolin-2-yl)benzyliden]pyrrolidin
aus 3-[4-(2-Oxazolin-2-yl)benzyliden]pyrrolidin und 4-Chlorbenzoylchlorid.
$R_f$-Wert: 0,36 (Kieselgel; Methylenchlorid/Methanol = 24:1, v:v).

## Beispiel 4

### 4-[4-(2-Oxazolin-2-yl)benzyliden]-1-(4-pentinoyl)piperidin

356 mg (2,2 mMol) N,N'-Carbonyldiimidazol in 3 ml Tetrahydrofuran werden bei Raumtemperatur mit 200 mg (2 mMol) 4-Pentinsäure in 2 ml Tetrahydrofuran versetzt. Es wird eine Stunde bei 40 °C gerührt. Danach werden 363 mg (1,5 mMol) 4-[4-(2-Oxazolin-2-yl)benzyliden]piperidin zugegeben und weitere 2 Stunden bei 40 °C gerührt. Anschließend wird die Reaktionslösung eingeengt und der Rückstand durch Säulenchromatographie gereinigt (Kieselgel; Essigsäureethylester). Man erhält 320 mg (66% der Theorie) der Titelverbindung vom Schmelzpunkt 118 °C.
$^1$H-NMR-Spektrum (200 MHz, $CDCl_3$); Signale bei ppm:
2.0 (s,1H), 2.3-2.7 (m,8H), 3.4-3.8 (m,4H), 4,05 (t,2H), 4.4 (t,2H), 6.4 (s,1H), 7.2 (d,2H), 2.9 (d,2H).

Auf dieselbe Weise wurde erhalten:
a) 4-[4-(2-Oxazolin-2-yl)benzyliden]-1-(4-phenyl-3-butenoyl)piperidin
aus 4-[4-(2-Oxazolin-2-yl)benzyliden]piperidin, 4-Phenyl-3-butensäure und N,N'-carbonyldiimidazol.
Schmelzpunkt: 139 °C.

## Beispiel 5

### 4-[4-(2-Oxazolin-2-yl)benzyl]-1-(4-trifluormethylbenzoyl)piperidin

441 mg (1 mMol) 4-[4-(2-Oxazolin-2-yl)benzyliden]-1-(4-trifluormethylbenzoyl)piperidin werden in 10 ml wasserfreiem Ethanol und 2,5 ml Essigsäureethylester in Gegenwart von 0,5 g Palladium-Kohle (10%) hydriert (Raumtemperatur, 30 Minuten, Wasserstoffdruck 50 psi). Anschließend wird vom Katalysator abgesaugt, das Filtrat bei einer Badtemperatur von 50 °C unter reduziertem Druck eingedampft und der Rückstand durch Säulenchromatographie gereinigt (Kieselgel; Essigsäureethylester). Man erhält 200 mg

(48% der Theorie) der Titelverbindung vom Schmelzpunkt 115 °C.
$^1$H-NMR-Spektrum (200 MHz, $CDCl_3$); Signale bei ppm:
1,0-1,4 (m,2H), 1,6-1,9 (m,3H), 2,6 (d,2H), 2,7-3,1 (m,2H), 3,6 (m,1H), 4,05 (t,2H), 4,4 (t,2H), 4,7 (m,1H), 7,15 (d,2H), 7,5 (d,2H), 7,65 (d,2H), 7,85 (d,2H).

Auf dieselbe Weise wurden erhalten:

a) 1-(4-Chlorbenzoyl)-4-[4-(R-4-methyl-2-oxazolin-2-yl)benzyl]piperidin
aus 1-(4-Chlorbenzoyl)-4-[4-(R-4-methyl-2-oxazolin-2-yl)benzyliden]piperidin
Farbloses Harz.
$R_f$-Wert: 0,37 (Kieselgel; Essigsäureethylester)

b) 1-(4-Chlorbenzoyl)-4-[4-(2-oxazolin-2-yl)benzyl]piperidin
aus 1-(4-Chlorbenzoyl)-4-[4-(2-oxazolin-2-yl)benzyliden]piperidin.
Schmelzpunkt: 136-138 °C.

c) 1-(4-Chlorbenzoyl)-3-[4-(2-oxazolin-2-yl))benzyl]piperidin
aus 1-(4-Chlorbenzoyl)-3-[4-(2-oxazolin-2-yl)benzyliden]piperidin.
Farbloses Harz.
$R_f$-Wert: 0,5 (Kieselgel; Essigsäureethylester)

Beispiel 6

1-(4-Chlorbenzoyl)-4-[2-fluor-4-(2-oxazolin-2-yl)benzyliden]piperidin

0,4 g (0,95 mMol) 1-(4-Chlorbenzoyl)-4-[2-fluor-4-(2-hydroxyethylaminocarbonyl)benzyliden]piperidin in 20 ml Tetrahydrofuran werden in der Siedehitze portionsweise während 45 Minuten mit 0,3 g (1,1 mMol) Methyl-N-(triethylamoniosulfonyl)carbamat (Burgess-Reagenz) versetzt. Es wird zwei Stunden weiter erhitzt, eingedampft und der Rückstand mit Wasser versetzt, dreimal mit Methylenchlorid extrahiert, mit wasserfreiem Natriumsulfat getrocknet, eingedampft und der Rückstand über eine Kieselgelsäule gereinigt (Essigsäureethylester/Petrolether = 10:4, v:v). Man erhält 0,105 g (26% der Theorie) der Titelverbindung vom Schmelzpunkt 158-160 °C.
$^1$H-NMR-Spektrum (200 MHz, DMSO-$d_6$); Signale bei ppm:
2.4 (m,4H), 3.4-3.7 (m,4H), 3.95 (t,2H), 4.4 (t,2H), 6,35 (s,1H), 7.3-7.7 (m,7H).

Auf dieselbe Weise wurden dargestellt:

a) 1-(4-Chlorbenzoyl)-4-[3-methyl-4-(2-oxazolin-2-yl)benzyliden]piperidin
aus 1-(4-Chlorbenzoyl)-4-[4-(2-hydroxyethylaminocarbonyl)-3-methyl-benzyliden]piperidin und Burgess-Reagenz.
Schmelzpunkt: 88-90 °C.

b) 4-[2-Brom-4-(2-oxazolin-2-yl)benzyliden]-1-(4-chlorbenzoyl)piperidin
aus 4-[2-Brom-4-(2-hydroxyethylaminocarbonyl)benzyliden]-1-(4-chlorbenzoyl)piperidin und Burgess-Reagenz.
Schmelzpunkt: 185-187 °C.

c) 1-[4-Chlorbenzoyl)-4-[2-methoxy-4-(2-oxazolin-2-yl)benzyliden]piperidin
aus 1-(4-Chlorbenzoyl)-4-[4-(2-hydroxyethylaminocarbonyl)-2-methoxy-benzyliden]piperidin und Burgess-Reagenz.
Schmelzpunkt: 150-152 °C.

Im folgenden wird die Herstellung pharmazeutischer Anwendungsformen anhand einiger Beispiele beschrieben:

Beispiel I

Tabletten mit 5 mg 1-(4-Chlorbenzoyl)-4-[4-(2-oxazolin-2-yl)benzyliden]piperidin

Zusammensetzung:

| 1 Tablette enthält: | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragèes mit 5 mg 1-(4-Chlorbenzoyl)-4-[4-(2-oxazolin-2-yl)benzyliden]piperidin

Die nach Beispiel I hergestellten Tabletten werden nach bekanntem Verfahren mit einer Hülle überzogen, die im wesentlichen aus Zucker und Talkum besteht. Die fertigen Dragèes werden mit Hilfe von Bienenwachs poliert.

| | |
|---|---|
| Dragèegewicht: | 300 mg |

Beispiel III

Suppositorien mit 5 mg 1-(4-Chlorbenzoyl)-4-[4-(2-oxazolin-2-yl)benzyliden]piperidin

Zusammensetzung:

| 1 Zäpfchen enthält: | |
|---|---|
| Wirkstoff | 5,0 mg |
| Zäpfchenmasse (z.B. Witepsol W 45(R)) | 1 695,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus. Zäpfchengewicht: 1,7 g.

Beispiel IV

Kapseln mit 5 mg N-(4-Chlorbenzoyl)-4-[4-(2-oxazolin-2-yl)benzyliden]piperidin

Zusammensetzung:

| 1 Kapsel enthält: | |
|---|---|
| Wirksubstanz | 5,0 mg |
| Lactose | 82,0 mg |
| Stärke | 82,0 mg |
| Magnesiumstearat | 1,0 mg |
| | 170,0 mg |

Herstellungsverfahren:

Die Pulvermischung wird intensiv gemischt und auf einer Kapselabfüllmaschine in Hartgelatine-Steckkapseln der Größe 3 abgefüllt, wobei das Endgewicht laufend überprüft wird.

Beispiel V

Tabletten mit 5 mg 1-(4-Chlorbenzoyl)-4-[4-(2-oxazolin-2-yl)benzyl]piperidin

Zusammensetzung:

| 1 Tablette enthält: | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mg |

Beispiel VI

Creme für die topische Verabreichung mit 1 g 1-(4-Chlorbenzoyl)-4-[4-(2-oxazolin-2-yl)benzyliden]piperidin

Eine Formulierung für die topische Verabreichung der Verbindungen der Formel I kann folgende Zusammensetzung aufweisen

| | | |
|---|---|---|
| 1. | Wirkstoff | 1,0 g |
| 2. | Stearylalkohol | 4,0 g |
| 3. | Cetylalkohol | 4,0 g |
| 4. | Mineralöl | 3,0 g |
| 5. | Polysorbat 60 | 4,5 g |
| 6. | Sorbitanstearat | 4,5 g |
| 7. | Propylenglycol | 10,0 g |
| 8. | Methylparaben | 0,18 g |
| 9. | Propylparaben | 0,02 g |
| 10. | Wasser | q.s. ad 100,00 g |

Die Bestandteile 2-6 werden auf 80 °C erwärmt bis alles geschmolzen ist. Danach wird Bestandteil 1 in der öligen Phase gelöst. Bestandteil 7 und 10 werden auf 90 °C erwärmt und die Bestandteile 8 und 9 werden in der so erhaltenen wässrigen Phase gelöst. Danach wird die wässrige Phase zur Ölphase gegeben und rasch gerührt, so daß eine Emulsion erhalten wird. Danach läßt man langsam auf 50 °C abkühlen um die Emulsion zu verfestigen. Unter weiterem Rühren wird das Präparat auf Raumtemperatur abgekühlt.

Das folgende Beispiel beschreibt die Herstellung eines Futtermittels für Legehennen:

Beispiel VII

Futtermittel für Legehennen, enthaltend als Wirkstoff 1-(4-Chlorbenzoyl)-4-[4-(2-oxazolin-2-yl)benzyliden]-piperidin

| | |
|---|---|
| Mais | 633 g/kg |
| Sojabohnenmehl | 260 g/kg |
| Fleischmehl | 40 g/kg |
| Futterfett | 25 g/kg |
| Sojaöl | 17 g/kg |
| Bicalciumphosphat | 12 g/kg |
| Calciumcarbonat | 6 g/kg |
| Vitamin-Mineralstoffmischung | 5 g/kg |
| Wirkstoff | 2 g/kg |

Diese Komponenten in den angegebenen Mengen ergeben nach sorgfältigem Mischen 1 kg Futter.

## Patentansprüche

1. Aryliden-1-azacycloalkane und Arylalkyl-1-azacycloalkane der allgemeinen Formel I,

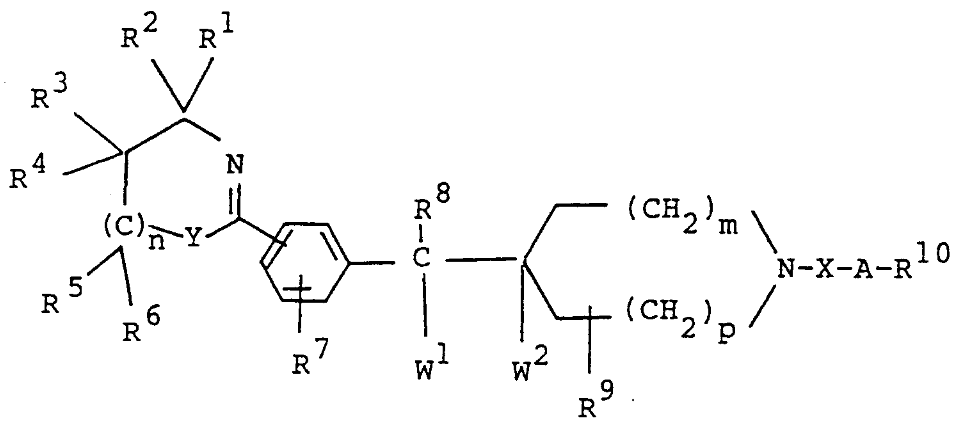

(I)

in der
n die Zahlen 0 oder 1,
m die Zahlen 1 oder 2,
p die Zahlen 0 oder 1,
A eine Einfachbindung, eine geradkettige oder verzweigte Alkylengruppe mit 1 bis 17 Kohlenstoffatomen, eine Alkenylengruppe mit 2 bis 17 Kohlenstoffatomen oder eine Alkinylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,
Y ein Sauerstoff- oder Schwefelatom oder eine $>NR^{11}$-Gruppe,
$R^1$ bis $R^6$ jeweils ein Wasserstoffatom oder
einer, zwei oder drei der Reste $R^1$ bis $R^6$, wobei die Reste gleich oder verschieden sein können, eine geradkettige oder verzweigte Alkylgruppe mit 1 bis 4 Kohlenstoffatomen, die durch eine Hydroxy-, Alkoxy-, Alkylthio- oder Dialkylaminogruppe oder durch eine gegebenenfalls durch ein Halogenatom oder eine Alkylgruppe substituierte Phenylgruppe substituiert sein kann, oder eine Alkoxycarbonylgruppe und die übrigen der Reste $R^1$ bis $R^6$ jeweils ein Wasserstoffatom,
wobei einer, zwei oder alle drei der Reste $R^1$, $R^3$ und $R^5$ auch eine gegebenenfalls durch eine Alkylgruppe oder ein Halogenatom substituierte Phenylgruppe bedeuten können,
$R^7$ ein Wasserstoff- oder Halogenatom, eine Alkyl- oder Alkoxygruppe,
$R^8$ und $R^9$ unabhängig voneinander jeweils ein Wasserstoffatom oder eine Alkylgruppe,
$R^{10}$ ein Wasserstoffatom, eine Cycloalkylgruppe mit 3 bis 6 Kohlenstoffatomen, eine gegebenenfalls durch ein Halogenatom, eine geradkettige oder verzweigte Alkylgruppe mit 1 bis 4 Kohlenstoffatomen, eine Trifluormethyl-, Alkoxy-, Cyano-, Nitro-, Alkylsulfonyl- oder Phenylgruppe substituierte Phenylgruppe, eine durch zwei Trifluormethylgruppen, zwei bis fünf Halogenatome oder durch ein Halogenatom und eine Alkylgruppe substituierte Phenylgruppe, eine gegebenenfalls durch ein Fluoratom substituierte Naphthyl- oder Tetrahydronaphthylgruppe, eine Pyridylgruppe oder eine gegebenenfalls durch ein Halogenatom oder eine Alkylgruppe substituierte Thienylgruppe,
$R^{11}$ ein Wasserstoffatom oder eine Alkylgruppe,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkyl-, Alkoxy-, Alkylthio- und Alkylsulfonylreste jeweils 1 bis 3 Kohlenstoffatome enthalten können und die vorstehend erwähnten Halogenatome jeweils ein Fluor-, Chlor- oder Bromatom bedeuten können,
deren Enantiomere, Diastereomere und deren Salze.

2. Aryliden-1-azacycloalkane und Arylalkyl-1-azacycloalkane gemäß Anspruch 1 der allgemeinen Formel Ia,

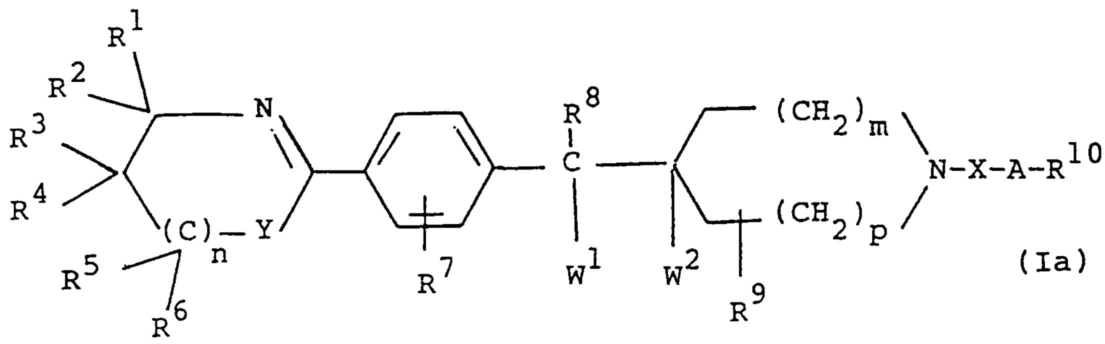

(Ia)

in der
n die Zahlen 0 oder 1,
m die Zahlen 1 oder 2,
p die Zahl 0 oder 1,
A eine Einfachbindung, eine geradkettige oder verzweigte Alkylengruppe mit 1 bis 17 Kohlenstoffatomen, eine Alkenylengruppe mit 2 bis 4 Kohlenstoffatomen oder eine Alkinylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,
Y ein Sauerstoff- oder Schwefelatom oder eine $>NR^{11}$-Gruppe,
$R^1$ bis $R^4$ jeweils ein Wasserstoffatom oder
einer oder zwei der Reste $R^1$ bis $R^4$ unabhängig voneinander jeweils eine geradkettige oder verzweigte Alkylgruppe mit 1 bis 4 Kohlenstoffatomen, die durch eine Hydroxy-, Alkylthio- oder Dialkylaminogruppe oder durch eine gegebenenfalls durch ein Halogenatom substituierte Phenylgruppe substituiert sein kann, oder eine Phenylgruppe und die übrigen der Reste $R^1$ bis $R^4$ jeweils ein Wasserstoffatom,
$R^5$ und $R^6$, die gleich oder verschieden sein können, ein Wasserstoffatom oder eine Methylgruppe,
$R^7$ ein Wasserstoff- oder Halogenatom, eine Alkyl- oder Alkoxygruppe,
$R^8$ ein Wasserstoffatom oder eine Alkylgruppe,
$R^9$ ein Wasserstoffatom,
$R^{10}$ ein Wasserstoffatom, eine Cycloalkylgruppe mit 3 bis 6 Kohlenstoffatomen, eine gegebenenfalls durch ein oder zwei Halogenatome, eine geradkettige oder verzweigte Alkylgruppe mit 1 bis 4 Kohlenstoffatomen, eine Trifluormethyl-, Methoxy-, Cyano-, Nitro-, Methylsulfonyl- oder Phenylgruppe substituierte Phenylgruppe, eine durch zwei Trifluormethylgruppen oder durch ein Halogenatom und eine Methylgruppe substituierte Phenylgruppe, eine durch drei bis fünf Fluoratome substituierte Phenylgruppe, eine gegebenenfalls durch ein Fluoratom substituierte Naphthylgruppe, eine Tetrahydronaphthyl- oder Pyridylgruppe oder eine gegebenenfalls durch ein Halogenatom substituierte Thienylgruppe und
$R^{11}$ ein Wasserstoffatom oder eine Alkylgruppe bedeuten,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkylreste jeweils 1 bis 3 Kohlenstoffatome enthalten können und die vorstehend erwähnten Halogenatome jeweils ein Fluor-, Chlor- oder Bromatom bedeuten können,
deren Enantiomere, Diastereomere und deren Salze.

3. Aryliden-1-azacycloalkane und Arylalkyl-1-azacycloalkane der allgemeinen Formel Ia gemäß Anspruch 2,
in der
n die Zahlen 0 oder 1,
m die Zahlen 1 oder 2,
p die Zahlen O oder 1,
A eine Einfachbindung, eine geradkettige oder verzweigte Alkylengruppe mit 1 bis 17 Kohlenstoffatomen oder eine Alkenylengruppe mit 2 bis 4 Kohlenstoffatomen,
$W^1$ und $W^2$ jeweils ein Wasserstoffatom oder zusammen eine Kohlenstoff-Kohlenstoffbindung,
X eine Carbonyl- oder Sulfonylgruppe,

Y ein Sauerstoffatom oder eine > $NR^{11}$-Gruppe,
$R^1$ bis $R^4$ jeweils ein Wasserstoffatom oder
einer oder zwei der Reste $R^1$ bis $R^4$ unabhängig voneinander jeweils eine geradkettige oder verzweigte Alkylgruppe mit 1 bis 4 Kohlenstoffatomen, und die übrigen der Reste $R^1$ bis $R^4$ jeweils ein Wasserstoffatom,
$R^5$ und $R^6$, die gleich oder verschieden sein können, ein Wasserstoffatom oder eine Methylgruppe,
$R^7$ ein Wasserstoff- oder Halogenatom, eine Methyl- oder Methoxygruppe,
$R^8$ ein Wasserstoffatom oder eine Methylgruppe,
$R^9$ ein Wasserstoffatom,
$R^{10}$ ein Wasserstoffatom, eine Cycloalkylgruppe mit 3 bis 6 Kohlenstoffatomen, eine gegebenenfalls durch ein oder zwei Halogenatome, fünf Fluoratome, eine Alkylgruppe, eine oder zwei Trifluormethylgruppen oder durch ein Halogenatom und eine Alkylgruppe substituierte Phenylgruppe, eine in 4-Position gegebenenfalls durch ein Fluoratom substituierte 1-Naphthylgruppe, eine 2-Naphthylgruppe, eine 1,2,3,4-Tetrahydro-2-naphthylgruppe, eine Pyridyl- oder 4-Biphenylgruppe oder eine gegebenenfalls durch ein Halogenatom substituierte Thienylgruppe und
$R^{11}$ ein Wasserstoffatom oder eine Methylgruppe bedeuten,
wobei A keine Einfachbindung sein kann, wenn X die Sulfonylgruppe und $R^{10}$ ein Wasserstoffatom bedeuten, und
wobei, sofern nichts anderes erwähnt wurde, die vorstehend erwähnten Alkylteile jeweils 1 bis 3 Kohlenstoffatome enthalten können und die vorstehend erwähnten Halogenatome jeweils ein Fluor- oder Chloratom bedeuten können,
deren Enantiomere, Diastereomere und deren Salze.

**4.** Aryliden-1-azacycloalkane und Arylalkyl-1-azacycloalkane der allgemeinen Formel Ia gemäß Anspruch 2,
in der
n die Zahlen 0 oder 1,
m die Zahl 1,
p die Zahlen O oder 1,
A eine Einfachbindung,
$W^1$ und $W^2$ jeweils ein Wasserstoffatom oder zusammen eine Kohlenstoff-Kohlenstoffbindung,
X eine Carbonylgruppe,
Y ein Sauerstoffatom,
$R^1$ bis $R^6$ jeweils ein Wasserstoffatom,
$R^7$ ein Wasserstoff- oder Halogenatom oder eine Methylgruppe,
$R^8$ und $R^9$ jeweils ein Wasserstoffatom,
$R^{10}$ eine in Position 4 durch ein Fluor-, Chlor- oder Bromatom oder eine Trifluormethylgruppe substituierte Phenylgruppe, eine 4-Chlor-3-methylphenylgruppe, eine 5-Chlor-2-thienylgruppe oder eine Cyclohexylgruppe bedeuten,
und deren Salze.

**5.** Folgende Verbindungen der allgemeinen Formel I gemäß Anspruch 1:
(1) 1-(4-Chlorbenzoyl)-4-[4-(2-oxazolin-2-yl)-benzyliden]piperidin
(2) 1-(4-Chlorbenzoyl)-4-[4-(4,5-dihydro-6H-oxazin-2-yl)benzyliden]piperidin
(3) 4-[4-(2-Oxazolin-2-yl)benzyliden]-1-(4-trifluormethylbenzoyl)piperidin
(4) 1-(4-Chlor-3-methylbenzoyl)-4-[4-(2-oxazolin-2-yl)benzyliden]piperidin
(5) 1-(4-Fluorbenzoyl)-4-[4-(2-oxazolin-2-yl)benzyliden]piperidin
(6) 1-(5-Chlor-2-thienoyl)-4-[4-(2-oxazolin-2-yl)benzyliden]piperidin
(7) 1-Cyclohexancarbonyl-4-[4-(2-oxazolin-2-yl)benzyliden]piperidin,
(8) 4-[4-(2-Oxazolin-2-yl)benzyl]-1-(4-trifluormethylbenzoyl)piperidin
(9) 1-(4-Chlorbenzoyl)-4-[4-(2-oxazolin-2-yl)-benzyl]piperidin
(10) 1-(4-Chlorbenzoyl)-3-[4-(2-oxazolin-2-yl)benzyliden]pyrrolidin
(11) 1-(4-Chlorbenzoyl)-4-[2-fluor-4-(2-oxazolin-2-yl)benzyliden]piperidin
(12) 1-(4-Chlorbenzoyl)-4-[3-methyl-4-(2-oxazolin-2-yl)benzyliden]piperidin
und deren Salze.

**6.** Physiologisch verträgliche Salze der Verbindungen nach mindestens einem der Ansprüche 1 bis 5 mit anorganischen oder organischen Säuren.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels zur Inhibition der Cholesterolbiosynthese, zur Behandlung oder Prophylaxe von Hyperlipidämien, zur Behandlung von Erkrankungen, die mit überhöhter Zellproliferation im Zusammenhang stehen, zur Prophylaxe und Behandlung von Gallensteinleiden oder zur Behandlung von Mykosen.

9. Verwendung einer Verbindung nach mindestens einem der Ansprüche 1 bis 6 zur Herstellung eines Futtermittels für Legehennen zur Erzeugung cholesterolarmer Eier.

10. Verfahren zur Herstellung eines Arzneimittels gemäß Anspruch 7, dadurch gekennzeichnet, daß auf nichtchemischem Wege eine Verbindung nach mindestens einem der Ansprüche 1 bis 6 in einen oder mehrere inerte Trägerstoffe und/oder Verdünnungsmittel eingearbeitet wird.

11. Verfahren zur Herstellung der Verbindungen nach mindestens einem der Ansprüche 1 bis 6, dadurch gekennzeichnet, daß

a) eine Verbindung der allgemeinen Formel II,

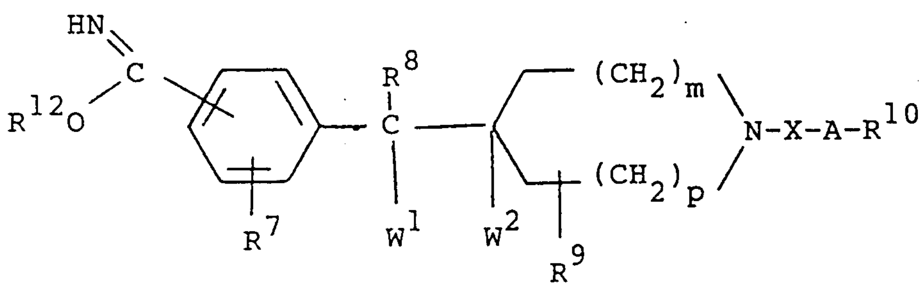

(II)

in der

m, p, A, $W^1$, $W^2$, X und $R^7$ bis $R^{10}$ die in den Ansprüchen 1 bis 5 erwähnten Bedeutungen besitzen und $R^{12}$ eine Alkylgruppe mit 1 bis 10 Kohlenstoffatomen bedeutet, mit einer Verbindung der allgemeinen Formel III,

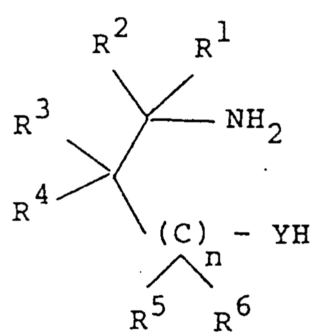

(III)

in der

n, Y und $R^1$ bis $R^6$ die in den Ansprüchen 1 bis 5 erwähnten Bedeutungen besitzen, umgesetzt wird oder

b) zur Herstellung von Verbindungen der allgemeinen Formel I, in der n, m, p, A, X, Y und $R^1$ bis $R^{10}$ die in den Ansprüchen 1 bis 5 erwähnten Bedeutungen besitzen und $W^1$ und $W^2$ zusammen eine Kohlenstoff-Kohlenstoffbindung bedeuten, ein Phosphonester der allgemeinen Formel IV,

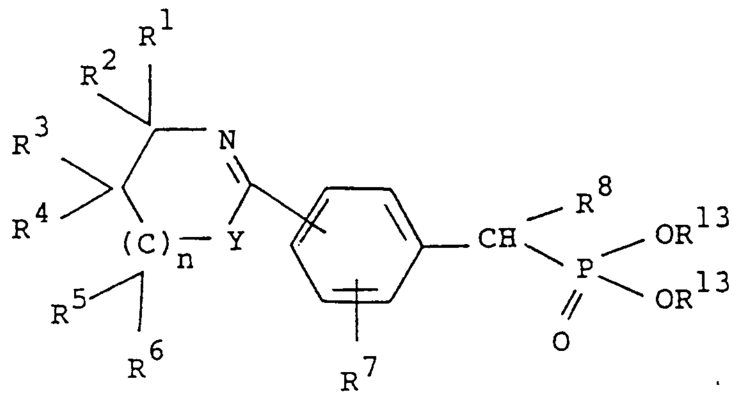

(IV)

in der
n, Y und $R^1$ bis $R^8$ die in den Ansprüchen 1 bis 5 erwähnten Bedeutungen besitzen und $R^{13}$ eine Alkylgruppe mit 1 bis 10 Kohlenstoffatomen bedeutet, mit einer Verbindung der allgemeinen Formel V,

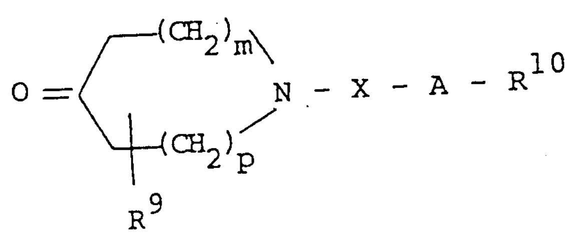

(V)

in der
m, p, A, X, $R^9$ und $R^{10}$ die in den Ansprüchen 1 bis 5 erwähnten Bedeutungen besitzen, umgesetzt wird oder
c) eine Verbindung der allgemeinen Formel VI,

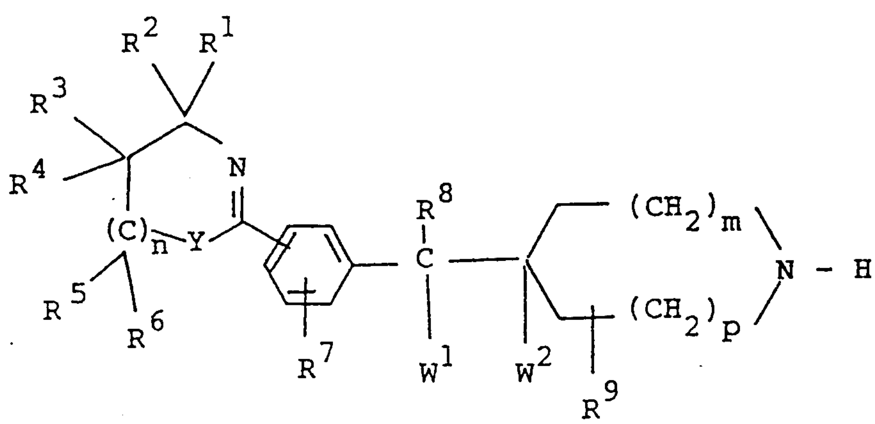

(VI)

in der
n, m, p, $W^1$, $W^2$, Y und $R^1$ bis $R^9$ die in den Ansprüchen 1 bis 5 erwähnten Bedeutungen besitzen, mit einer Verbindung der allgemeinen Formel VII,

$Z^1$ - X - A - $R^{10}$ (VII)

in der
A, X und $R^{10}$ die in den Ansprüchen 1 bis 5 erwähnten Bedeutungen besitzen und $Z^1$ eine reaktive Austrittsgruppe bedeutet, umgesetzt wird oder

d) zur Herstellung von Verbindungen der allgemeinen Formel I, in der n, m, p, X und $R^7$ bis $R^9$ die in den Ansprüchen 1 bis 5 erwähnten Bedeutungen besitzen, A eine Einfachbindung oder eine geradkettige oder verzweigte Alkylengruppe mit 1 bis 17 Kohlenstoffatomen, $W^1$ und $W^2$ jeweils ein Wasserstoffatom und Y ein Sauerstoffatom oder die $>NR^{11}$-Gruppe bedeuten, wobei $R^{11}$ die in den Ansprüchen 1 bis 5 erwähnten Bedeutungen besitzt, $R^1$ bis $R^6$ mit Ausnahme der durch eine Alkylthiogruppe substituierten geradkettigen oder verzweigten Alkylgruppe mit 1 bis 4 Kohlenstoffatomen und $R^{10}$ mit Ausnahme der gegebenenfalls durch ein Halogenatom substituierten Thienylgruppe die in den Ansprüchen 1 bis 5 erwähnten Bedeutungen besitzen, wobei A keine Einfachbindung sein kann, wenn X die Sulfonylgruppe und $R^{10}$ ein Wasserstoffatom bedeuten, eine Verbindung der allgemeinen Formel I',

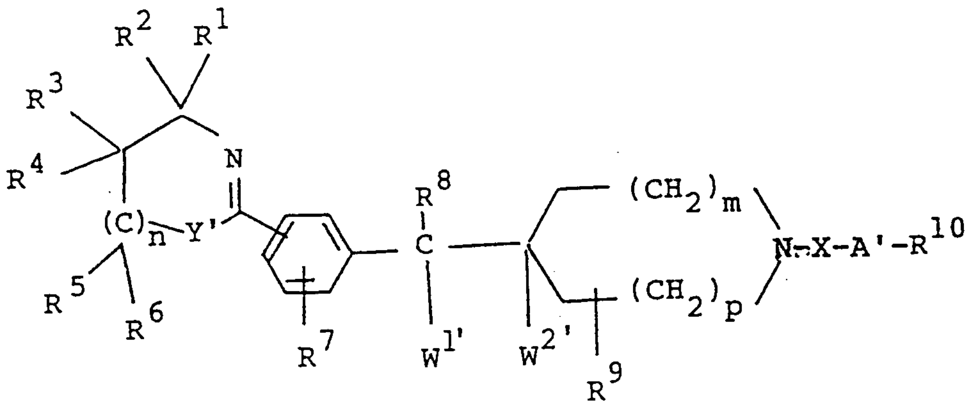

(I')

in der

n, m, p, X und $R^7$ bis $R^9$ die in den Ansprüchen 1 bis 5 erwähnten Bedeutungen besitzen, A' eine Einfachbindung oder eine geradkettige oder verzweigte Alkylengruppe mit 1 bis 17 Kohlenstoffatomen, $W^{1'}$ und $W^{2'}$ zusammen eine Kohlenstoff-Kohlenstoffbindung und Y' ein Sauerstoffatom oder die $>NR^{11}$-Gruppe bedeuten, wobei $R^{11}$ die in den Ansprüchen 1 bis 5 erwähnten Bedeutungen besitzt, $R^1$ bis $R^6$ mit Ausnahme der durch eine Alkylthiogruppe substituierten geradkettigen oder verzweigten Alkylgruppe mit 1 bis 4 Kohlenstoffatomen und $R^{10}$ mit Ausnahme der gegebenenfalls durch ein Halogenatom substituierten Thienylgruppe die in den Ansprüchen 1 bis 5 erwähnten Bedeutungen besitzen, wobei A keine Einfachbindung sein kann, wenn X die Sulfonylgruppe und $R^{10}$ ein Wasserstoffatom bedeuten, hydriert wird oder

e) zur Herstellung von Verbindungen der allgemeinen Formel I, in der n, m, p, A, X, $W^1$, $W^2$ und $R^1$ bis $R^{10}$ die in den Ansprüchen 1 bis 5 erwähnten Bedeutungen besitzen und Y ein Sauerstoffatom bedeutet, eine Verbindung der allgemeinen Formel VIII,

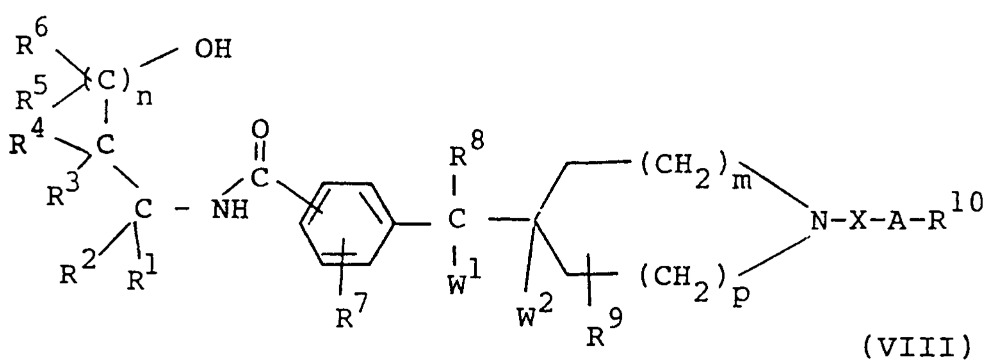

(VIII)

in der

n, m, p, A, X, $W^1$, $W^2$ und $R^1$ bis $R^{10}$ die in den Ansprüchen 1 bis 5 erwähnten Bedeutungen besitzen, cyclisiert wird und

erforderlichenfalls eine gegebenenfalls in einer Verbindung der Formel IV vorhandene Hydroxygruppe vor der Durchführung der Umsetzung b) durch eine geeignete Schutzgruppe geschützt wird und die Schutzgruppe nach beendeter Reaktion wieder abgespalten wird und/oder
gegebenenfalls eine in einer Verbindung der allgemeinen Formel VI vorhandene Hydroxygruppe bei der Durchführung der Umsetzung c) durch Verwendung von zwei Äquivalenten einer Verbindung der allgemeinen Formel VII in die entsprechende Estergruppe übergeführt wird und die Estergruppe anschließend wieder verseift wird oder
eine so erhaltene Verbindung der allgemeinen Formel I in ihr Salz mit einer anorganischen oder organischen Säure übergeführt wird.

## Claims

**1.** Arylidene-1-azacycloalkanes and arylalkyl-1-azacycloalkanes of general formula I

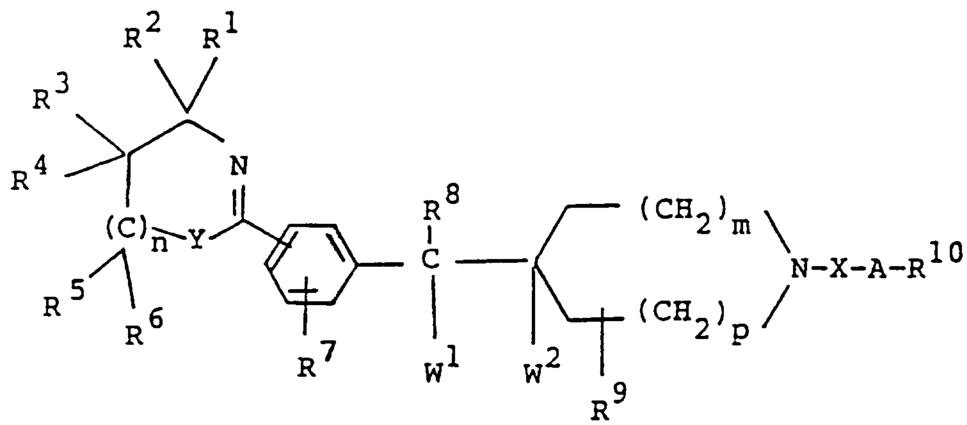

(I)

(wherein:
n denotes the numbers 0 or 1;
m denotes the numbers 1 or 2;
p denotes the numbers 0 or 1;
A denotes a single bond, a straight-chained or branched $C_{1-17}$-alkylene group, a $C_{2-17}$-alkenylene group or a $C_{2-4}$-alkynylene group;
$W^1$ and $W^2$ each denote a hydrogen atom or together denote a carbon-carbon bond,
X denotes a carbonyl or sulphonyl group;
Y denotes an oxygen or sulphur atom or an $>NR^{11}$ group;
$R^1$ to $R^6$ in each case denote a hydrogen atom, or
one, two or three of the groups $R^1$ to $R^6$, which groups may be identical or different, denote a straight-chained or branched $C_{1-4}$-alkyl group which may be substituted by a hydroxy, alkoxy, alkylthio or dialkylamino group or by a phenyl group which is optionally substituted by a halogen atom or an alkyl group, or denote an alkoxycarbonyl group and the remaining groups $R^1$ to $R^6$ each denote a hydrogen atom;
whilst one, two or all three of the groups $R^1$, $R^3$ and $R^5$ may also denote a phenyl group which is optionally substituted by an alkyl group or by a halogen atom;
$R^7$ denotes a hydrogen or halogen atom or an alkyl or alkoxy group;
$R^8$ and $R^9$ independently of each other denote a hydrogen atom, or an alkyl group;
$R^{10}$ denotes a hydrogen atom, a $C_{3-6}$-cycloalkyl group, a phenyl group optionally substituted by a halogen atom, by a straight-chained or branched $C_{1-4}$-alkyl group, a trifluoromethyl, alkoxy, cyano, nitro, alkylsulphonyl or phenyl group, a phenyl group substituted by two trifluoromethyl groups, two to five halogen atoms or by one halogen atom and an alkyl group, a naphthyl or tetrahydronaphthyl group optionally substituted by a fluorine atom, a pyridyl group or a thienyl group optionally substituted by a halogen atom or by an alkyl group; and
$R^{11}$ denotes a hydrogen atom or an alkyl group;
whilst A cannot be a single bond if X denotes the sulphonyl group and $R^{10}$ denotes a hydrogen atom, and
wherein, unless otherwise stated, the above mentioned alkyl, alkoxy, alkylthio and alkylsulphonyl groups may each contain 1 to 3 carbon atoms and the above mentioned halogen atoms may each denote a

fluorine, chlorine or bromine atom)
and the enantiomers, diastereomers and salts thereof.

**2.** Arylidene-1-azacycloalkanes and arylalkyl-1-azacycloalkanes of the general formula Ia according to claim 1,

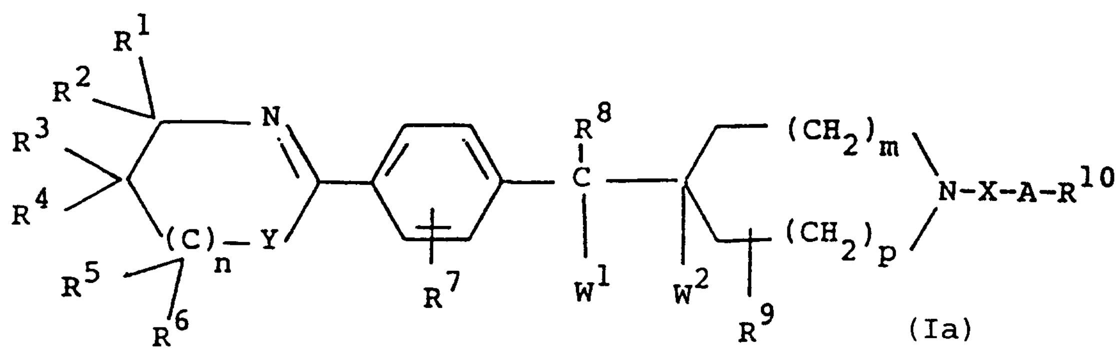

(Ia)

(wherein:
n denotes the numbers 0 or 1;
m denotes the numbers 1 or 2;
p denotes the number 0 or 1;
A denotes a single bond, a straight-chained or branched $C_{1-17}$-alkylene group, or a $C_{2-4}$-alkenylene or a $C_{2-4}$-alkynylene group;
$W^1$ and $W^2$ each denote a hydrogen atom or together denote a carbon-carbon bond,
X denotes a carbonyl or sulphonyl group;
Y denotes an oxygen or sulphur atom or an $>NR^{11}$ group;
$R^1$ to $R^4$ in each case denote a hydrogen atom, or
one or two of the groups $R^1$ to $R^4$, which may be identical or different, each denote a straight-chained or branched $C_{1-4}$-alkyl group which may be substituted by a hydroxy, alkylthio or dialkylamino group or by a phenyl group itself optionally substituted by a halogen atom, or denote a phenyl group, and the remaining groups $R^1$ to $R^4$ each denote a hydrogen atom;
$R^5$ and $R^6$, which may be identical or different, denote a hydrogen atom or a methyl group;
$R^7$ denotes a hydrogen or halogen atom or an alkyl or alkoxy group,
$R^8$ denotes a hydrogen atom, or an alkyl group;
$R^9$ denotes a hydrogen atom;
$R^{10}$ denotes a hydrogen atom, a $C_{3-6}$-cycloalkyl group, a phenyl group optionally substituted by one or two halogen atoms, a straight-chained or branched $C_{1-4}$-alkyl group, a trifluoromethyl, methoxy, cyano, nitro, methylsulphonyl or phenyl group; a phenyl group substituted by two trifluoromethyl groups or by a halogen atom and a methyl group; a phenyl group substituted by three to five fluorine atoms; a naphthyl group optionally substituted by a fluorine atom; a tetrahydronaphthyl or pyridyl group or a thienyl group optionally substituted by a halogen atom; and
$R^{11}$ denotes a hydrogen atom or an alkyl group;
whilst A cannot be a single bond if X denotes the sulphonyl group and $R^{10}$ denotes a hydrogen atom, and
wherein, unless otherwise stated, the above mentioned alkyl groups may each contain 1 to 3 carbon atoms and the above mentioned halogen atoms may each denote a fluorine, chlorine or bromine atom)
and the enantiomers, diastereomers and salts thereof.

**3.** Arylidene-1-azacycloalkanes and arylalkyl-1-azacycloalkanes of the general formula Ia, according to claim 2,
wherein:
n denotes the numbers 0 or 1,
m denotes the numbers 1 or 2,
p denotes the number 0 or 1,
A denotes a single bond, a $C_{1-17}$-straight-chained or branched alkylene or $C_{2-4}$-alkenylene group,
$W^1$ and $W^2$ each denote a hydrogen atom or together denote a carbon-carbon bond,

X denotes a carbonyl or sulphonyl group,
Y denotes an oxygen atom or an $>NR^{11}$ group,
$R^1$ to $R^4$ in each case denote a hydrogen atom or
one or two of the groups $R^1$ to $R^4$ denote a straight-chained or branched $C_{1-4}$-alkyl group, and the remaining groups $R^1$ to $R^4$ each denote a hydrogen atom,
$R^5$ and $R^6$, which may be identical or different, denote a hydrogen atom or a methyl group,
$R^7$ denotes a hydrogen or halogen atom, or a methyl or methoxy group,
$R^8$ denotes a hydrogen atom or a methyl group,
$R^9$ denotes a hydrogen atom,
$R^{10}$ denotes a hydrogen atom, a $C_{3-6}$-cycloalkyl group, a phenyl group optionally substituted by one or two halogen atoms, five fluorine atoms, an alkyl group, one or two trifluoromethyl groups or by a halogen atom and an alkyl group, a 1-naphthyl group optionally substituted by a fluorine atom in the 4-position, a 2-naphthyl group, a 1,2,3,4-tetrahydro-2-naphthyl group, a pyridyl or 4-biphenyl group, or a thienyl group optionally substituted by a halogen atom,
$R^{11}$ denotes a hydrogen atom or a methyl group,
whilst A cannot be a single bond if X denotes the sulphonyl group and $R^{10}$ denotes a hydrogen atom, and
wherein, unless otherwise stated, the above mentioned alkyl moieties may each contain 1 to 3 carbon atoms and the above mentioned halogen atoms may each denote a fluorine or chlorine atom,
and the enantiomers, diastereomers and salts thereof.

**4.** Arylidene-1-azacycloalkanes and arylalkyl-1-azacycloalkanes of the general formula Ia, according to claim 2,
wherein:
n denotes the numbers 0 or 1,
m denotes the number 1,
p denotes the number 0 or 1,
A denotes a single bond,
$W^1$ and $W^2$ each denote a hydrogen atom or together denote a carbon-carbon bond,
X denotes a carbonyl group,
Y denotes an oxygen atom,
$R^1$ to $R^6$ in each case denote a hydrogen atom,
$R^7$ denotes a hydrogen or halogen atom or a methyl group,
$R^8$ and $R^9$ each denote a hydrogen atom,
$R^{10}$ denotes a phenyl group substituted in the 4-position by a fluorine, chlorine or bromine atom or by a trifluoromethyl group, or $R^{10}$ denotes a 4-chloro-3-methylphenyl group, a 5-chloro-2-thienyl group or a cyclohexyl group,
and the salts thereof.

**5.** The following compounds of the general formula I according to claim 1:
(1) 1-(4-chlorobenzoyl)-4-[4-(2-oxazolin-2-yl)benzylidene]piperidine
(2) 1-(4-chlorobenzoyl)-4-[4-(4,5-dihydro-6H-oxazin-2-yl)benzylidene]piperidine (3) 4-[4-(2-oxazolin-2-yl)benzylidene]-1-(4-trifluoro methylbenzoyl)piperidine
(4) 1-(4-chloro-3-methylbenzoyl)-4-[4-(2-oxazolin-2-yl)benzylidene]piperidine
(5) 1-(4-fluorobenzoyl)-4-[4-(2-oxazolin-2-yl)benzylidene]piperidine
(6) 1-(5-chloro-2-thienoyl)-4-[4-(2-oxazolin-2-yl)benzylidene]piperidine
(7) 1-cyclohexanecarbonyl-4-[4-(2-oxazolin-2-yl)benzylidene]piperidine
(8) 4-[4-(2-oxazolin-2-yl)benzyl]-1-(4-trifluoromethylbenzoyl)piperidine
(9) 1-(4-chlorobenzoyl)-4-[4-(2-oxazolin-2-yl)-benzyl]piperidine
(10) 1-(4-chlorobenzoyl)-3-[4-(2-oxazolin-2-yl)benzylidene]pyrrolidine
(11) 1-(4-chlorobenzoyl)-4-[2-fluoro-4-(2-oxazolin-2-yl)benzylidene]piperidine
(12) 1-(4-chlorobenzoyl)-4-[3-methyl-4-(2-oxazolin-2-yl)benzylidene]piperidine
and the salts thereof.

**6.** Physiologically acceptable salts of the compounds according to at least one of claims 1 to 5 with inorganic or organic acids.

**7.** Medicaments, containing a compound according to at least one of claims 1 to 5 or a physiologically acceptable salt according to claim 6 optionally together with one or more inert excipients and/or diluents.

**8.** Use of a compound according to at least one of claims 1 to 6 for the preparation of a drug for inhibiting cholesterol biosynthesis, for the treatment or prophylaxis of hyperlipidaemias, for the treatment of disorders which are connected with excessive cell proliferation, for the prophylaxis and treatment of gallstone trouble or for treating mycoses.

**9.** Use of a compound according to at least one of claims 1 to 6 for the preparation of a foodstuff for laying hens for the production of low-cholesterol eggs.

**10.** Process for the preparation of a medicament according to claim 7, characterized in that a compound according to at least one of claims 1 to 6 is incorporated into one or more inert excipients and/or diluents by non-chemical means.

**11.** Process for the preparation of the compounds according to at least one of claims 1 to 6, characterized in that

a) a compound of the general formula II

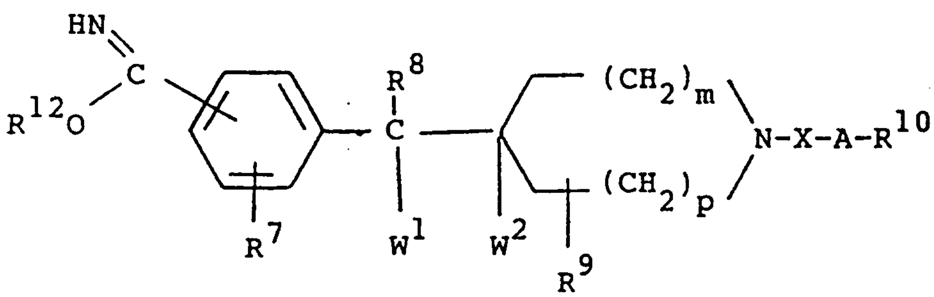

(II)

(wherein
m, p, A, $W^1$, $W^2$, X and $R^7$ to $R^{10}$ are as defined in claims 1 to 5 and $R^{12}$ denotes a $C_{1-10}$-alkyl group)
is reacted with a compound of the general formula III

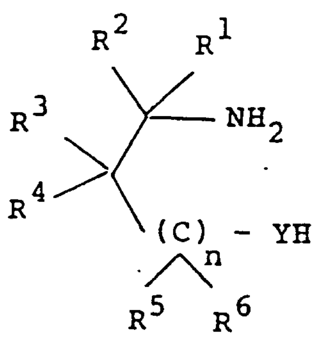

(III)

wherein
n, Y and $R^1$ to $R^6$ are as defined in claims 1 to 5, or
b) in order to prepare compounds of general formula I wherein n, m, p, A, X, Y and $R^1$ to $R^{10}$ are defined as in claims 1 to 5 and $W^1$ and $W^2$ together denote a carbon-carbon bond, a phosphonate ester of the general formula IV

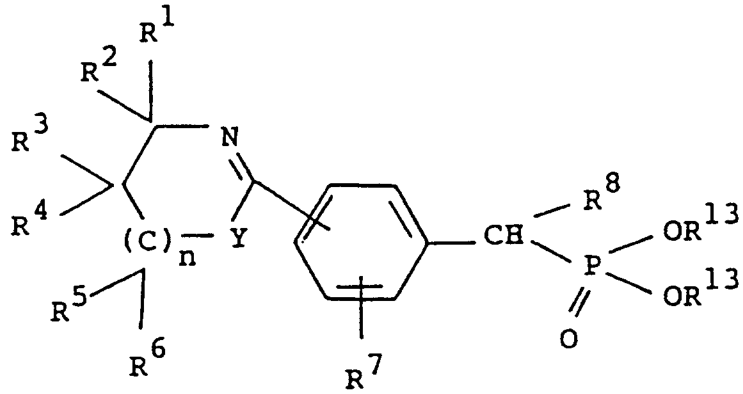

(IV)

(wherein
n, Y and $R^1$ to $R^8$ are as defined in claims 1 to 5 and $R^{13}$ denotes a $C_{1-10}$-alkyl group)
is reacted with a compound of the general formula V,

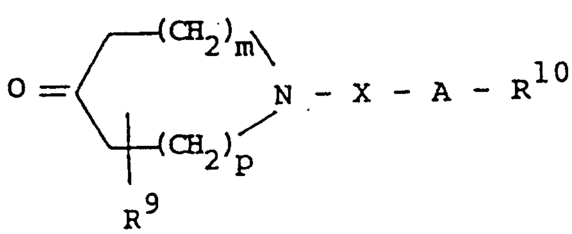

(V)

wherein
m, p, A, X, $R^9$ and $R^{10}$ are as defined in claims 1 to 5, or
c) a compound of the general formula VI

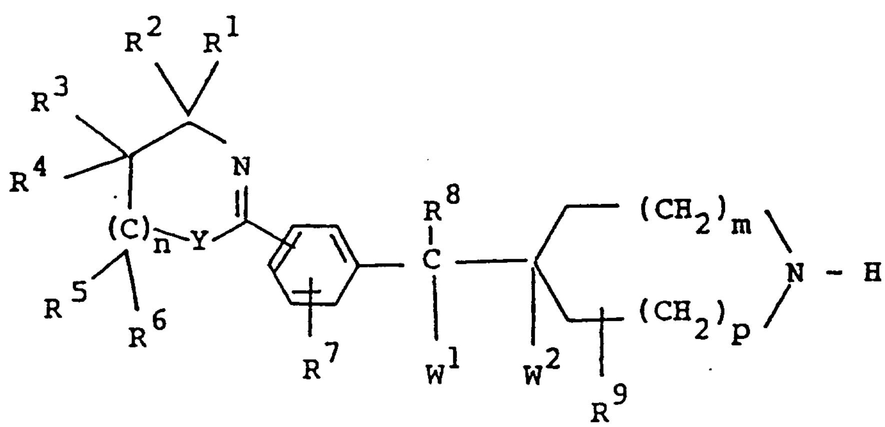

(VI)

(wherein
n, m, p, $W^1$, $W^2$, Y and $R^1$ to $R^9$ are as defined in Claims 1 to 5)
is reacted with a compound of the general formula VII

$Z^1$ - X - A - $R^{10}$ (VII)

wherein
A, X and $R^{10}$ are as defined in claims 1 to 5 and $Z^1$ denotes a reactive leaving group, or
d) in order to prepare compounds of general formula I wherein n, m, p, X and $R^7$ to $R^9$ have the meanings given in claims 1 to 5, A denotes a single bond or a straight-chained or branched $C_{1-17}$-

alkylene group, $W^1$ and $W^2$ each denote a hydrogen atom and Y denotes an oxygen atom or the $>NR^{11}$ group, where $R^{11}$ is defined as in claims 1 to 5, $R^1$ to $R^6$ have the meanings given in claims 1 to 5 with the exception of the straight-chained or branched $C_{1-4}$-alkyl group substituted by an alkylthio group, and $R^{10}$ has the meanings given in claims 1 to 5 with the exception of the thienyl group optionally substituted by a halogen atom, whilst A cannot be a single bond if X denotes a sulphonyl group and $R^{10}$ denotes a hydrogen atom,
a compound of general formula I'

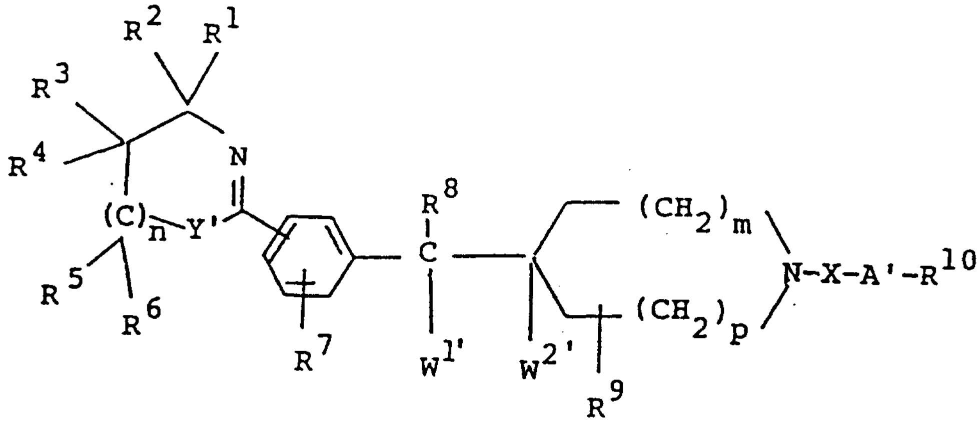

(I')

wherein wherein
n, m, p, X and $R^7$ to $R^9$ have the meanings given in claims 1 to 5, A' denotes a single bond or a straight-chained or branched $C_{1-17}$-alkylene group, $W^{1'}$ and $W^{2'}$ together denote a carbon-carbon bond and Y' denotes an oxygen atom or the $>NR^{11}$ group, where $R^{11}$ is defined as in claims 1 to 5, $R^1$ to $R^6$ have the meanings given in claims 1 to 5 with the exception of the straight-chained or branched $C_{1-4}$-alkyl group substituted by an alkylthio group, and $R^{10}$ has the meanings given in claims 1 to 5 with the exception of the thienyl group optionally substituted by a halogen atom, whilst A cannot denote a single bond if X denotes the sulphonyl group and $R^{10}$ denotes a hydrogen atom, is hydrogenated or
e) in order to prepare compounds of general formula I wherein n, m, p, A, X, $W^1$, $W^2$ and $R^1$ to $R^{10}$ have the meanings given in claims 1 to 5 and Y denotes an oxygen atom, a compound of general formula VIII

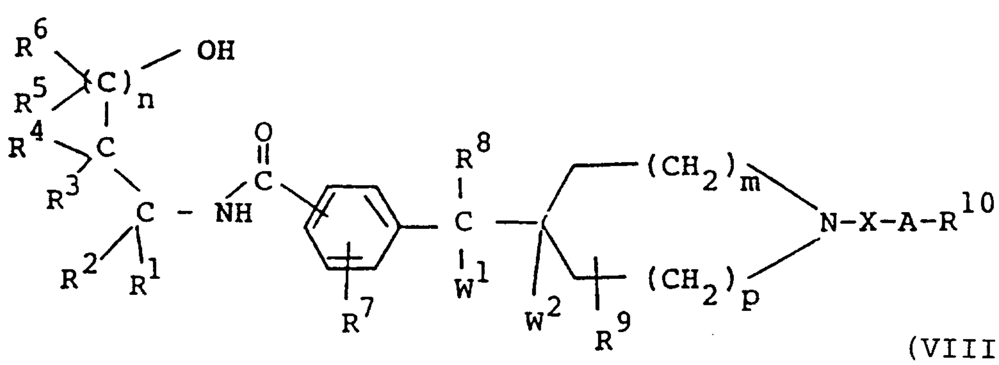

(VIII)

wherein
n, m, p, A, X, $W^1$, $W^2$ and $R^1$ to $R^{10}$ have the meanings given in claims 1 to 5, is cyclised and
if necessary, a hydroxy group which may be present in a compound of the formula IV is protected by a suitable protecting group before carrying out reaction b) and the protecting group is removed again after the reaction is complete and/or
if appropriate, a hydroxy group present in a compound of the general formula VI is converted into the corresponding ester group when carrying out reaction c) by the use of two equivalents of a

compound of the general formula VII and the ester group is then saponified again, or
a compound of the general formula I thus obtained is converted into its salt using an inorganic or organic acid.

## Revendications

1. Arylidène-1-azacycloalcanes et arylalkyl-1-azacycloalcanes de formule générale I

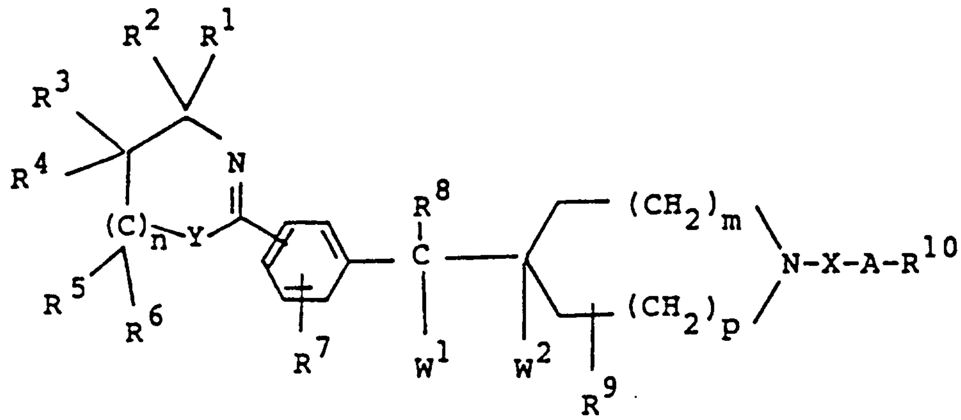

(I)

dans laquelle
n représente les nombres 0 ou 1,
m représente les nombres 1 ou 2,
p représente les nombres 0 ou 1,
A représente une liaison simple, un groupe alkylène linéaire ou ramifié de 1 à 17 atomes de carbone, un groupe alcénylène de 2 à 17 atomes de carbone ou un groupe alcynylène de 2 à 4 atomes de carbone,
$W^1$ et $W^2$ représentent chacun un atome d'hydrogène ou ensemble une liaison carbone-carbone,
X représente un groupe carbonyle ou sulfonyle,
Y représente un atome d'oxygène ou de soufre ou un groupe $>NR^{11}$,
$R^1$ à $R^6$ représentent chacun un atome d'hydrogène ou
un, deux ou trois des restes $R^1$ à $R^6$, les restes pouvant être identiques ou différents, représentent un groupe alkyle linéaire ou ramifié de 1 à 4 atomes de carbone, qui peut être substitué par un groupe hydroxyle, alcoxy, alkylthio ou dialkylamino ou par un groupe phényle éventuellement substitué par un atome d'halogène ou un groupe alkyle, ou un groupe alcoxycarbonyle et les autres restes parmi $R^1$ à $R^6$ représentent chacun un atome d'hydrogène,
un, ou deux des restes $R^1$, $R^3$ et $R^5$ ou ces trois restes pouvant aussi représenter un groupe phényle éventuellement substitué par un groupe alkyle ou par un atome d'halogène,
$R^7$ représente un atome d'hydrogène ou d'halogène, un groupe alkyle ou alcoxy,
$R^8$ et $R^9$ représentent chacun indépendamment l'un de l'autre un atome d'hydrogène ou un groupe alkyle,
$R^{10}$ représente un atome d'hydrogène, un groupe cycloalkyle de 3 à 6 atomes de carbone, un groupe phényle éventuellement substitué par un atome d'halogène, un groupe alkyle linéaire ou ramifié de 1 à 4 atomes de carbone, un groupe trifluorométhyle, alcoxy, cyano, nitro, alkylsulfonyle ou phényle, un groupe phényle substitué par deux groupes trifluorométhyle, deux à cinq atomes d'halogène ou par un atome d'halogène et un groupe alkyle, un groupe naphtyle ou tétrahydronaphtyle éventuellement substitué par un atome de fluor, un groupe pyridyle ou un groupe thiényle éventuellement substitué par un atome d'halogène ou un groupe alkyle,
$R^{11}$ représente un atome d'hydrogène ou un groupe alkyle,
A ne pouvant pas être une liaison simple lorsque X représente le groupe sulfonyle et $R^{10}$ représente un atome d'hydrogène, et,
sauf indication contraire, les restes alkyle, alcoxy, alkylthio et alkylsulfonyle cités ci-dessus pouvant contenir chacun 1 à 3 atomes de carbone et les atomes d'halogène cités ci-dessus pouvant représenter chacun un atome de fluor, de chlore ou de brome,
leurs énantiomères, diastéréoisomères et leurs sels.

2. Arylidène-1-azacycloalcanes et arylalkyl-1-azacycloalcanes selon la revendication 1 de formule générale Ia

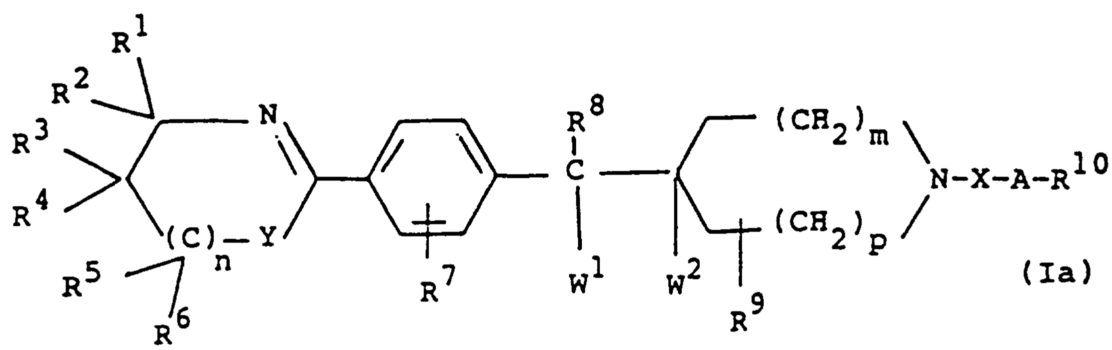

(Ia)

dans laquelle
n représente les nombres 0 ou 1,
m représente les nombres 1 ou 2,
p représente les nombres 0 ou 1,
A représente une liaison simple, un groupe alkylène linéaire ou ramifié de 1 à 17 atomes de carbone, un groupe alcénylène de 2 à 4 atomes de carbone ou un groupe alcynylène de 2 à 4 atomes de carbone,
$W^1$ et $W^2$ représentent chacun un atome d'hydrogène ou ensemble une liaison carbone-carbone,
X représente un groupe carbonyle ou sulfonyle,
Y représente un atome d'oxygène ou de soufre ou un groupe $>NR^{11}$,
$R^1$ à $R^4$ représentent chacun un atome d'hydrogène ou
un ou deux des restes $R^1$ à $R^4$ représentent chacun indépendamment les uns des autres un groupe alkyle linéaire ou ramifié de 1 à 4 atomes de carbone, qui peut être substitué par un groupe hydroxyle, alkylthio ou dialkylamino ou par un groupe phényle éventuellement substitué par un atome d'halogène, ou un groupe phényle et les autres restes parmi $R^1$ à $R^4$ représentent chacun un atome d'hydrogène,
$R^5$ et $R^6$, qui peuvent être identiques ou différents, représentent un atome d'hydrogène ou un groupe méthyle,
$R^7$ représente un atome d'hydrogène ou d'halogéne, un groupe alkyle ou alcoxy,
$R^8$ représente un atome d'hydrogène ou un groupe alkyle,
$R^9$ représente un atome d'hydrogène,
$R^{10}$ représente un atome d'hydrogène, un groupe cycloalkyle de 3 à 6 atomes de carbone, un groupe phényle éventuellement substitué par un ou deux atomes d'halogène, un groupe alkyle linéaire ou ramifié de 1 à 4 atomes de carbone, un groupe trifluorométhyle, méthoxy, cyano, nitro, méthylsulfonyle ou phényle, un groupe phényle substitué par deux groupes trifluorométhyle ou par un atome d'halogène et un groupe méthyle, un groupe phényle substitué par trois à cinq atomes de fluor, un groupe naphtyle éventuellement substitué par un atome de fluor, un groupe tétrahydronaphtyle ou pyridyle ou un groupe thiényle éventuellement substitué par un atome d'halogène et
$R^{11}$ représente un atome d'hydrogène ou un groupe alkyle,
A ne pouvant pas être une liaison simple lorsque X représente le groupe sulfonyle et $R^{10}$ représente un atome d'hydrogène, et,
sauf indication contraire, les restes alkyle cités ci-dessus pouvant contenir chacun 1 à 3 atomes de carbone et les atomes d'halogène cités ci-dessus pouvant représenter chacun un atome de fluor, de chlore ou de brome,
leurs énantiomères, diastéréoisomères et leurs sels.

3. Arylidène-1-azacycloalcanes et arylalkyl-1-azacycloalcanes de formule générale Ia selon la revendication 2
dans laquelle
n représente les nombres 0 ou 1,
m représente les nombres 1 ou 2,
p représente les nombres 0 ou 1,

A représente une liaison simple, un groupe alkylène linéaire ou ramifié de 1 à 17 atomes de carbone ou un groupe alcénylène de 2 à 4 atomes de carbone,

$W^1$ et $W^2$ représentent chacun un atome d'hydrogène ou ensemble une liaison carbone-carbone,

X représente un groupe carbonyle ou sulfonyle,

Y représente un atome d'oxygène ou un groupe $>NR^{11}$,

$R^1$ à $R^4$ représentent chacun un atome d'hydrogène ou

un ou deux des restes $R^1$ à $R^4$ représentent chacun indépendamment les uns des autres un groupe alkyle linéaire ou ramifié de 1 à 4 atomes de carbone, et les autres restes parmi $R^1$ à $R^4$ représentent chacun un atome d'hydrogène,

$R^5$ et $R^6$, qui peuvent être identiques ou différents, représentent un atome d'hydrogène ou un groupe méthyle,

$R^7$ représente un atome d'hydrogène ou d'halogène, un groupe méthyle ou méthoxy,

$R^8$ représente un atome d'hydrogène ou un groupe méthyle,

$R^9$ représente un atome d'hydrogène,

$R^{10}$ représente un atome d'hydrogène, un groupe cycloalkyle de 3 à 6 atomes de carbone, un groupe phényle éventuellement substitué par un ou deux atomes d'halogène, cinq atomes de fluor, un groupe alkyle, un ou deux groupes trifluorométhyle ou par un atome d'halogène et un groupe alkyle, un groupe 1-naphtyle éventuellement substitué en position 4 par un atome de fluor, un groupe 2-naphtyle, un groupe 1,2,3,4-tétrahydro-2-naphtyle, un groupe pyridyle ou 4-biphényle ou un groupe thiényle éventuellement substitué par un atome d'halogène et

$R^{11}$ représente un atome d'hydrogène ou un groupe méthyle,

A ne pouvant pas être une liaison simple lorsque X représente le groupe sulfonyle et $R^{10}$ représente un atome d'hydrogène, et,

sauf indication contraire, les restes alkyle cités ci-dessus pouvant contenir chacun 1 à 3 atomes de carbone et les atomes d'halogène cités ci-dessus pouvant représenter chacun un atome de fluor ou de chlore,

leurs énantiomères, diastéréoisomères et leurs sels.

**4.** Arylidène-1-azacycloalcanes et arylalkyl-1-azacycloalcanes de formule générale Ia selon la revendication 2

dans laquelle

n représente les nombres 0 ou 1,

m représente le nombre 1,

p représente les nombres 0 ou 1,

A représente une liaison simple,

$W^1$ et $W^2$ représentent chacun un atome d'hydrogène ou ensemble une liaison carbone-carbone,

X représente un groupe carbonyle,

Y représente un atome d'oxygène,

$R^1$ à $R^6$ représentent chacun un atome d'hydrogène,

$R^7$ représente un atome d'hydrogène ou d'halogène ou un groupe méthyle,

$R^8$ et $R^9$ représentent chacun un atome d'hydrogène,

$R^{10}$ représente un groupe phényle substitué en position 4 par un atome de fluor, de chlore ou de brome ou par un groupe trifluorométhyle, un groupe 4-chloro-3-méthylphényle, un groupe 5-chloro-2-thiényle ou un groupe cyclohexyle,

et leurs sels.

**5.** Composés suivants de formule générale I selon la revendication 1:

(1) 1-(4-chlorobenzoyl)-4-[4-(2-oxazolin-2-yl)benzylidène]pipéridine

(2) 1-(4-chlorobenzoyl)-4-[4-(4,5-dihydro-6H-oxazin-2-yl)benzylidène]pipéridine

(3) 4-[4-(2-oxazolin-2-yl)benzylidène]-1-(4-trifluorométhyl-benzoyl)pipéridine

(4) 1-(4-chloro-3-méthylbenzoyl)-4-[4-(2-oxazolin-2-yl)benzylidène]pipéridine

(5) 1-(4-fluorobenzoyl)-4-[4-(2-oxazolin-2-yl)benzylidène]pipéridine

(6) 1-(5-chloro-2-thiénoyl)-4-[4-(2-oxazolin-2-yl)benzylidène]pipéridine

(7) 1-cyclohexanecarbonyl-4-[4-(2-oxazolin-2-yl)benzylidène]pipéridine

(8) 4-[4-(2-oxazolin-2-yl)benzyl]-1-(4-trifluorométhylbenzoyl)pipéridine

(9) 1-(4-chlorobenzoyl)-4-[4-(2-oxazolin-2-yl)benzyl]pipéridine

(10) 1-(4-chlorobenzoyl)-3-[4-(2-oxazolin-2-yl)benzylidène]pyrrolidine

(11) 1-(4-chlorobenzoyl)-4-[2-fluoro-4-(2-oxazolin-2-yl)benzylidène]pipéridine

(12) 1-(4-chlorobenzoyl)-4-[3-méthyl-4-(2-oxazolin-2-yl)benzylidène]pipéridine et leurs sels.

**6.** Sels physiologiquement acceptables des composés selon au moins l'une des revendications 1 à 5 avec des acides inorganiques ou organiques.

**7.** Médicament contenant un composé selon au moins l'une des revendications 1 à 5 ou un sel physiologiquement acceptable selon la revendication 6 et éventuellement un ou plusieurs supports et/ou diluants inertes.

**8.** Utilisation d'un composé selon au moins l'une des revendications 1 à 6 pour la préparation d'un médicament pour l'inhibition de la biosynthèse du cholestérol, pour le traitement ou la prophylaxie des hyperlipidémies, pour le traitement des maladies qui sont en rapport avec une prolifération cellulaire accrue, pour la prophylaxie et le traitement des lésions provoquées par des calculs biliaires ou pour le traitement des mycoses.

**9.** Utilisation d'un composé selon au moins l'une des revendications 1 à 6 pour la préparation d'un aliment pour poules pondeuses pour produire des oeufs pauvres en cholestérol.

**10.** Procédé de préparation d'un médicament selon la revendication 7, caractérisé en ce qu'un composé selon au moins l'une des revendications 1 à 6 est incorporé par voie non chimique dans un ou plusieurs supports et/ou diluants inertes.

**11.** Procédé de préparation des composés selon au moins l'une des revendications 1 à 6, caractérisé en ce que

a) un composé de formule générale II

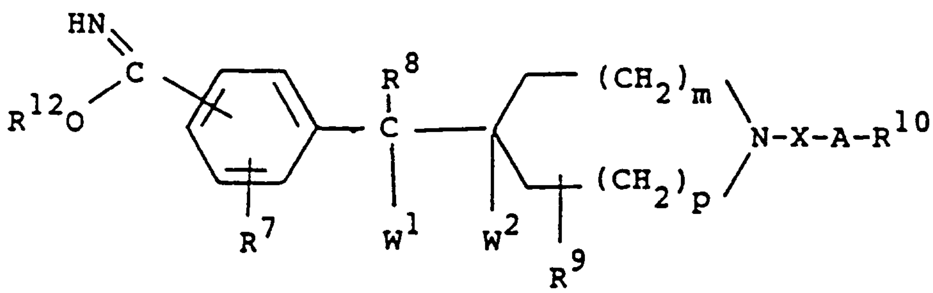

(II)

dans laquelle

m, p, A, $W^1$, $W^2$, X et $R^7$ à $R^{10}$ possèdent les significations indiquées dans les revendications 1 à 5 et $R^{12}$ représente un groupe alkyle de 1 à 10 atomes de carbone, est mis à réagir avec un composé de formule générale III

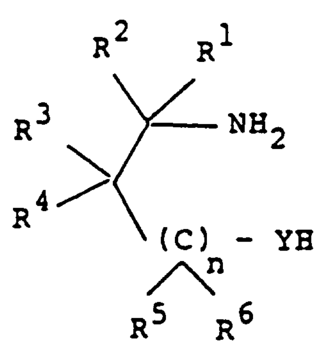

(III)

dans laquelle

n, Y et $R^1$ à $R^6$ possèdent les significations indiquées dans les revendications 1 à 5, ou bien

b) pour la préparation de composés de formule générale I dans laquelle n, m, p, A, X, Y et $R^1$ à $R^{10}$ possèdent les significations indiquées dans les revendications 1 à 5 et $W^1$ et $W^2$ représentent ensemble une liaison carbone-carbone, un ester phosphonique de formule générale IV

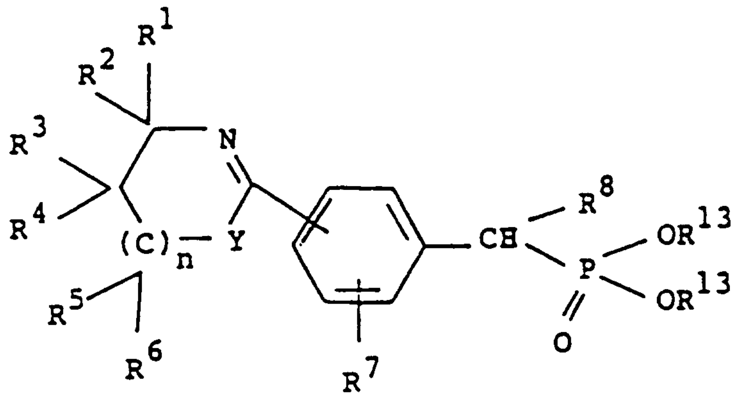

(IV)

dans laquelle

n, Y et $R^1$ à $R^8$ possèdent les significations indiquées dans les revendications 1 à 5 et $R^{13}$ représente un groupe alkyle de 1 à 10 atomes de carbone, est mis à réagir avec un composé de formule générale V

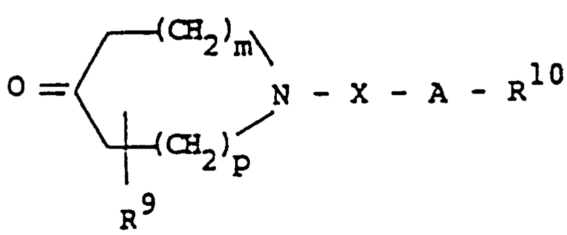

(V)

dans laquelle

m, p, A, X, $R^9$ et $R^{10}$ possèdent les significations indiquées dans les revendications 1 à 5, ou bien

c) un composé de formule générale VI

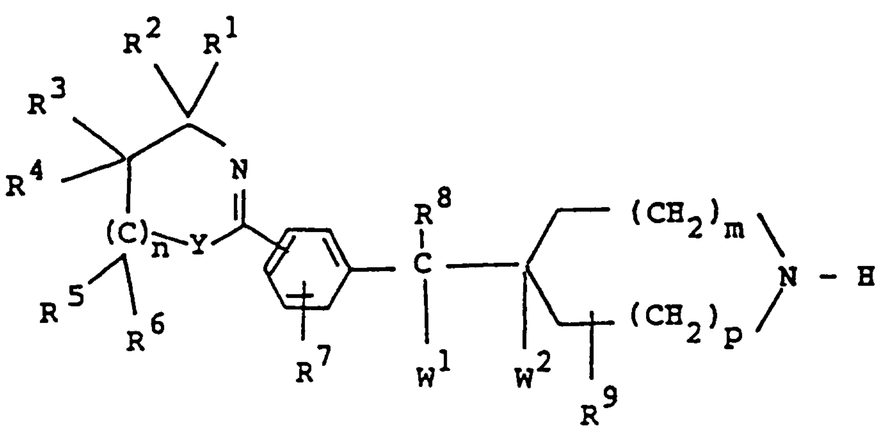

(VI)

dans laquelle n, m, p, $W^1$, $W^2$, Y et $R^1$ à $R^9$ possèdent les significations indiquées dans les revendications 1 à 5, est mis à réagir avec un composé de formule générale VII

$Z^1$ - X - A - $R^{10}$ (VII)

dans laquelle

A, X et $R^{10}$ possèdent les significations indiquées dans les revendications 1 à 5 et $Z^1$ représente un groupe partant réactif, ou bien

d) pour la préparation de composés de formule générale I dans laquelle n, m, p, X et $R^7$ à $R^9$ possèdent les significations indiquées dans les revendications 1 à 5, A représente une liaison simple ou un groupe alkylène linéaire ou ramifié de 1 à 17 atomes de carbone, $W^1$ et $W^2$ représentent chacun un atome d'hydrogène et Y représente un atome d'oxygène ou le groupe $>NR^{11}$, $R^{11}$ possédant les significations indiquées dans les revendications 1 à 5, $R^1$ à $R^6$ possédant, à l'exception du groupe alkyle linéaire ou ramifié de 1 à 4 atomes de carbone substitué par un groupe alkylthio, et $R^{10}$ possédant, à l'exception du groupe thiényle éventuellement substitué par un atome d'halogène, les significations indiquées dans les revendications 1 à 5, A ne pouvant pas être une liaison simple lorsque X représente le groupe sulfonyle et $R^{10}$ représente un atome d'hydrogène, un composé de formule générale I'

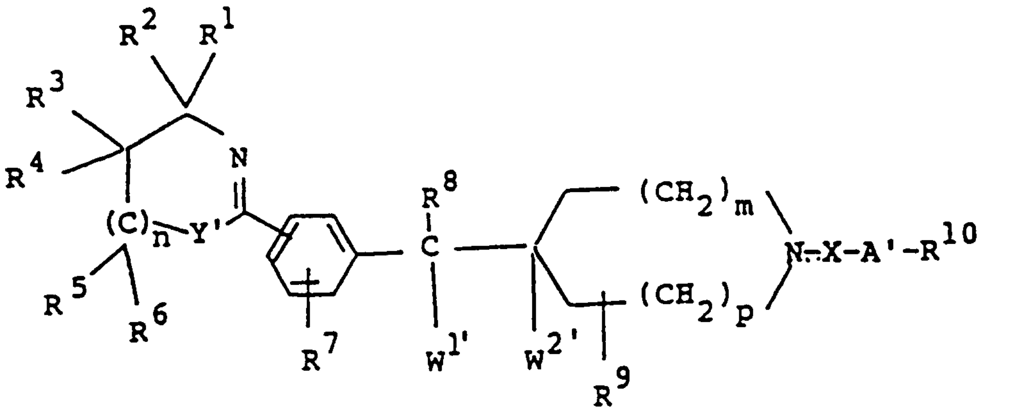

(I')

dans laquelle n, m, p, X et $R^7$ à $R^9$ possèdent les significations indiquées dans les revendications 1 à 5, A' représente une liaison simple ou un groupe alkylène linéaire ou ramifié de 1 à 17 atomes de carbone, $W^{1'}$ et $W^{2'}$ représentent ensemble une liaison carbone-carbone et Y' représente un atome d'oxygène ou le groupe $>NR^{11}$, $R^{11}$ possédant les significations indiquées dans les revendications 1 à 5, $R^1$ à $R^6$ possédant, à l'exception du groupe alkyle linéaire ou ramifié de 1 à 4 atomes de carbone substitué par un groupe alkylthio, et $R^{10}$ possédant, à l'exception du groupe thiényle éventuellement substitué par un atome d'halogène, les significations indiquées dans les revendications 1 à 5, A ne pouvant pas être une liaison simple lorsque X représente le groupe sulfonyle et $R^{10}$ représente un atome d'hydrogène, est hydrogéné ou bien

e) pour la préparation de composés de formule générale I dans laquelle n, m, p, A, X, $W^1$, $W^2$ et $R^1$ à $R^{10}$ possèdent les significations indiquées dans les revendications 1 à 5 et Y représente un atome d'oxygène, un composé de formule générale VIII

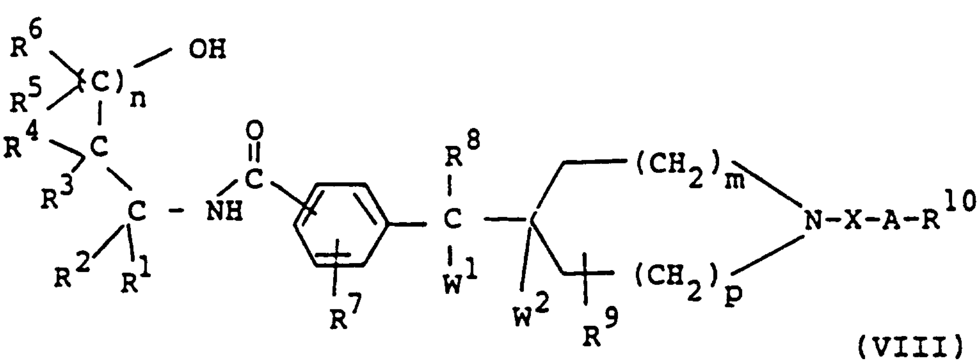

(VIII)

dans laquelle

n, m, p, A, X, $W^1$, $W^2$ et $R^1$ à $R^{10}$ possèdent les significations indiquées dans les revendications 1 à 5, est cyclisé et

si nécessaire un groupe hydroxyle éventuellement présent dans un composé de formule IV est protégé par un groupe protecteur approprié avant la conduite de la réaction b) et le groupe protecteur est de nouveau clivé après la fin de la réaction et/ou

éventuellement un groupe hydroxyle présent dans un composé de formule générale VI est converti en le groupe ester correspondant lors de la conduite de la réaction c) au moyen de deux équivalents d'un composé de formule générale VII et le groupe ester est ensuite de nouveau saponifié ou

un composé de formule générale I ainsi obtenu est converti en son sel avec un acide inorganique ou organique.